# EUROPEAN PATENT APPLICATION

(11) **EP 4 428 540 A1**
(43) Date of publication of application: **11.09.2024**
(21) Application number: 24161708.3
(22) Date of filing: 06.03.2024
(51) Int. Cl.: G01N 35/00, B01F 31/20

(54) **SPECIMEN ANALYZER AND SPECIMEN ANALYSIS METHOD**

(30) Priority: 08.03.2023 JP 2023035980
(71) Applicant: SYSMEX CORPORATION, Kobe-shi, Hyogo 651-0073 (JP)
(72) Inventor: Misawa, Kota, Kobe-shi, Hyogo 651-0073 (JP); Nakanishi, Noriyuki, Kobe-shi, Hyogo 651-0073 (JP); Ikuta, Junya, Kobe-shi, Hyogo 651-0073 (JP); Mizuhashi, Toru, Kobe-shi, Hyogo 651-0073 (JP); Kumagai, Atsushi, Kobe-shi, Hyogo 651-0073 (JP)
(74) Representative: Hoffmann Eitle

(57) **Abstract**

Disclosed is a specimen analyzer comprising: a rack transport part configured to transport a rack accommodating a plurality of specimen containers; an agitating part configured to agitate a specimen in each of the specimen containers; a suction part configured to suction the specimen from the specimen container; a container transport part configured to transport the specimen container between the rack on the rack transport part, the agitating part, and a suction position for specimen suctioning by the suction part; a measurement part configured to measure a measurement sample prepared from the specimen suctioned by the suction part; and a controller configured to control at least the agitating part, the suction part, and the container transport part, wherein the specimen analyzer is configured such that control by the controller causes, during a period from start to end of agitation of one of the specimen containers, at least one of transferring to the suction position, suctioning of a specimen, and returning to the rack to be executed on another one of the specimen containers.

## Description

### CROSS REFERENCE TO RELATED APPLICATIONS

This application claims priority from prior Japanese Patent Application No. 2023-035980, filed on March 8, 2023, entitled "SPECIMEN ANALYZER AND SPECIMEN ANALYSIS METHOD", the entire content of which is incorporated herein by reference.

### FIELD OF THE INVENTION

The present invention relates to a specimen analyzer and a specimen analysis method for agitating a specimen in a container, suctioning the agitated specimen from inside the container, and analyzing the suctioned specimen.

### BACKGROUND OF THE INVENTION

Japanese Laid-Open Patent Publication No. 2012-173251 discloses a specimen analyzer including: a specimen transport device which transports a rack accommodating a plurality of specimen containers; and a measurement unit. The measurement unit includes a hand part, a specimen container transfer part, and a piercer. In Japanese Laid-Open Patent Publication No. 2012-173251, when any of the specimen containers accommodated in the rack is placed at a specimen container take-out position by the specimen transport device, the hand part grips and takes out the specimen container from the rack and is rotated as a pendulum while gripping the specimen container, to agitate a specimen in the specimen container. After the agitation is ended, the hand part sets the specimen container onto the specimen container transfer part. The specimen container transfer part transfers the specimen container to a suction position. At the suction position, the piercer suctions the specimen in the specimen container. The specimen container having been subjected to suctioning of the specimen is transferred to a specimen setting position by the specimen container transfer part, is taken out from the specimen container transfer part by the hand part, and then is returned to the original position in the rack. Thereafter, when a next specimen container accommodated in the rack is placed at the specimen container take-out position, the measurement unit executes the above operations on the next specimen container.

In the above Japanese Laid-Open Patent Publication No. 2012-173251, the series of operations described above is repeated by being performed on each of the specimen containers. The series of operations includes: taking out the specimen container from the rack; performing agitation; transferring the specimen container to the suction position; suctioning a specimen therefrom; transferring the specimen container to the specimen setting position; taking out the specimen container from the specimen container transfer part; and returning the specimen container to the rack. In order to improve the efficiency of specimen testing work, further increase in the number of specimens to be processed per unit time by such a specimen analyzer in the case of analyzing a plurality of specimens has been desired.

The present invention is directed to increasing the number of specimens to be processed per unit time by a specimen analyzer in the case of analyzing a plurality of specimens.

### SUMMARY OF THE INVENTION

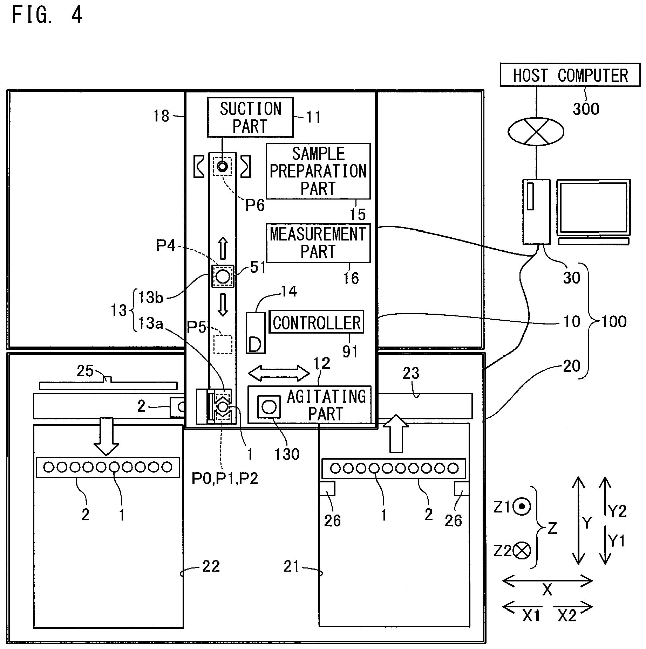

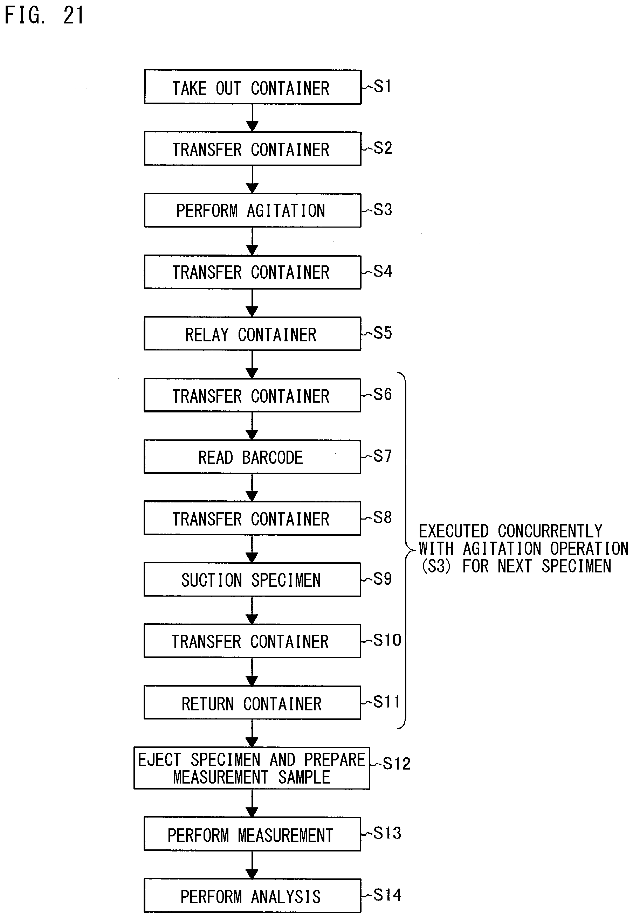

In order to attain the above object, as shown in FIG. 4 and FIG. 21, a specimen analyzer (100) according to the present invention includes: a rack transport part (20) configured to transport a rack (2) accommodating a plurality of specimen containers (1); an agitating part (12) configured to agitate a specimen in each of the specimen containers (1); a suction part (11) configured to suction the specimen from the specimen container (1); a container transport part (13) configured to transport the specimen container (1) between the rack (2) on the rack transport part (20), the agitating part (12), and a suction position (P6) for specimen suctioning by the suction part (11); a measurement part (16) configured to measure a measurement sample prepared from the specimen suctioned by the suction part (11); and a controller (91) configured to control at least the agitating part (12), the suction part (11), and the container transport part (13). The specimen analyzer (100) is configured such that control by the controller (91) causes, during a period from start to end of agitation of one of the specimen containers (1), at least one of transferring to the suction position (P6), suctioning of a specimen, and returning to the rack (2) to be executed on another one of the specimen containers (1).

The specimen analyzer (100) according to the present invention is configured such that control by the controller (91) causes, during the period from start to end of agitation of the one specimen container (1), at least one of transferring to the suction position (P6), suctioning of a specimen, and returning to the rack (2) to be executed on the other specimen container (1). Consequently, agitation processing for the one specimen container (1) by the agitating part (12) and processing for the other specimen container (1) (at least one of transferring to the suction position (P6), suctioning of a specimen, and returning to the rack (2)) can be executed as concurrent processing operations that coincide in time with each other. Therefore, as compared to a case where the processing for the one specimen container (1) and the processing for the other specimen container (1) are executed separately and sequentially, the number of specimens to be processed per unit time by the specimen analyzer (100) in the case of analyzing a plurality of specimens can be increased by a number corresponding to the period during which the agitation processing for the one specimen container (1) and the processing for the other specimen container (1) coincide in time with each other.

A specimen analysis method according to the present invention is, as shown in FIG. 21, a specimen analysis method to be performed by a specimen analyzer (100) configured to analyze a specimen contained in a specimen container (1), the specimen analysis method including: a step (S1) of taking out, from a rack (2) accommodating a plurality of the specimen containers (1), any of the specimen containers (1); a step (S3) of agitating a specimen in the specimen container (1) having been taken out; a step (S8) of transferring, to a suction position (P6), the specimen container (1) having been agitated; a step (S9) of suctioning the specimen from the specimen container (1) transferred to the suction position (P6); a step (S11) of returning, to the rack (2), the specimen container (1) having been subjected to the suctioning of the specimen; and a step (S13) of measuring a measurement sample prepared from the suctioned specimen. During a period from start to end of the agitating of one of the specimen containers (1), at least one of the transferring to the suction position (P6) (step S8), the suctioning of a specimen (step S9), and the returning to the rack (2) (step S13) is executed on another one of the specimen containers (1).

In the specimen analysis method according to the present invention, during the period from start to end of the agitating of the one specimen container (1), at least one step among the transferring to the suction position (P6), the suctioning of a specimen, and the returning to the rack (2) is executed on the other specimen container (1). Consequently, agitation processing for the one specimen container (1) and processing for the other specimen container (1) (at least one of the transferring to the suction position (P6), the suctioning of a specimen, and the returning to the rack (2)) can be executed as concurrent processing operations that coincide in time with each other. Therefore, as compared to a case where the processing for the one specimen container (1) and the processing for the other specimen container (1) are executed separately and sequentially, the number of specimens to be processed per unit time by the specimen analyzer (100) in the case of analyzing a plurality of specimens can be increased by a number corresponding to the period during which the agitation processing for the one specimen container (1) and the processing for the other specimen container (1) coincide in time with each other.

The present invention enables increase of the number of specimens to be processed per unit time by a specimen analyzer in the case of analyzing a plurality of specimens.

### BRIEF DESCRIPTION OF THE DRAWINGS

FIG. 1 is a schematic perspective view of a specimen analyzer in the present embodiment;
FIG. 2 is a schematic top view showing a rack transport part;
FIG. 3 is a schematic perspective view showing a specimen rack and specimen containers;
FIG. 4 is a schematic diagram showing the specimen analyzer;
FIG. 5 is a perspective view schematically showing the inside of a measurement unit;
FIG. 6 is a schematic top view for explaining arrangement of an agitating part and a container transport part;
FIG. 7 a side view for explaining a container transfer part;
FIG. 8 is a perspective view for explaining the agitating part;
FIG. 9 is a perspective view for explaining the agitating part in a state where a holding part is rotated to a predetermined angle;
FIG. 10 is a cross-sectional view for explaining the agitating part;
FIG. 11 is a perspective view for explaining only the holding part;
FIG. 12 is a cross-sectional view for explaining the holding part in a state where a specimen container is fixed;
FIG. 13 is a diagram for explaining rotation of the holding part at the time of agitation;
FIG. 14 is a diagram for explaining a state where the holding part is rotated to the predetermined angle;
FIG. 15 is a diagram for explaining relative movement between an arm and the holding part each of which has been stopped at the predetermined angle;
FIG. 16 is a diagram for explaining a state where the holding part is at a preparatory position at the time of end of agitation;
FIG. 17 is a diagram for explaining relative movement between the arm and the holding part each of which has been stopped at an origin position;
FIG. 18 is a diagram for explaining a sample preparation part;
FIG. 19 is a diagram for explaining connection between each of reaction parts and a measurement part;
FIG. 20 is a block diagram showing a configuration regarding control of the specimen analyzer;
FIG. 21 is a flowchart showing the flow of a measurement operation for one specimen container;
FIG. 22 is a flowchart showing the flow of a process to be performed through agitating part control;
FIG. 23 is a diagram for explaining each of states of the agitating part;
FIG. 24 is a flowchart showing the flow of a process to be performed through container transfer part control;
FIG. 25 is a flowchart showing the flow of the process to be performed through the container transfer part control;
FIG. 26 is a diagram for explaining each of states of the container transfer part;
FIG. 27 is a flowchart showing the flow of a process to be performed through hand part control;
FIG. 28 is a flowchart showing the flow of a process to be performed through suction part control;
FIG. 29 is a flowchart showing the flow of a process to be performed through reading part control;
FIG. 30 is a time chart of agitation and suction operations (first container) in the specimen analyzer;
FIG. 31 is a time chart of agitation and suction operations ((N+1)^{th} container) in the specimen analyzer;
FIG. 32 is a diagram for explaining the positions of the agitating part and the container transfer part at a time point t4;
FIG. 33 is a diagram for explaining the positions of the agitating part and the container transfer part at a time point t6;
FIG. 34 is a diagram for explaining the positions of the agitating part and the container transfer part at a time point t8;
FIG. 35 is a diagram for explaining the positions of the agitating part and the container transfer part at a time point 114;
FIG. 36 is a diagram for explaining the positions of the agitating part and the container transfer part at a time point t17;
FIG. 37 is a diagram for explaining the positions of the agitating part and the container transfer part at a time point t20;
FIG. 38 is a diagram for explaining the positions of the agitating part and the container transfer part at a time point t25;
FIG. 39 is a diagram for explaining the positions of the agitating part and the container transfer part at a time point t28;
FIG. 40 is a diagram for explaining the positions of the agitating part and the container transfer part at a time point t35;
FIG. 41 is a diagram for explaining the positions of the agitating part and the container transfer part at a time point t40;
FIG. 42 is a diagram for explaining the positions of the agitating part and the container transfer part at a time point t45;
FIG. 43 is a diagram for explaining selection of reaction parts to each of which a specimen is ejected;
FIG. 44 is a diagram for explaining a first modification of the operation by the specimen analyzer;
FIG. 45 is a diagram for explaining a second modification of the operation by the specimen analyzer;
FIG. 46 is a diagram for explaining a third modification of the operation by the specimen analyzer;
FIG. 47 is a diagram for explaining a fourth modification of the operation by the specimen analyzer;
FIG. 48 is a diagram for explaining a fifth modification of the operation by the specimen analyzer;
FIG. 49 is a schematic diagram showing a specimen analyzer for performing agitation in a modification;
FIG. 50 is a schematic diagram showing a gripping/agitating part;
FIG. 51 is a schematic diagram showing a specimen analyzer in a sixth modification; and
FIG. 52 is a flowchart showing a measurement process for an interruption specimen to be executed by a controller of the specimen analyzer in the sixth modification.

### DETAILED DESCRIPTION

Hereinafter, an embodiment will be described with reference to the drawings.

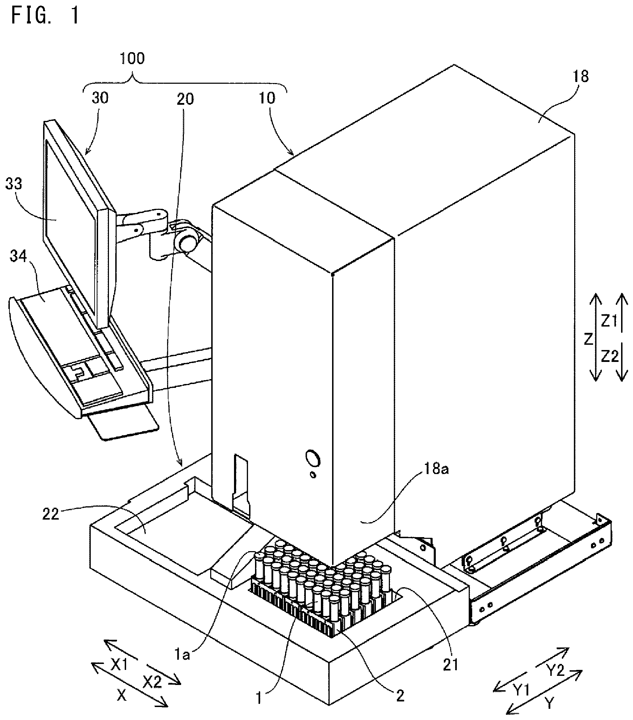

Firstly, an overall configuration of a specimen analyzer 100 will be described with reference to FIG. 1 to FIG. 20. In the present embodiment, an example in which the specimen analyzer 100 is a device for analyzing a blood specimen and is specifically a blood cell counter will be described.

As shown in FIG. 1, the specimen analyzer 100 includes: one measurement unit 10; a rack transport part 20 disposed on a front surface side (Y1 direction side) relative to the measurement unit 10; and an analysis part 30 implemented by a personal computer (PC) electrically connected to the measurement unit 10 and the rack transport part 20. In addition, the analysis part 30 is connected to a host computer 300 (see FIG. 4).

Hereinafter, two directions orthogonal to each other in a horizontal plane are defined as an X direction and a Y direction. In the X direction, a direction to one side and a direction to the other side are respectively defined as an X1 direction and an X2 direction. In the Y direction, a direction to one side and a direction to the other side are respectively defined as a Y1 direction and a Y2 direction. An up-down direction orthogonal to the X direction and the Y direction is defined as a Z direction, and an upward direction and a downward direction in the Z direction are respectively defined as a Z1 direction and a Z2 direction.

### (Rack Transport Part)

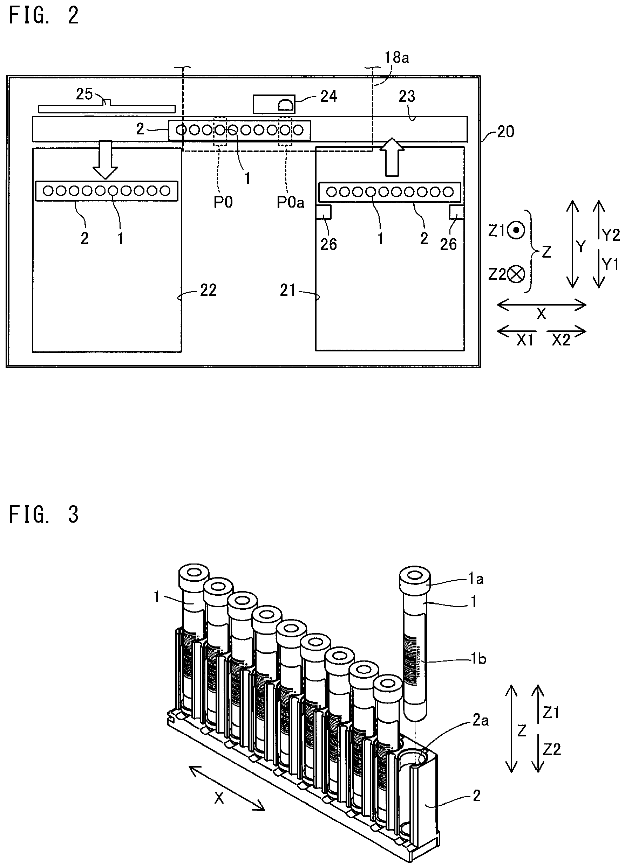

As shown in FIG. 1, the rack transport part 20 is configured to transport a rack 2 accommodating a plurality of specimen containers 1. As shown in FIG. 2, the rack transport part 20 includes: a pre-analysis rack holding part 21; a post-analysis rack holding part 22; a transport path 23 which linearly moves the rack 2 in the X1 direction and the X2 direction horizontally; an information reading part 24; and a rack send-out part 25 which moves the rack 2 into the post-analysis rack holding part 22.

As shown in FIG. 3, the rack 2 has the shape of a substantially rectangular parallelepiped. The rack 2 has a plurality of holding holes 2a extending downward from the upper surface thereof. One specimen container 1 is inserted into one of the holding holes 2a, whereby the specimen container 1 is held. The plurality of holding holes 2a are arranged in a linear pattern at intervals along the longitudinal direction of the rack 2.

Each of the specimen containers 1 is a cylindrical container that has a cylindrical shape and that has an opened upper end and a closed bottom portion. A specimen collected from a subject is contained in the specimen container 1. The specimen is a whole blood specimen. The specimen container 1 has an upper end portion to which a cap 1a for closing the opening is attached. A portion or the entirety of the cap 1a is made from a flexible material such as rubber, and the cap 1a can be pierced (penetrated) by a suction tube. A barcode label 1b indicating identification information for identifying the specimen container 1 is pasted on the side surface of the specimen container 1. The barcode label 1b may be a label indicating a two-dimensional code instead of a barcode.

As shown in FIG. 2, the pre-analysis rack holding part 21 is configured to be able to hold a plurality of the racks 2 each accommodating specimen containers 1 containing pre-analysis specimens. Each of the pre-analysis racks 2 is placed onto the pre-analysis rack holding part 21 by a user. The pre-analysis rack holding part 21 has rack sending parts 26 and is configured to, through movement of the rack sending parts 26 in the Y2 direction, push out the racks 2 held on the pre-analysis rack holding part 21 onto the transport path 23 one by one.

The transport path 23 extends in the X direction and is connected to the pre-analysis rack holding part 21 and the post-analysis rack holding part 22. The transport path 23 is configured to transport the specimen containers 1, which are held by each of the racks 2 received from the pre-analysis rack holding part 21, to a take-out position P0 at which the measurement unit 10 takes in a specimen. In addition, the transport path 23 is configured to, before transporting each of the specimen containers 1 to the take-out position P0, transport the specimen container 1 to a rack reading position P0a at which the information reading part 24 reads information. The information reading part 24 is a barcode reader that optically reads the barcode label 1b on each of the specimen containers 1 transported to the rack reading position P0a. The transport path 23 transports, to the rack send-out part 25, each of racks 2 holding specimen containers 1 having been subjected to suctioning of specimens.

The rack send-out part 25 is disposed so as to be opposed to the post-analysis rack holding part 22 with the transport path 23 interposed therebetween and is configured to move in the Y1 direction horizontally. In addition, the rack send-out part 25 is configured to, by moving in the Y1 direction horizontally, push out the rack 2 on the transport path 23 to the post-analysis rack holding part 22 side.

The post-analysis rack holding part 22 is configured to be able to hold a plurality of the racks 2 each accommodating specimen containers 1 containing post-analysis specimens. The post-analysis rack holding part 22 receives and accumulates the racks 2 sent out from a position on the transport path 23 by the rack send-out part 25. Each of the racks 2 holding the post-analysis specimen containers 1 is collected from the post-analysis rack holding part 22 by the user.

### (Measurement Unit)

As shown in FIG. 4, the measurement unit 10 includes a suction part 11, an agitating part 12, a container transport part 13, a reading part 14, a sample preparation part 15, a measurement part 16, and a controller 91. The suction part 11, the agitating part 12, the container transport part 13, the reading part 14, the sample preparation part 15, the measurement part 16, and the controller 91 are accommodated in a unit cover 18 (see FIG. 1).

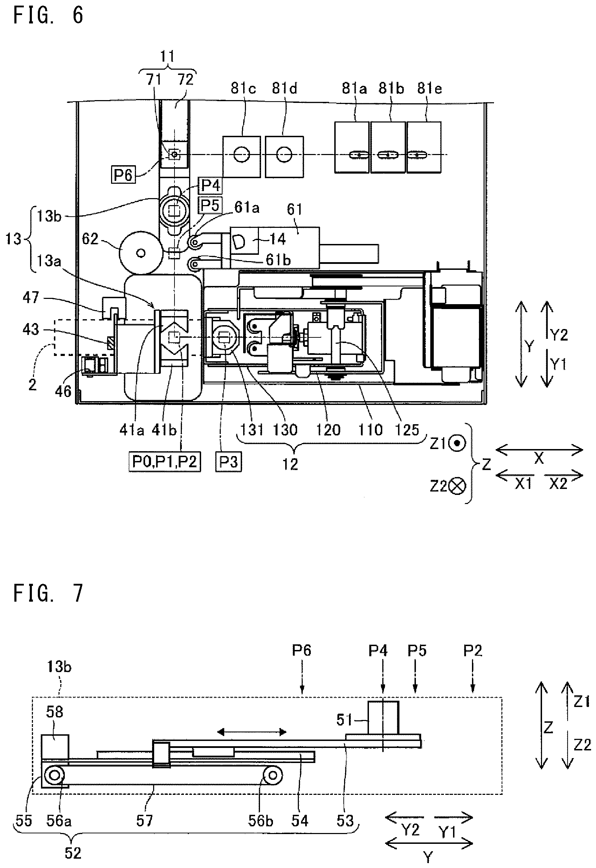

The suction part 11 is configured to suction a specimen from each of the specimen containers 1. The suction part 11 is disposed above a suction position P6 on the rear side (Y2 direction side) of the inside of the unit cover 18. The suction part 11 suctions the specimen from the specimen container 1 transported to the suction position P6 by the container transport part 13. The suction part 11 is configured to supply the suctioned specimen to reaction parts (81a to 81e; see FIG. 6) (described later) of the sample preparation part 15.

The agitating part 12 is configured to agitate the specimen in the specimen container 1. The agitating part 12 performs agitation processing for the specimen as a pretreatment that precedes suctioning of the specimen by the suction part 11. Particles sedimented in the specimen container 1 are dispersed in the liquid component of the specimen through the agitation processing. The agitating part 12 is disposed on the front side (Y1 direction side) of the inside of the unit cover 18. The agitating part 12 is configured to perform the agitation processing on the specimen container 1 relayed from the container transport part 13.

As shown in FIG. 1, the unit cover 18 is disposed at a position on the rear side (Y2 direction side) relative to the rack transport part 20, but a front surface portion on the Y1 side of the unit cover 18 protrudes to the Y1 side so as to be located upward (in the Z1 direction) of the rack transport part 20. The agitating part 12 is accommodated in a protruding portion 18a, protruding to the Y1 side, of the unit cover 18 and is disposed at a position (see FIG. 4) overlapping with the transport path 23 in a top view.

The container transport part 13 is configured to transport each of the specimen containers 1 between the rack 2 on the rack transport part 20, the agitating part 12, and the suction position P6 for specimen suctioning by the suction part 11. The container transport part 13 includes a hand part 13a and a container transfer part 13b.

The hand part 13a can be placed above the transport path 23 of the rack transport part 20 and is configured to grip each of the specimen containers 1 and move in the up-down direction. The hand part 13a can take out, from the rack 2 on the transport path 23, the specimen container 1 placed at the take-out position P0 among the specimen containers 1 held by the rack 2 and insert the specimen container 1 into the rack 2. The hand part 13a can insert the gripped specimen container 1 into the container transfer part 13b and take out the specimen container 1 from the container transfer part 13b. The hand part 13a can insert the gripped specimen container 1 into the agitating part 12 and take out the specimen container 1 from the agitating part 12. The operations of inserting and taking out the specimen container 1 will be described in detail later.

The container transfer part 13b is configured to receive, from the hand part 13a, the specimen container 1 having been agitated by the agitating part 12 and transfer the specimen container 1 to the suction position P6. The agitating part 12 is configured to receive, from the hand part 13a, the specimen container 1 having been taken out from the rack 2 and agitate the specimen in the specimen container 1. Thus, the hand part 13a can execute the operations of taking out a specimen container 1 from the rack 2, relaying the specimen container 1 to the container transfer part 13b, and relaying the specimen container 1 to the agitating part 12. Therefore, the device configuration can be simplified as compared to a case where these operations are executed by separate mechanisms.

The reading part 14 is configured to read identification information from the barcode label 1b (see FIG. 3) provided on the specimen container 1. The reading part 14 is provided so as to be adjacent to a reading position P5 on a movement path for movement by the container transfer part 13b. The reading part 14 is an optical barcode reader. The reading part 14 is configured to read the identification information on the specimen container 1 transferred to the reading position P5 by the container transfer part 13b.

The sample preparation part 15 prepares a sample, for detection, from the specimen suctioned by the suction part 11. The sample preparation part 15 is configured to prepare a measurement sample by reacting the specimen suctioned by the suction part 11 and a reagent with each other. The measurement sample prepared by the sample preparation part 15 is supplied to the measurement part 16.

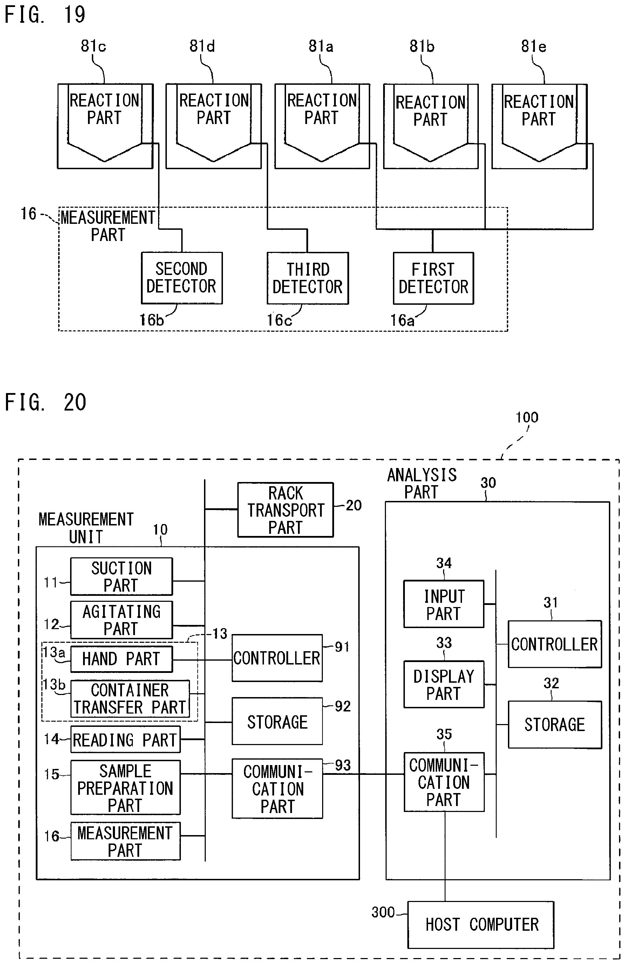

The measurement part 16 is configured to measure the measurement sample prepared from the specimen suctioned by the suction part 11. Information indicating presence of detection-target components contained in the measurement sample (i.e., specimen) is detected. The detection-target components include particles such as red blood cells (RBC), white blood cells (WBC), and platelets (PLT). In addition, the detection-target components include a blood pigment (specifically, hemoglobin (HGB)) in blood. Data of a measurement result obtained in the measurement part 16 is transmitted through a communication part 93 (FIG. 20) to the analysis part 30.

The controller 91 is configured to control the agitating part 12, the suction part 11, and the container transport part 13. In the present embodiment, the controller 91 controls not only the agitating part 12, the suction part 11, and the container transport part 13 but also each of parts (the measurement part 16, the reading part 14, the sample preparation part 15, and the like) accommodated in the measurement unit 10. In addition, the controller 91 transmits the data of the measurement result obtained in the measurement part 16, through the communication part 93 (FIG. 20) to the analysis part 30.

The analysis part 30 is configured to analyze the data of the measurement result obtained from the measurement unit 10, to obtain an analysis result (a red blood cell count, a platelet count, a hemoglobin amount, a white blood cell count, or the like) regarding each of measurement items.

### (Detailed Configuration of Measurement Unit)

Next, a detailed configuration of the measurement unit 10 will be described.

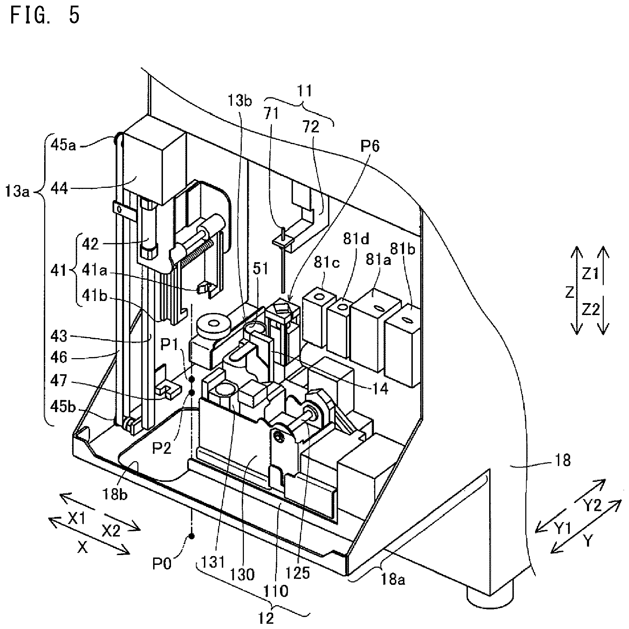

As shown in FIG. 5, the hand part 13a is disposed at a corner on the X1 direction side inside the protruding portion 18a of the unit cover 18. The hand part 13a is disposed above (see FIG. 4) the transport path 23 of the rack transport part 20. Specifically, the hand part 13a is disposed at a position directly above the take-out position P0 on the transport path 23.

The hand part 13a includes: a pair of gripping pieces 41a and 41b opposed to each other in a horizontal direction (Y direction); and an actuator 42 which actuates the pair of gripping pieces 41a and 41b so as to cause opening/closing therebetween. The actuator 42 is an air cylinder. The actuator 42 may be an electric motor. In addition, the hand part 13a includes a hand movement mechanism which moves the pair of gripping pieces 41a and 41b and the actuator 42 in the up-down direction. The hand movement mechanism includes: a guide rail 43 extending in the Z direction; a hand lifting/lowering motor 44; a pair of pulleys 45a and 45b; and an annular belt 46. A movable part 41 composed of the pair of gripping pieces 41a and 41b and the actuator 42 is slidably attached to the guide rail 43. The hand lifting/lowering motor 44 is a stepping motor. The hand lifting/lowering motor 44 rotationally drives the pulley 45a, to perform circulation drive of the belt 46 extended on and between the pulley 45a and the pulley 45b. The movable part 41 is connected to a portion of the belt 46 and is moved in the Z 1 direction and the Z2 direction along the guide rail 43 in association with circulation drive of the belt 46. The hand movement mechanism is a linear motion mechanism which moves the hand part 13a (movable part 41) in only the Z direction.

Inside the protruding portion 18a of the unit cover 18, an opening 18b is formed at a position immediately below the hand part 13a. The hand part 13a moves through the opening 18b in the downward direction (Z2 direction) to grip, by the pair of gripping pieces 41a and 41b, the specimen container 1 held by the rack 2 on the transport path 23 (the specimen container 1 placed at the take-out position P0). Then, the hand part 13a directly moves in the upward direction (Z1 direction) to take out the specimen container 1 from the rack 2. Meanwhile, the hand part 13a in a state of gripping the specimen container 1 moves through the opening 18b in the downward direction (Z2 direction) to insert the gripped specimen container 1 into the rack 2. Then, the hand part 13a releases the specimen container 1 being gripped by the pair of gripping pieces 41a and 41b and moves in the upward direction (Z1 direction) to return, to the rack 2, the specimen container 1 having been taken out. Relaying of a specimen container 1 with respect to the container transfer part 13b and the agitating part 12 is also performed at the respective height positions for the container transfer part 13b and the agitating part 12 through operations of gripping the specimen container 1 by the gripping pieces 41a and 41b and releasing the gripped specimen container 1.

The hand part 13a is provided with an origin sensor 47 which detects an origin position in the Z direction of the movable part 41. Control is performed so as to position the hand part 13a at each of: a position at which the specimen container 1 is taken out from and inserted (returned) into the rack 2 by the hand part 13a; a position at which the specimen container 1 is inserted into and taken out from the container transfer part 13b by the hand part 13a; a position at which the specimen container 1 is inserted into and taken out from the agitating part 12 by the hand part 13a; and the like. This control is performed by the controller 91 (see FIG. 4) on the basis of the distance from the origin position to said position.

As shown in FIG. 6, the container transfer part 13b is disposed away from the hand part 13a in the Y2 direction. As shown in FIG. 7, the container transfer part 13b includes a container holding part 51 and a holding part movement mechanism 52. The container holding part 51 is a cylindrical member having a diameter corresponding to the outer diameter of each specimen container 1 and has an opened upper end portion and a closed bottom portion. The container holding part 51 receives the bottom portion of the specimen container 1 inside the cylindrical container holding part 51, to hold the specimen container 1 from the lower side. The holding part movement mechanism 52 is configured to linearly move the container holding part 51 in the Y1 direction and the Y2 direction. The holding part movement mechanism 52 is a linear motion mechanism which moves the container holding part 51 in only the Y direction. The holding part movement mechanism 52 includes: a support plate 53 supporting the lower surface of the container holding part 51; a guide rail 54 extending in the Y direction; a transfer-part motor 55; a pair of pulleys 56a and 56b; and an annular belt 57. The support plate 53 on which the container holding part 51 is provided is slidably attached to the guide rail 54. The transfer-part motor 55 rotationally drives the pulley 56a, to perform circulation drive of the belt 57 extended on and between the pulley 56a and the pulley 56b. The support plate 53 is connected to a portion of the belt 57 and is moved in the Y1 direction and the Y2 direction along the guide rail 54 in association with circulation drive of the belt 57.

Consequently, the container transfer part 13b linearly moves the container holding part 51 in the Y direction as shown in FIG. 7. The container transfer part 13b moves the container holding part 51 in the Y1 direction and the Y2 direction, to transfer the specimen container 1 held by the container holding part 51. Specifically, the container transfer part 13b is configured to move the specimen container 1 held by the container holding part 51 to the suction position P6, a second position P2, a fourth position P4, and the reading position P5. These positions are arranged in the order of the second position P2, the reading position P5, the fourth position P4, and the suction position P6 from the Y1 direction side.

The second position P2 is a position at which the hand part 13a executes insertion and taking-out of the specimen container 1 with respect to the container transfer part 13b. That is, in the top view, the second position P2 coincides with the take-out position P0 (see FIG. 5) for taking out the specimen container 1 from the rack 2 on the transport path 23. The second position P2 is a position on the upper side (Z1 side) relative to the take-out position P0. The reading position P5 is a position at which the reading part 14 reads identification information from the specimen container 1. The fourth position P4 is a shunt position separated in a horizontal direction (XY direction) from the agitating part 12 present at a first position P1. The suction position P6 is a position at which the suction part 11 suctions the specimen in the specimen container 1 held by the container holding part 51.

As shown in FIG. 7, the container transfer part 13b is provided with an origin sensor 58 which detects an origin position in the Y direction of the container holding part 51. Control is performed so as to position the container transfer part 13b at each of the second position P2, the reading position P5, the fourth position P4, and the suction position P6. This control is performed by the controller 91 (see FIG. 4) on the basis of the distance from the origin position to said position. Hereinafter, movement of the container holding part 51 of the container transfer part 13b to any of the above positions is sometimes expressed simply as movement of the container transfer part 13b. The position of the container transfer part 13b is based on a position at which the specimen container 1 is held by the container holding part 51 (the center position of the cylindrical container holding part 51).

As shown in FIG. 6, the reading part 14 includes a rotation part 61 which rotates, in a circumferential direction, the specimen container 1 held by the container holding part 51. The rotation part 61 advances a pair of support rollers 61a and 61b toward a drive roller 62 such that the specimen container 1 is sandwiched between the drive roller 62 and the pair of support rollers 61a and 61b. In this state, the rotation part 61 rotates the drive roller 62, to rotate the specimen container 1 in the circumferential direction (about the central axis line of the specimen container 1). The reading part 14 is disposed on the X2 side relative to the reading position P5 and reads identification information from the barcode label 1b (see FIG. 3) on the specimen container 1 rotated by the rotation part 61. Consequently, the identification information can be read by the reading part 14 regardless of the position in the circumferential direction of the barcode label 1b.

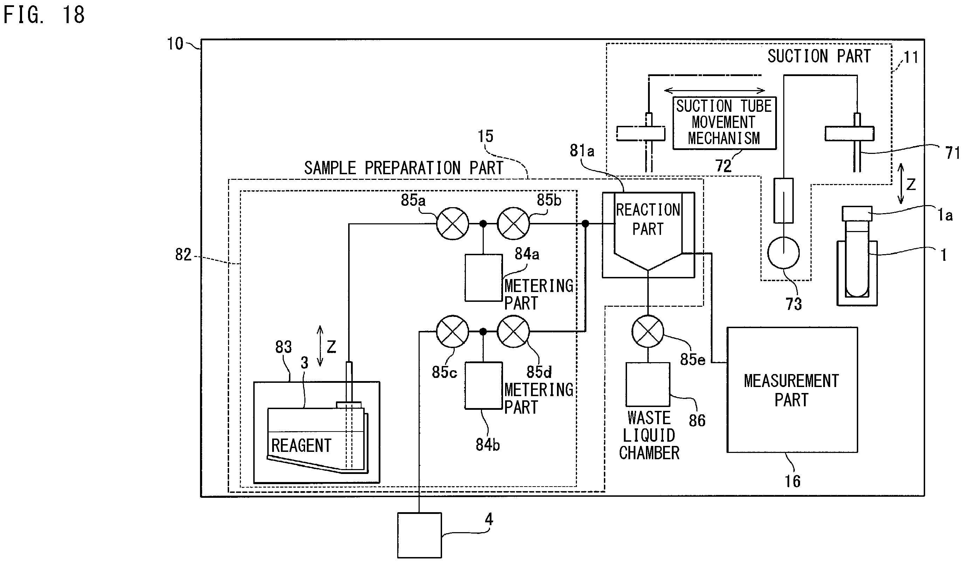

As shown in FIG. 5, a suction tube 71 of the suction part 11 is disposed above the suction position P6. The suction part 11 includes: the suction tube 71 extending in the Z direction; a suction tube movement mechanism 72 which moves the suction tube 71; and a metering part 73 (see FIG. 18). The suction tube 71 is a tube member formed such that the tip thereof can penetrate (pierce) the cap 1a of the specimen container 1. The suction tube movement mechanism 72 can move the suction tube 71 in the Z direction and the X direction. The suction part 11 moves the suction tube 71 in the downward direction from above the specimen container 1 placed at the suction position P6 (see FIG. 6). Consequently, the suction tube 71 penetrates the cap 1a (see FIG. 3) of the specimen container 1 such that the tip portion of the suction tube 71 is inserted into the specimen container 1.

As shown in FIG. 6, the plurality of reaction parts 81a to 81e of the sample preparation part 15 are arranged, in a linear pattern, away from the suction position P6 in the X2 direction. After suctioning a specimen at the suction position P6, the suction part 11 moves the suction tube 71 to one or more positions among the positions of the plurality of reaction parts 81a to 81e by the suction tube movement mechanism 72 and ejects the specimen. Consequently, the suctioned specimen is supplied to the sample preparation part 15.

The agitating part 12 is disposed away from the hand part 13a in the X2 direction. The agitating part 12 has a holding part 130 for holding the specimen container 1 and can move the holding part 130 in the X1 direction and the X2 direction. The agitating part 12 is configured to linearly move to the first position P1 and a third position P3 along a first direction (X direction) in the horizontal plane. The position of the agitating part 12 is based on a position at which the specimen container 1 is held by the holding part 130 (the center position of a holding hole 131a of a holding member 131 described later).

The first position P1 is a position at which the hand part 13a executes insertion and taking-out of the specimen container 1 with respect to the agitating part 12 (holding part 130). That is, in the top view, the first position P1 coincides with the take-out position P0 (see FIG. 5) for the specimen container 1. In the top view, the first position P1 coincides with the second position P2. The first position P1, the second position P2, and the take-out position P0 coincide with one another in an XY plane and differ from one another in the Z direction.

In this manner, the agitating part 12 is configured to linearly move to the first position P1 and the third position P3 along the first direction (X direction) in the horizontal plane, and the container transfer part 13b is configured to linearly move to the second position P2 and the fourth position P4 along a second direction (Y direction) intersecting with the first direction (X direction) in the top view. Further, the first position P1 and the second position P2 are each a position at which a movement path of the container transfer part 13b and a movement path of the agitating part 12 intersect with each other in the top view. Consequently, the specimen container 1 can be relayed and retracted merely by causing linear reciprocating movement of each of the agitating part 12 and the container transfer part 13b. Thus, the structure for moving the agitating part 12 and the container transfer part 13b can be simplified. In addition, in the case of a simple linear reciprocating movement as in the present embodiment, the mechanisms related to the movement easily lead to obtainment of rigidity and accuracy, and the operation speed can be set to be high, whereby the processing time can be shortened owing to the increased operation speed.

Therefore, in the present embodiment, the hand part 13a is configured to execute, at the first position P1, insertion and taking-out of the specimen container 1 with respect to the agitating part 12 and execute, at the second position P2, insertion and taking-out of the specimen container 1 with respect to the container transfer part 13b. Consequently, the hand part 13a can execute, at positions (the first position P1 and the second position P2) coinciding with each other in the top view, insertion and taking-out of the specimen container 1 with respect to the agitating part 12 and insertion and taking-out of the specimen container 1 with respect to the container transfer part 13b merely through upward/downward movement. Thus, the movement range of the hand part 13a can be made small, whereby the structure of the hand part 13a can be simplified. In addition, since the movement range of the hand part 13a is made small, the times required for insertion and taking-out of the specimen container 1 with respect to the agitating part 12 and the container transfer part 13b can be shortened.

In addition, the hand part 13a is configured to perform taking-out and insertion of the specimen container 1 with respect to the rack 2, insertion and taking-out of the specimen container 1 with respect to the agitating part 12 placed at the first position P1, and insertion and taking-out of the specimen container 1 with respect to the container transfer part 13b placed at the second position P2, through upward/downward movement without moving in the horizontal direction. Consequently, the hand part 13a only has to move upward/downward, and thus no mechanism for moving the hand part 13a in the horizontal direction needs to be provided. As a result, the structure of the hand part 13a can be effectively simplified.

The third position P3 is a position at which the agitating part 12 executes agitation processing on the specimen container 1. Third position P3 is also a shunt position separated in the horizontal direction (XY direction) from the container transfer part 13b present at the second position P2. That is, in the present embodiment, the first position P1 for the agitating part 12 and the second position P2 for the container transfer part 13b are set to positions at which the agitating part 12 and the container transfer part 13b interfere (i.e., come into contact) with each other. To prevent this interference, control by the controller 91 causes the agitating part 12 to be placed at the third position P3 while the container transfer part 13b (container holding part 51) is present at the second position P2 or the reading position P5. Meanwhile, control by the controller 91 causes the container transfer part 13b to be placed at the fourth position P4 or the suction position P6 while the agitating part 12 is present at the first position P1.

### (Detailed Configuration of Agitating Part)

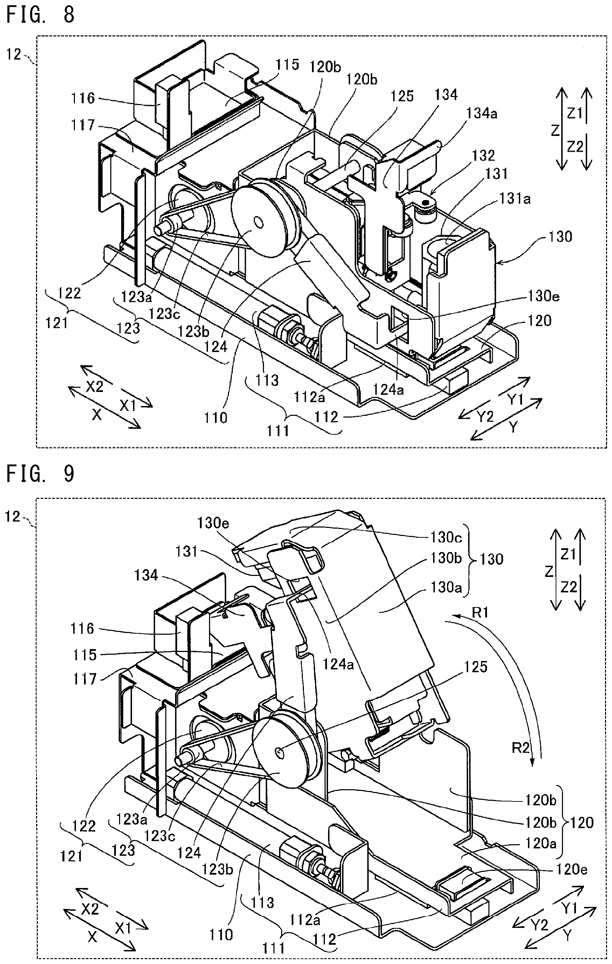

FIG. 8 is a perspective view for explaining the agitating part 12. The agitating part 12 is mainly composed of three parts which are a base part 110, a slide part 120, and the holding part 130. The slide part 120 is disposed on the base part 110 so as to be movable in the X direction, and the holding part 130 is rotatably disposed on the slide part 120. FIG. 9 shows a state where the holding part 130 is rotated.

### <Base Part>

As shown in FIG. 8, the base part 110 is a support plate supporting the entirety of the agitating part 12 and is fixed to the unit cover 18 (see FIG. 5) of the measurement unit 10. The agitating part 12 includes, on the base part 110, a linear motion mechanism 111 and a fall-off object reception part 115. The fall-off object reception part 115 will be described later.

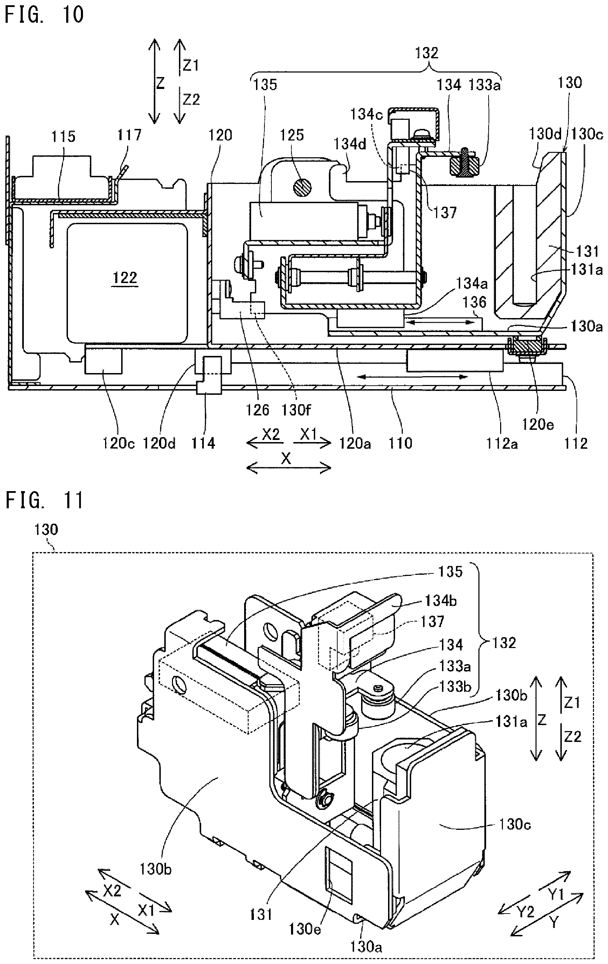

The linear motion mechanism 111 is configured to linearly move the slide part 120 in the X direction. The linear motion mechanism 111 includes a guide rail 112 and a slide drive part 113 which are provided on the base part 110 and which extend in the X direction. The slide part 120 is movably attached on the guide rail 112 via a slider block 112a (see FIG. 10). The slide drive part 113 applies drive force to the slide part 120, to move the slide part 120 in the X direction along the guide rail 112. The slide drive part 113 is an air cylinder and is configured to cause a cylinder fixed to the base part 110 to advance/retract, in the X direction, a rod connected to the slide part 120. The linear motion mechanism 111 causes advancing/retracting movement of the slide part 120 in the X direction, to move the holding part 130 disposed on the slide part 120 to the first position P1 (see FIG. 6) and the third position P3 (see FIG. 6).

As shown in FIG. 10, the base part 110 is provided with a slide detector 114. The slide detector 114 detects the position of the slide part 120. The slide detector 114 is a transmission-type photoelectric sensor and detects detection pieces provided to the slide part 120. The slide part 120 is provided with detection pieces 120c and 120d arranged at an interval in the X direction. The interval between the detection piece 120c and the detection piece 120d is equal to the interval (distance) between the first position P1 and the third position P3. The slide detector 114 detects the slide part 120 present at the first position P1 through blockage of light by the detection piece 120c. The slide detector 114 detects the slide part 120 present at the third position P3 through blockage of light by the detection piece 120d. No light blockage member is provided between the detection piece 120c and the detection piece 120d. Therefore, in a state where light is blocked by neither the detection piece 120c nor the detection piece 120d, the slide detector 114 detects that the slide part 120 is placed at neither the first position P1 nor the third position P3. Consequently, whether slide movement of the slide part 120 by the linear motion mechanism 111 has been normally executed is ascertained. The controller 91 (see FIG. 4) controls the agitating part 12 (holding part 130) to move to the first position P1 and the third position P3 on the basis of a detection signal from the slide detector 114.

### <Slide Part>

The slide part 120 is an intermediate support plate supporting the holding part 130 and a drive part 121 which drives the holding part 130. As shown in FIG. 9, the slide part 120 has: a bottom surface portion 120a provided with the slider block 112a; and a pair of side plate portions 120b raised upward from both respective ends in the Y direction of the bottom surface portion. One end and the other end of a rotation shaft 125 (see FIG. 8) by which the holding part 130 is swingably supported are supported by the pair of side plate portions 120b such that the rotation shaft 125 is rotatable. The rotation shaft 125 extends in a predetermined direction (Y direction) in the horizontal plane and supports a portion, of the holding part 130, near the X2-side end such that the holding part 130 is rotatable.

As shown in FIG. 8, on the slide part 120, the holding part 130 is located at a position on the X1 side, and the drive part 121 is located on the X2 side relative to the holding part 130. The slide part 120 includes a support member 120e (see FIG. 9) supporting a bottom surface portion 130a of the holding part 130 from below.

The drive part 121 has an electric rotation motor 122 and a transmission mechanism 123. The transmission mechanism 123 has: a pulley 123a attached to an output shaft of the rotation motor 122; a pulley 123b attached to the rotation shaft 125; an annular belt 123c wound on the pulley 123a and the pulley 123b; and an arm 124 provided so as to rotate together with the pulley 123b around the rotation shaft 125. The rotation motor 122 rotates the pulley 123a, to rotate the pulley 123b via the annular belt 123c. The arm 124 has one end portion provided so as to be rotatable around the rotation shaft 125. The arm 124 has another end portion provided with an engagement protrusion 124a inserted into an engagement hole 130e of the holding part 130. When the arm 124 is rotated around the rotation shaft 125 in association with rotation of the pulley 123b, the engagement protrusion 124a in the engagement hole 130e is brought into contact with the holding part 130 (i.e., an inner peripheral edge of the engagement hole 130e) so as to move the holding part 130. As a result, the holding part 130 is rotated (see FIG. 9) around the rotation shaft 125.

Consequently, as shown in FIG. 9, the drive part 121 can drive the arm 124 to one side in the rotation direction such that the holding part 130 is rotated anticlockwise (in an R1 direction) and can drive the arm 124 to the other side in the rotation direction such that the holding part 130 having been rotated in the R1 direction is rotated clockwise (in an R2 direction).

### <Holding Part>

As shown in FIG. 11, the holding part 130 includes the holding member 131 and a fixation part 132. The holding part 130 has the bottom surface portion 130a (see FIG. 9), a pair of side surface portions 130b, and a front surface portion 130c. The holding member 131 and the fixation part 132 are provided on the inner side of the holding part 130. The upper side of the holding part 130 is opened. As shown in FIG. 8 and FIG. 9, the holding part 130 is, at portions thereof near the X2-side ends of the pair of side surface portions 130b, rotatably attached to the rotation shaft 125.

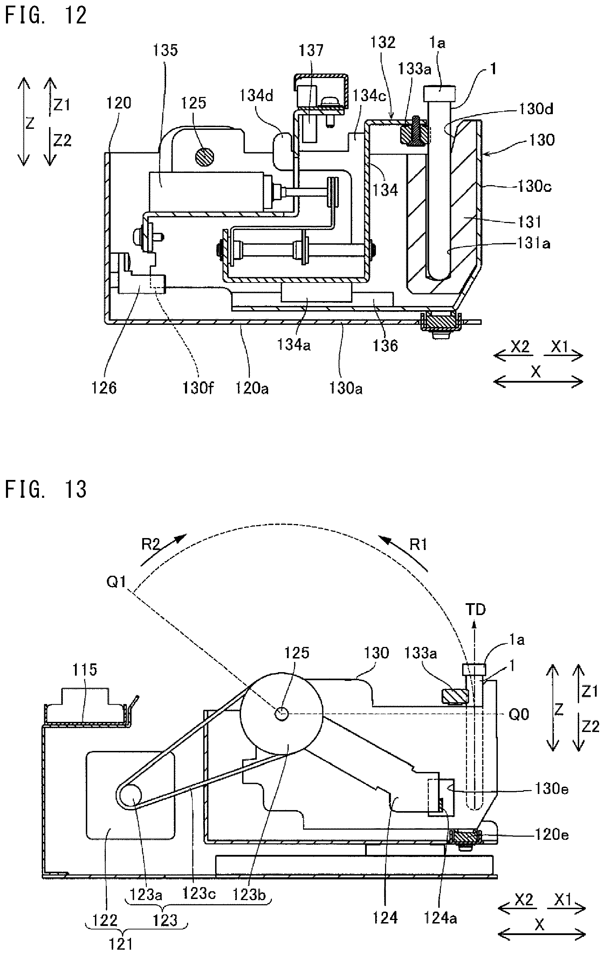

The holding member 131 is provided so as to be adjacent to the front surface portion 130c on the X1 side of the holding part 130. The holding member 131 has the holding hole 131a (see FIG. 10) extending from the upper surface thereof to a portion near the bottom thereof. The specimen container 1 can be inserted into the holding hole 131a. The holding part 130 is configured to receive, in the holding hole 131a, the specimen container 1 so as to hold a lower portion and a circumferential side surface of the specimen container 1. The height of the holding member 131 (the depth of the holding hole 131a) is lower than the height of the specimen container 1 such that an upper portion of the specimen container 1 inserted into the holding hole 131a is exposed (see FIG. 12) to the upper side from the holding hole 131a.

The fixation part 132 is configured to fix the specimen container 1 placed in the holding part 130 and release the fixed specimen container 1. Specifically, the fixation part 132 has: a pair of (two) contact members 133a and 133b having the shapes of rollers; a support part 134 supporting the contact members 133a and 133b; and a fixation drive part 135 which moves the support part 134 in the X direction to a fixation position and a release position.

The pair of contact members 133a and 133b are supported at X2-side positions relative to the holding member 131 by the support part 134 configured to be linearly movable in the X direction. The pair of contact members 133a and 133b are provided at positions opposed, in the X direction, to the side surface of the specimen container 1 supported by the holding member 131. As shown in FIG. 10, the support part 134 has a slider block 134a which slides on a fixation part rail 136 provided on the bottom surface portion 130a of the holding part 130. The fixation part rail 136 extends in the X direction inside the holding part 130. The fixation drive part 135 is an air cylinder having: a cylinder part fixed to the holding part 130; and a rod part connected to the support part 134.

The fixation drive part 135 advances the rod part in the X1 direction, to move the support part 134 and the contact members 133a and 133b in the X1 direction toward the holding member 131 (i.e., specimen container 1). As shown in FIG. 12, the contact members 133a and 133b reach, through the movement in the X1 direction, the fixation position at which the contact members 133a and 133b come into contact with the side surface of the specimen container 1. A contact surface 130d (see FIG. 10) is provided at a position, on the X1 side of the holding member 131, that faces the contact members 133a and 133b in the X direction. The fixation drive part 135 maintains, at the fixation position, a drive force for advancing the rod part so as to press (urge) the specimen container 1 against the contact surface 130d in the X1 direction. By being thus pressed against the contact surface 130d, the specimen container 1 is fixed to the holding part 130. The specimen container 1 is fixed so as to be in contact with three points, i.e., the pair of contact members 133a and 133b and the contact surface 130d.

The fixation drive part 135 retracts the rod part in the X2 direction, to move the support part 134 and the contact members 133a and 133b in the X2 direction and retract each of the contact members 133a and 133b to the release position (the position shown in FIG. 10) away from the specimen container 1. Consequently, the specimen container 1 having been fixed is released. In the state of being released, the specimen container 1 can be inserted into the holding hole 131a of the holding member 131 and taken out from inside the holding hole 131a.

As shown in FIG. 11, the support part 134 is provided with a fall-off prevention part 134b. The fall-off prevention part 134b is disposed at a position that is above the pair of contact members 133a and 133b and that is slightly away therefrom to the X1 side. Therefore, when the fixation part 132 enters a fixation state where the specimen container 1 is fixed, the fall-off prevention part 134b is located above the fixed specimen container 1 and is opposed, in the Z direction, to the upper surface (cap 1a) of the specimen container 1. Consequently, should the specimen container 1 in the fixation state start to come out of the holding member 131 during agitation by the agitating part 12, the cap 1a of the specimen container 1 comes into contact with the fall-off prevention part 134b, whereby the specimen container 1 can be prevented from falling off.

In addition, the holding part 130 is provided with a container detector 137. As shown in FIG. 10, the container detector 137 is a transmission-type photoelectric sensor and detects detection pieces provided to the support part 134. The support part 134 is provided with detection pieces 134c and 134d arranged at an interval in the X direction. When the fixation drive part 135 is in a retraction state, the container detector 137 detects the support part 134 present at the release position through blockage of light by the detection piece 134c. No light blockage member is provided between the detection piece 134c and the detection piece 134d. When the support part 134 is in the fixation position as shown in FIG. 12, the container detector 137 detects a non-light-blocking section between the detection piece 134c and the detection piece 134d. Therefore, in a state where light is blocked by neither the detection piece 134c nor the detection piece 134d, the container detector 137 detects that: the specimen container 1 is held by the holding member 131; and the fixation part 132 is in the fixation state. Meanwhile, when the support part 134 is moved in the X1 direction toward the fixation position in a state where no specimen container 1 is held by the holding member 131, the contact members 133a and 133b pass through the fixation position to reach a movement limit in the X1 direction. At the movement limit on the X1 side, the detection piece 134d blocks light of the container detector 137. Therefore, the container detector 137 detects that no specimen container 1 is held by the holding member 131, on the basis of the fact that: the fixation drive part 135 is in an advancement state; and the container detector 137 is in a light-blocked state. The controller 91 (see FIG. 4) controls the fixation part 132 to fix and release the specimen container 1 on the basis of a detection signal from the container detector 137. In addition, the controller 91 (see FIG. 4) determines, on the basis of the detection signal from the container detector 137, whether or not a specimen container 1 is present in the agitating part 12, i.e., whether or not a specimen container 1 has been relayed by the hand part 13a.

### <Configuration and Operation of Agitating Part>

As described above, the agitating part 12 includes: the holding part 130 which holds the lower portion of each of the specimen containers 1; and the drive part 121 which causes reciprocating movement of the holding part 130 in the predetermined direction. The drive part 121 causes reciprocating movement of the holding part 130 in the predetermined direction (the R1 direction and the R2 direction), whereby the agitating part 12 performs agitation. Consequently, it is possible to shake and agitate the specimen through the reciprocating movement while stably holding the specimen container 1 from below.

As shown in FIG. 13, the drive part 121 causes reciprocating rotation of the holding part 130 around the rotation shaft 125. In addition, the holding part 130 is configured to hold the specimen container 1 oriented such that the longitudinal direction of the specimen container 1 extends along a tangent line direction to a rotation trajectory around the rotation shaft 125. As described above, the holding part 130 holds the specimen container 1 oriented such that the longitudinal direction (i.e., central axis line) of the specimen container 1 extends along the Z direction inside the holding member 131 (see FIG. 12). The trajectory of the holding member 131 is an in-XZ-plane rotation trajectory having a center at the rotation shaft 125 and having a radius which is the distance from the rotation shaft 125 to the holding member 131. Therefore, the specimen container 1 is subjected to reciprocating rotation in the R1 direction and the R2 direction, in a state of being oriented in the tangent line direction TD to the rotation trajectory. Consequently, through the reciprocating rotation in a state where the specimen container 1 is held such that the longitudinal direction thereof extends along the tangent line direction TD to the rotation trajectory, the specimen is moved between the bottom portion and the upper end portion (cap 1a) of the specimen container 1, whereby the specimen can be effectively agitated. Further, the specimen container 1 can be held in the holding part 130 by the fixation part 132 (see FIG. 12) also when the specimen container 1 is subjected to reciprocating rotation in the tangent line direction TD to the rotation trajectory.

In addition, the drive part 121 is configured to cause reciprocating rotation of the holding part 130 around the rotation shaft 125 extending in the horizontal direction (Y direction). The holding part 130 is configured to hold the lower portion of the specimen container 1 oriented so as to extend along the up-down direction at an origin position Q0 for the reciprocating rotation. The holding part 130 is rotated around the rotation shaft 125 extending in the horizontal direction, and thus, can more effectively agitate the specimen by utilizing the effect of gravity at the time of reciprocating rotation.

The controller 91 is configured to control the drive part 121 to rotate the holding part 130 from the origin position Q0 to a predetermined angle Q1 larger than 90 degrees. As shown in FIG. 13, the holding part 130 is oriented horizontally (such that the specimen container 1 stands vertically) at the origin position Q0 by being supported by the support member 120e of the slide part 120. With the origin position Q0 being the origin (0 degrees) for rotation angles, the holding part 130 is rotated in the R1 direction to the predetermined angle Q1. The predetermined angle Q1 is about 140 degrees. When the rotation angle reaches the predetermined angle Q1, the rotation of the holding part 130 is stopped, and then the holding part 130 is rotated in the opposite direction (R2 direction) to the origin position Q0. When the rotation angle reaches the origin position Q0, the rotation in the R2 direction is stopped.

At the time of agitation, the controller 91 causes reciprocating rotation composed of: rotation in the R1 direction from the origin position Q0 to the predetermined angle Q1; and rotation in the R2 direction from the predetermined angle Q1 to the origin position Q0. The reciprocating rotation is repetitively executed a predetermined number of times. The predetermined number is, for example, eight.

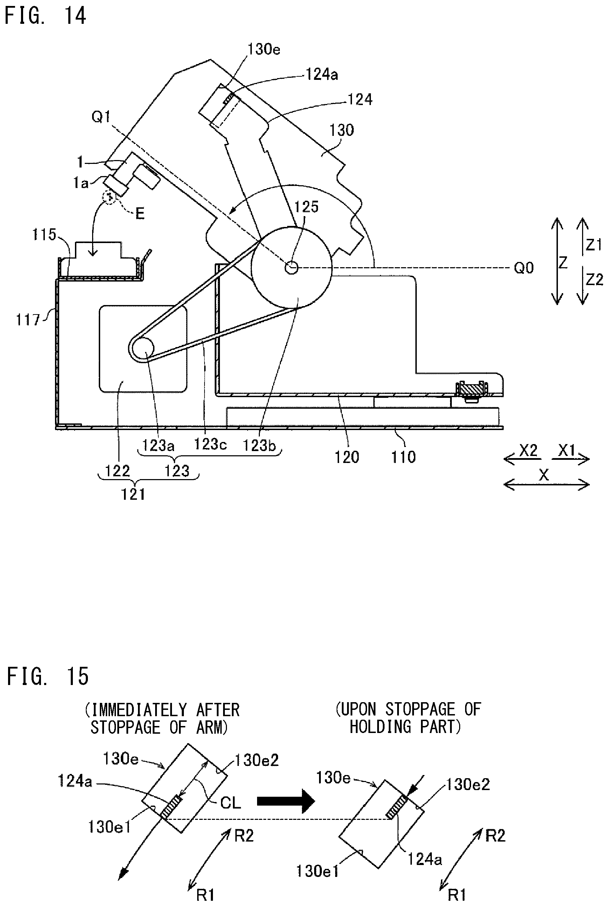

Here, as shown in FIG. 14, the fall-off object reception part 115 is disposed at a position below (in the Z2 direction) the upper end portion (cap 1a) of the specimen container 1 in a state of having been rotated to the predetermined angle Q1. Specifically, the fall-off object reception part 115 is disposed on a mount 117 formed, near the X2-side end of the base part 110, at a position above the rotation motor 122 of the slide part 120. The fall-off object reception part 115 has the shape of a tray having: a relatively wide bottom surface; and a peripheral side surface raised from the outer periphery of the bottom surface. The fall-off object reception part 115 is configured to be detachable from and re-attachable to the mount 117 of the base part 110. Consequently, the fall-off object reception part 115 has a function of receiving adhesion matter having fallen off from the specimen container 1.

Specifically, the predetermined angle Q1 is larger than 90 degrees, and thus, when the holding part 130 reaches the predetermined angle Q1, the specimen container 1 is stopped in such an orientation as to be obliquely tilted downward such that the cap 1a at the upper end portion is at a lower position than the bottom portion of the specimen container 1. At this time, the fall-off object reception part 115 is present below the cap 1a. Therefore, adhesion matter E adhered on the upper surface of the cap 1a falls off from the cap 1a onto the fall-off object reception part 115 owing to the effect of inertia exhibited when the rotation is stopped.

Thus, the adhesion matter E adhered when the specimen has been contained in the specimen container 1 can be shaken off from the specimen container 1 onto the fall-off object reception part 115 by utilizing the agitation operation. The adhesion matter E is, for example, powdered blood resulting from coagulation of a blood specimen. Consequently, when the specimen is suctioned from the specimen container 1 having been agitated, a minute amount of the adhesion matter E can be inhibited from adhering on the suction tube or from entering the specimen that is suctioned. In the present embodiment, the fall-off object reception part 115 is configured to be detachable and re-attachable. Thus, at the time of regular maintenance or the like, the fall-off object reception part 115 can be detached from the mount 117, and the adhesion matter E received on the fall-off object reception part 115 can be removed, whereby workability in maintenance is excellent.

In order to ascertain the rotational position of the holding part 130, the agitating part 12 is provided with: an origin position detector 126 (see FIG. 10) for detecting that the holding part 130 is at the origin position Q0; and an end-point position detector 116 (see FIG. 8) for detecting that the holding part 130 has reached the predetermined angle Q1.

As shown in FIG. 10, the origin position detector 126 is a transmission-type photoelectric sensor and is provided to the slide part 120. When the holding part 130 is at the origin position Q0, light of the origin position detector 126 is blocked by detection pieces 130f provided as portions of the side surface portions 130b of the holding part 130. Consequently, the origin position detector 126 detects, in a light-blocked state thereof, that the holding part 130 is at the origin position Q0. When the holding part 130 is rotated in the R1 direction from the origin position Q0, the detection pieces 130f move away from the origin position detector 126. Therefore, the origin position detector 126 detects, in a non-light-blocked state thereof, that the holding part 130 is at a position other than the origin position Q0. The controller 91 performs control to stop the holding part 130 at the origin position Q0 on the basis of a detection result from the origin position detector 126.

As shown in FIG. 8, the end-point position detector 116 is a reflection-type photoelectric sensor and is provided on the mount 117 of the base part 110, i.e., near the fall-off object reception part 115. As shown in FIG. 9, the end-point position detector 116 is disposed on the Y2 side relative to the position that the holding part 130 reaches at the predetermined angle Q1. The end-point position detector 116 emits detection light in the Y1 direction. When the holding part 130 is rotated to the predetermined angle Q1, the holding part 130 (strictly speaking, the support part 134) is placed on an optical path of the detection light, whereby the end-point position detector 116 detects reflected light from the holding part 130. Consequently, in a state where the end-point position detector 116 has received the reflected light (in a state where the intensity of received light is higher than a threshold value), the end-point position detector 116 detects that the holding part 130 is at the predetermined angle Q1. In a case where the holding part 130 is not at the predetermined angle Q1, the detection light advances straight in the Y1 direction without being reflected. In a state where the end-point position detector 116 has not received any reflected light from the holding part 130 (in a state where the intensity of the received light is lower than the threshold value), the end-point position detector 116 detects that the holding part 130 is at an angle other than the predetermined angle Q1. The controller 91 performs control to stop the holding part 130 at the predetermined angle Q1 on the basis of a detection result from the end-point position detector 116.

Here, the agitating part 12 is configured to abruptly stop rotation of the holding part 130 when the holding part 130 reaches the predetermined angle Q1 from the origin position Q0. Consequently, the specimen in the specimen container 1 can be effectively agitated. Further, the adhesion matter E adhered on the specimen container 1 can be effectively caused to fall off from the specimen container 1 owing to inertia.

Specifically, in the agitation operation, the controller 91 controls the drive part 121 to stop rotation of the arm 124 and stop the holding part 130 at the predetermined angle Q1 when it is detected that the holding part 130 has reached the predetermined angle Q1 from the origin position Q0 as shown in FIG. 14.

Here, the arm 124 is connected to the holding part 130 in a state of allowing relative movement in the rotation direction by a predetermined amount CL (see FIG. 15). As shown in FIG. 15, a gap of the predetermined amount CL is formed between the inner peripheral edge of the engagement hole 130e formed in the holding part 130 and the engagement protrusion 124a of the arm 124. That is, the engagement hole 130e is formed so as to be larger than the external shape of the engagement protrusion 124a. The engagement hole 130e is a through-hole (see FIG. 11) having a rectangular shape and formed in either of the side surface portions 130b of the holding part 130. The engagement protrusion 124a is an elongated flat-plate-shaped protrusion extending along the longitudinal direction of the engagement hole 130e. Therefore, the holding part 130 is engaged with the arm 124 in a state where there is the gap of the predetermined amount CL.

When the holding part 130 is rotated in the R1 direction from the origin position Q0 to the predetermined angle Q1, the engagement protrusion 124a of the arm 124 comes into contact with an inner peripheral edge 130e1 on the R1 side of the engagement hole 130e so as to press the holding part 130, whereby the holding part 130 is rotated in the R1 direction. When the holding part 130 reaches the predetermined angle Q1 and rotation of the arm 124 is stopped, the holding part 130 continues to rotate by the gap of the predetermined amount CL owing to inertia. An inner peripheral edge 130e2 on the R2 side of the engagement hole 130e comes into contact (collides) with the engagement protrusion 124a, whereby the holding part 130 is stopped. In this manner, when the arm 124 rotated to the predetermined angle Q1 is stopped, the holding part 130 moves by the predetermined amount CL and collides with the arm 124 (engagement protrusion 124a) owing to inertia, to be abruptly stopped.

Consequently, as compared to a configuration in which the holding part 130 is stopped by merely stopping rotation of the rotation motor 122 of the drive part 121, a larger extent of deceleration (negative acceleration) can be applied to the holding part 130, whereby the adhesion matter E adhered on the specimen container 1 can be effectively removed. In addition, an operation of abruptly stopping the holding part 130 can be realized by utilizing coupling portions (the engagement protrusion 124a and the engagement hole 130e) of the arm 124 and the holding part 130. Thus, the configuration for abruptly stopping the holding part 130 can be realized without increasing the number of components of the agitating part 12, unlike in a case where a stopper for stopping the holding part 130 rotated to the predetermined angle Q1 is provided to the base part 110 or the like separately from the arm 124 and the holding part 130.

In addition, the controller 91 is configured to perform control to abruptly stop rotation of the holding part 130 when the holding part 130 reaches the origin position Q0 from the predetermined angle Q1 at a time of end of agitation.

The time of end of agitation mentioned herein refers to the time of end of rotation in the R2 direction from the predetermined angle Q1 to the origin position Q0 during the last time (i.e., eighth time) of reciprocating rotation among eight times of repetitively executed reciprocating rotation. Each time of reciprocating rotation is composed of: rotation in the R1 direction from the origin position Q0 to the predetermined angle Q1; and rotation in the R2 direction from the predetermined angle Q1 to the origin position Q0.

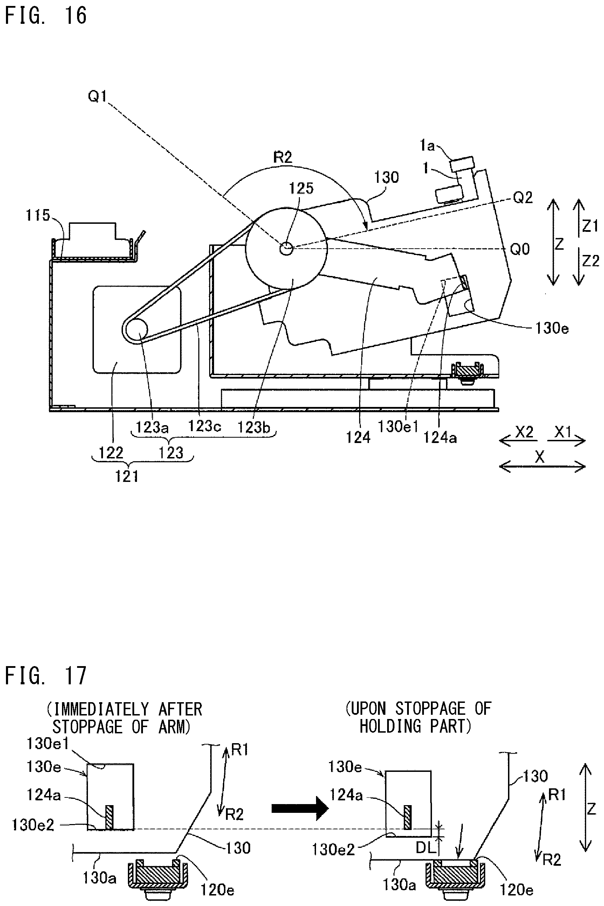

As shown in FIG. 16, the controller 91 causes the arm 124 to be rotated at a low speed from the predetermined angle Q1 to a preparatory position Q2 before the origin position Q0 in the rotation in the R2 direction during the last time of reciprocating rotation. The preparatory position Q2 is a position, on the R1 direction side relative to the origin position Q0, at a rotation angle between the origin position Q0 and the predetermined angle Q1. Thereafter, the controller 91 controls the drive part 121 to: rotate the arm 124 at a high speed from the preparatory position Q2 toward the origin position Q0; and, when it is detected that the holding part 130 has reached the origin position Q0, stop the rotation of the arm 124 and stop the holding part 130 at the origin position Q0. The movement speed of the arm 124 at the time of movement from the preparatory position Q2 to the origin position Q0 is higher than the movement speed at the time of causing the holding part 130 to freely fall off. That is, the engagement protrusion 124a of the arm 124 moves away from the inner peripheral edge 130e1 on the R1 side of the engagement hole 130e of the holding part 130 at the time of movement from the preparatory position Q2 to the origin position Q0.

As shown in FIG. 17, when the arm 124 is stopped at the origin position Q0, the holding part 130 continues to move by the gap DL owing to inertia, and the bottom surface portion 130a of the holding part 130 comes into contact (collides) with the support member 120e, whereby the holding part 130 is stopped. At the origin position Q0, the engagement protrusion 124a is away from each of the inner peripheral edge 130e1 on the R1 side and the inner peripheral edge 130e2 on the R2 side of the engagement hole 130e, i.e., is in contact with neither of these inner peripheral edges. In this manner, when the arm 124 rotated to the origin position Q0 is stopped, the holding part 130 moves by the gap DL and collides with the support member 120e owing to inertia, to be abruptly stopped.

As a result, at the origin position Q0, inertial force is exerted substantially vertically downward (in the Z2 direction) to the specimen container 1 held by the holding part 130, owing to the abrupt stop. Consequently, droplets of the specimen adhered on the inner surface of the cap 1a of the specimen container 1 through the agitation operation can be caused to fall off to the bottom portion side inside the specimen container 1 owing to inertia. In a case where the specimen is left on the inner surface of the cap 1a even at the time of suctioning of the specimen, fine droplets of the specimen adhered on the inner surface of the cap 1a might leak to outside upon insertion of the suction tube 71 into the specimen container 1 owing to a difference in pressure between the inner side and the outer side of the specimen container 1. Such fine droplets of the specimen adhered on the inner surface of the cap 1a can be inhibited from leaking upon insertion of the suction tube 71 by causing the droplets to fall off.

The controller 91 causes, for the first to seventh times of reciprocating rotation and rotation in the R1 direction from the origin position Q0 to the predetermined angle Q1 during the eighth time of reciprocating rotation, the holding part 130 to move through rotation at a predetermined high speed in the relevant directions. The controller 91 performs, only for rotation in the R2 direction from the predetermined angle Q1 to the origin position Q0 during the eighth time of reciprocating rotation, control to execute low-speed rotation to the preparatory position Q2 and high-speed rotation from the preparatory position Q2 to the origin position Q0 described above. Control to drive the rotation motor 122 in each of rotation in the R1 direction and rotation in the R2 direction is, for example, trapezoidal drive in which the target speed (angular speed) is set in two levels, i.e., a predetermined high-speed level and a predetermined low-speed level, and is switched according to the number of times of reciprocation in the agitation operation. Although the time of acceleration/deceleration (in particular, the time of deceleration) to the target speed from a stopped state can be set as appropriate, the time of acceleration/deceleration is preferably set to be short within a range of possible setting in order to effectively cause the holding part 130 to abruptly stop.

The agitating part 12 is configured as described above.

### (Sample Preparation Part)

As shown in FIG. 18, the sample preparation part 15 includes: the plurality of reaction parts 81a to 81e (see FIG. 6); and reagent supply parts 82 connected to the respective reaction parts 81a to 81e via flow paths. Each of the reaction parts 81a to 81e is a container having an opened upper portion.

Each of the plurality of reaction parts 81a to 81e receives the specimen suctioned by the suction part 11 and a reagent and reacts the specimen and the reagent with each other, to prepare a measurement sample. The suction part 11 includes the metering part 73 connected to the suction tube 71. The metering part 73 has a function of suctioning and ejecting a predetermined amount of specimen through the suction tube 71. The metering part 73 is implemented by a syringe pump. Consequently, the specimen contained in the specimen container 1 is suctioned and ejected by the suction part 11 to one or more supply-target reaction parts among the reaction parts 81a to 81e. FIG. 18 shows only a configuration including: the reaction part 81a among the plurality of reaction parts 81a to 81e; and the corresponding reagent supply part 82 connected to the reaction part 81a.

The reagent supply part 82 holds a reagent container 3 containing a predetermined amount of reagent. The reagent supply part 82 includes: a reagent container holder 83; a metering part 84a implemented by a diaphragm pump; and electromagnetic valves 85a and 85b which open/close a flow path through which the reagent having been suctioned is transferred to the metering part 84a and the reaction part 81a. In addition, the reagent supply part 82 includes a metering part 84b and electromagnetic valves 85c and 85d for transferring a reagent (a hemolytic agent or the like) from a large-capacity reagent container 4 disposed outside of the measurement unit 10.

The number of the reagent container holders 83 provided is two or more, and each of the reagent container holders 83 is configured such that a corresponding reagent container 3 is held so as to be detachable therefrom and re-attachable thereto. Consequently, the reagent supply part 82 holds a plurality of the reagent containers 3 containing a plurality of respective types of reagents corresponding to a measurement item. Each of the reagent containers 3 is replaced with a new reagent container 3 when the contained reagent runs out.

Control by the controller 91 causes the electromagnetic valve 85a to be opened and causes the electromagnetic valve 85b to be closed, whereby the metering part 84a suctions a predetermined amount of reagent in the reagent container 3 through the flow path into the metering part 84a. Consequently, the predetermined amount of reagent necessary for preparing a measurement sample is metered. In addition, control by the controller 91 causes the electromagnetic valve 85a to be closed and causes the electromagnetic valve 85b to be opened, whereby the metering part 84a can transfer the reagent metered in the metering part 84a to the reaction part 81a.

For the metering part 84b, the electromagnetic valve 85c, and the electromagnetic valve 85d which are connected to the large-capacity reagent container 4, operations of these respective parts are also controlled by the controller 91 in the same manner as the operations of the metering part 84a, the electromagnetic valve 85a, and the electromagnetic valve 85b. Consequently, respective types of reagents are transferred into the reaction parts 81a to 81e. In addition, a waste liquid chamber 86 for disposing of a measured (prepared) sample is provided inside the measurement unit 10 and is connected to each of the reaction parts 81a to 81e via an electromagnetic valve 85e. The measured (prepared) sample is disposed of in the waste liquid chamber 86 through opening/closing of the electromagnetic valve 85e by the controller 91.

As shown in FIG. 19, the measurement part 16 includes a first detector 16a which performs WBC detection (detection of white blood cells) through flow cytometry using a semiconductor laser. The measurement part 16 includes a second detector 16b which performs RBC detection (detection of red blood cells) and PLT detection (detection of platelets) through a sheath flow DC detection method. The measurement part 16 includes a third detector 16c which performs HGB detection (detection of blood pigments in blood) through an SLS-hemoglobin method. In addition, detection signals obtained by the measurement part 16 (the first detector 16a, the second detector 16b, and the third detector 16c) are transmitted to the analysis part 30 as data of a measurement result regarding the specimen.

The plurality of reaction parts 81a to 81e are provided correspondingly to measurement items. To each of the reaction parts 81a to 81e, a reagent (a staining liquid or the like) of a type corresponding to the relevant measurement item is supplied by the corresponding reagent supply part 82 (see FIG. 18). In each of the reaction parts 81a to 81e, a measurement sample corresponding to the relevant measurement item is prepared via a process including mixing and reaction between the specimen and the reagent. Then, the prepared measurement sample is supplied to the measurement part 16.

For example, each of the reaction part 81a and the reaction part 81b prepares a measurement sample for WBC detection (detection of white blood cells). To the reaction part 81a and the reaction part 81b, a hemolytic agent and a staining liquid are supplied as reagents. The hemolytic agent contains a surfactant, and red blood cells and platelets are dissolved therein by action of the surfactant. The staining liquid contains a fluorescent dye. The reaction part 81a and the reaction part 81b are connected to the first detector 16a of the measurement part 16. Each of cells in the measurement sample supplied to the first detector 16a is converted into electric signals corresponding to scattered light and fluorescence through flow cytometry. The electric signals are plotted on a scattergram in the analysis part 30, and statistical analysis processing is performed, whereby a count value of the number of white blood cells and values resulting from classification of the white blood cells are calculated.

The reaction part 81c prepares a measurement sample for RBC detection (detection of red blood cells) and PLT detection (detection of platelets). The reaction part 81c is connected to the second detector 16b which performs RBC detection (detection of red blood cells) and PLT detection (detection of platelets) through the sheath flow DC detection method. The reaction part 81d is connected to the third detector 16c which performs HGB detection (detection of blood pigments in blood) through the SLS-hemoglobin method. The reaction part 81e prepares a measurement sample for RET detection (detection of reticulocytes) and a measurement sample for PLT high-sensitivity detection (PLT-F detection). The reaction part 81e is connected to the first detector 16a. In an ordinary measurement order, WBC detection, RBC detection, HGB detection, and PLT detection are performed, and either the reaction part 81a or the reaction part 81b, the reaction part 81c, and the reaction part 81d each prepare a sample. The reaction part 81e is used only when a measurement item of RET and/or PLT-F is included in a measurement order.

### (Configuration regarding Control of Specimen Analyzer)

As shown in FIG. 20, the specimen analyzer 100 includes the controller 91, a storage 92, and the communication part 93 which are provided in the measurement unit 10.

The controller 91 includes: a processor implemented by a CPU; and a memory. The controller 91 executes a control program stored in the storage 92, to control each part of the measurement unit 10 and the rack transport part 20. The storage 92 is implemented by a semiconductor memory element and stores therein: the program to be executed by the controller 91; data of identification information and a measurement order; data (detection signal) of a measurement result obtained by the measurement part 16; a state of the agitating part 12 described later; and a state of the container transfer part 13b described later. The communication part 93 includes a communication interface for performing communication with the analysis part 30. Alternatively, the controller 91 and the storage 92 may each be implemented by an FPGA.

The analysis part 30 is mainly composed of: a controller 31 implemented by a CPU; a storage 32 including a ROM, a RAM, and a solid-state drive (SSD); a display part 33 implemented by a liquid crystal display; an input part 34 implemented by a keyboard and a mouse; and a communication part 35 implemented by a communication interface.

The analysis part 30 is communicably connected to the communication part 93 of the measurement unit 10 and the host computer 300 via the communication part 35. The analysis part 30 obtains, via the communication part 93, identification information read by each of the information reading part 24 of the rack transport part 20 and the reading part 14 of the measurement unit 10. The analysis part 30 identifies each of the specimen containers 1 held by the rack 2, on the basis of the identification information on the specimen container 1. The analysis part 30 inquires of the host computer 300 about the identification information, to obtain a measurement order for the specimen in the specimen container 1 held by the container transfer part 13b.

The analysis part 30 transmits the obtained measurement order to the controller 91 via the communication part 35 and the communication part 93. The controller 91 controls each part of the measurement unit 10 and the rack transport part 20 according to the transmitted measurement order. The controller 91 controls the suction part 11 and the sample preparation part 15 to prepare a measurement sample corresponding to a measurement item specified in the measurement order. Then, the controller 91 controls the measurement part 16 to perform measurement, corresponding to the measurement item, on the prepared measurement sample.

The controller 91 causes the measurement part 16 to measure the specimen in the specimen container 1, and obtains a detection signal from the measurement part 16. The controller 91 transmits the detection signal via the communication part 93 to the analysis part 30. The analysis part 30 analyzes the analyte content of the specimen or the like on the basis of the obtained detection signal. In addition, the analysis part 30 causes an analysis result to be displayed on the display part 33 and to be transmitted to the host computer 300.

### (Measurement Operation of Specimen Analyzer)

Next, the flow of a measurement operation executed on one specimen container 1 by the specimen analyzer 100 will be described with reference to FIG. 21. Operations of the measurement unit 10 and the rack transport part 20 are controlled by the controller 91. Measurement in the specimen analyzer 100 is performed through execution of a specimen analysis method of the present embodiment by the specimen analyzer 100 which analyzes a specimen contained in a specimen container 1.

As shown in FIG. 21, the specimen analysis method includes at least:
a step (S1) of taking out, from a rack 2 accommodating a plurality of the specimen containers 1, any of the specimen containers 1;
a step (S3) of agitating a specimen in the specimen container 1 having been taken out;
a step (S8) of transferring, to the suction position P6, the specimen container 1 having been agitated;
a step (S9) of suctioning the specimen from the specimen container 1 transferred to the suction position P6;
a step (S11) of returning, to the rack 2, the specimen container 1 having been subjected to suctioning of the specimen;
a step (S13) of measuring a measurement sample prepared from the suctioned specimen; and
a step (S14) of analyzing a signal obtained through measurement of the measurement sample.

In the present embodiment, the specimen analyzer 100 is configured such that control by the controller 91 causes, during a period from start to end of agitation of one of the specimen containers 1, transferring to the suction position P6 (S8), suctioning of a specimen (S9), and returning to the rack 2 (S11) to be executed on another one of the specimen containers 1.

Consequently, agitation processing for the one specimen container 1 by the agitating part 12 and processing for the other specimen container 1 (transferring to the suction position P6, suctioning of a specimen, and returning to the rack 2) can be executed as concurrent processing operations that coincide in time with each other. Therefore, as compared to a case where the processing for the one specimen container 1 and the processing for the other specimen container 1 are executed separately and sequentially, the number of specimens to be processed per unit time by the specimen analyzer 100 in the case of analyzing a plurality of specimens can be increased by a number corresponding to the period during which the agitation processing for the one specimen container 1 and the processing for the other specimen container 1 coincide in time with each other. Ordinarily, among a series of operations of taking out a specimen container 1 from the rack 2, agitating the specimen container 1, suctioning a specimen from the specimen container 1, and returning the specimen container 1 to the rack 2, the operation of agitating the specimen container 1 requires the longest time. The purpose of setting the time for this operation to be longest is to evenly distribute components of the specimen inside the specimen container 1 and improve the accuracy of analysis. For example, in the automatic multi-item blood cell analyzer XN-1000 (manufactured by Sysmex Corporation) as a conventional analyzer, the time required for the above series of operations from the operation of taking out a specimen container to the operation of returning the specimen container is about 36 seconds, and the time required for the operation of agitating the specimen container among the series of operations is about 12 seconds. That is, in the automatic multi-item blood cell analyzer XN-1000, an (N+1)^{th} specimen container can be taken out from the rack about 36 seconds after an N^{th} specimen container is taken out from the rack, and up to 100 specimens can be processed per hour. Meanwhile, in the specimen analyzer 100 in the present embodiment, while the agitation processing requiring the longest time is being performed on one specimen container 1, transferring to the suction position P6, suctioning of a specimen, and returning to the rack 2 are performed on another specimen container 1. Consequently, in a case where each of the operations requires a time equivalent to that required in the automatic multi-item blood cell analyzer XN-1000, the (N+1)^{th} specimen container 1 can be taken out from the rack 2 about 18 seconds after the N^{th} specimen container 1 is taken out from the rack 2, whereby up to 200 specimens can be processed per hour.

Hereinafter, the flow of a measurement operation to be executed on one specimen container 1 by the specimen analyzer 100 will be specifically described. First, in step S1, the rack transport part 20 transports one of a plurality of specimen containers 1 held by a rack 2 to the take-out position P0 (see FIG. 2). The hand part 13a takes out the specimen container 1 placed at the take-out position P0 among the plurality of specimen containers 1 held by the rack 2.

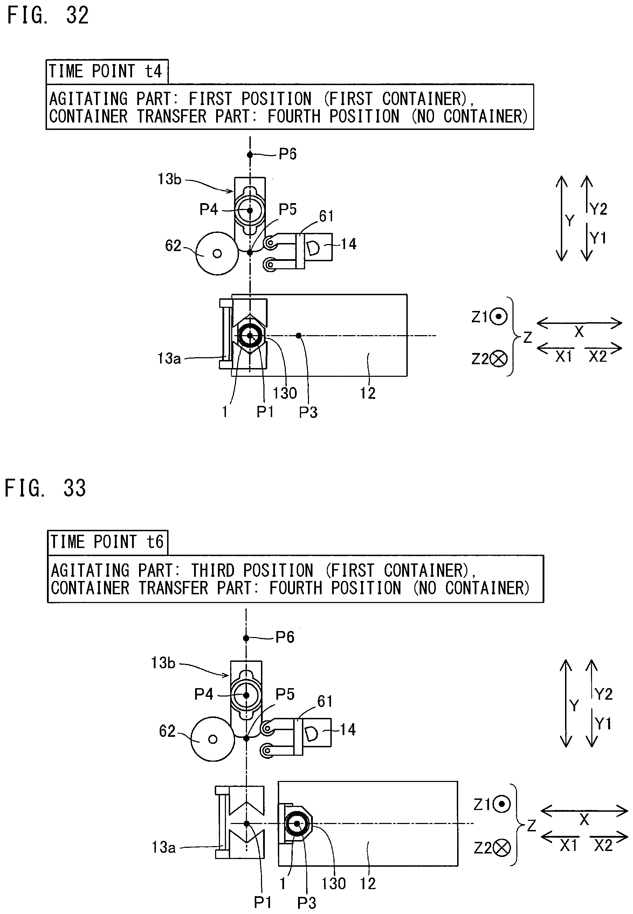

In step S2, with the hand part 13a and the agitating part 12, the specimen container 1 having been taken out from the rack 2 is relayed from the hand part 13a to the agitating part 12. Specifically, the hand part 13a (see FIG. 5) grips the specimen container 1 and lifts to a position above the first position P1, and then the agitating part 12 moves to the first position P1 (see FIG. 32). At the first position P1, the holding hole 131a of the holding part 130 is positioned immediately below the specimen container 1 gripped by the hand part 13a. The hand part 13a moves downward, whereby the specimen container 1 is inserted into the holding hole 131a of the holding part 130. Then, the hand part 13a releases the gripped specimen container 1 and moves upward, whereby the specimen container 1 is relayed to the holding part 130 of the agitating part 12. After the specimen container 1 is relayed to the agitating part 12, the agitating part 12 moves from the first position P1 to the third position P3 (see FIG. 33).

In step S3, the agitating part 12 agitates a specimen in the specimen container 1. The agitating part 12 moves the fixation part 132 to the fixation position (see FIG. 12) so as to fix the specimen container 1 to the holding part 130 and causes reciprocating rotation (see FIG. 13 and FIG. 14) of the holding part 130 by the drive part 121 while maintaining the fixation state. The reciprocating rotation is repeated eight times as described above.

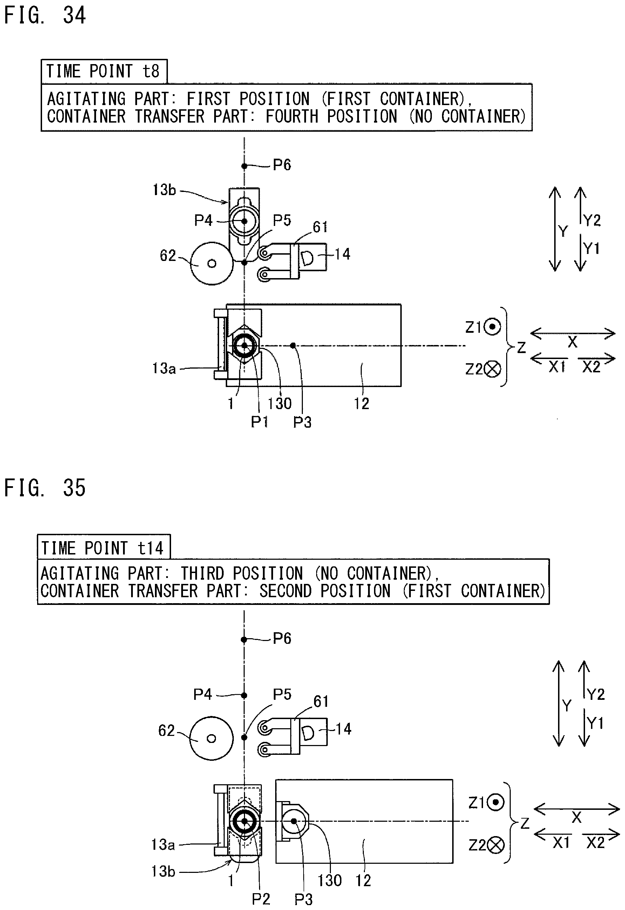

In step S4, the agitating part 12 moves from the third position P3 to the first position P1 (see FIG. 34).

In step S5, with the agitating part 12, the hand part 13a, and the container transfer part 13b, the specimen container 1 having been agitated is relayed from the agitating part 12 to the container transfer part 13b. First, the agitating part 12 moves the fixation part 132 to the release position (see FIG. 10) so as to release the fixed specimen container 1. Next, the hand part 13a takes out the specimen container 1 from the holding part 130 of the agitating part 12 having moved to the first position P1. When the specimen container 1 is taken out from the holding part 130, the agitating part 12 moves from the first position P1 to the third position P3.

After the agitating part 12 moves to the third position P3, the container transfer part 13b moves to the second position P2 (see FIG. 35). At the second position P2, the container holding part 51 of the container transfer part 13b is positioned immediately below the specimen container 1 gripped by the hand part 13a. The hand part 13a inserts the specimen container 1 into the container holding part 51 of the container transfer part 13b having moved to the second position P2. The hand part 13a releases the gripped specimen container 1 and moves upward, whereby relaying of the specimen container 1 to the container transfer part 13b is completed.

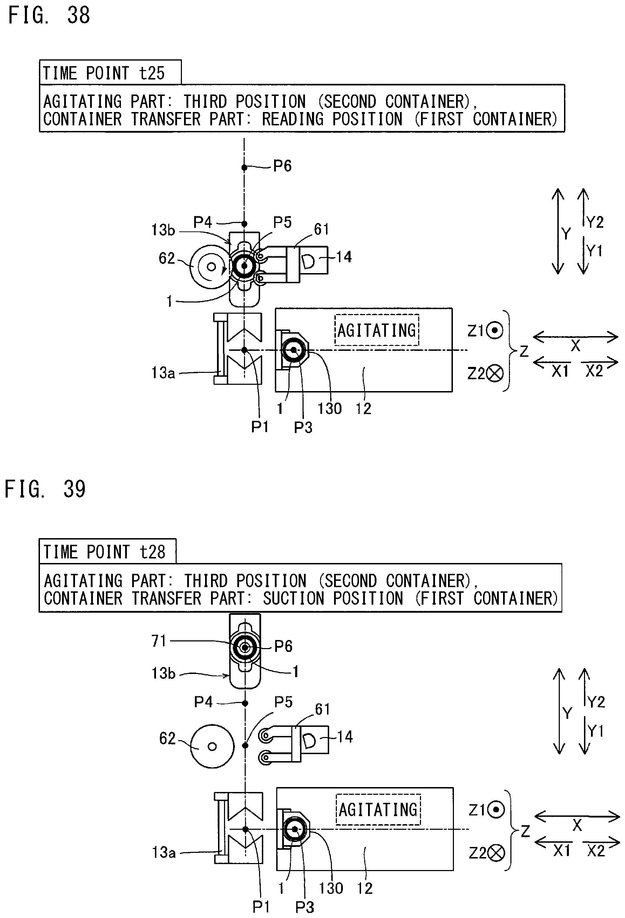

In step S6, the container transfer part 13b moves the specimen container 1 to the reading position P5 (see FIG. 38). In step S7, the reading part 14 reads identification information from the barcode on the specimen container 1 having been moved to the reading position P5.

In step S8, the container transfer part 13b moves the specimen container 1 to the suction position P6 (see FIG. 39). In step S9, the suction part 11 suctions the specimen from the specimen container 1 having been moved to the suction position P6.

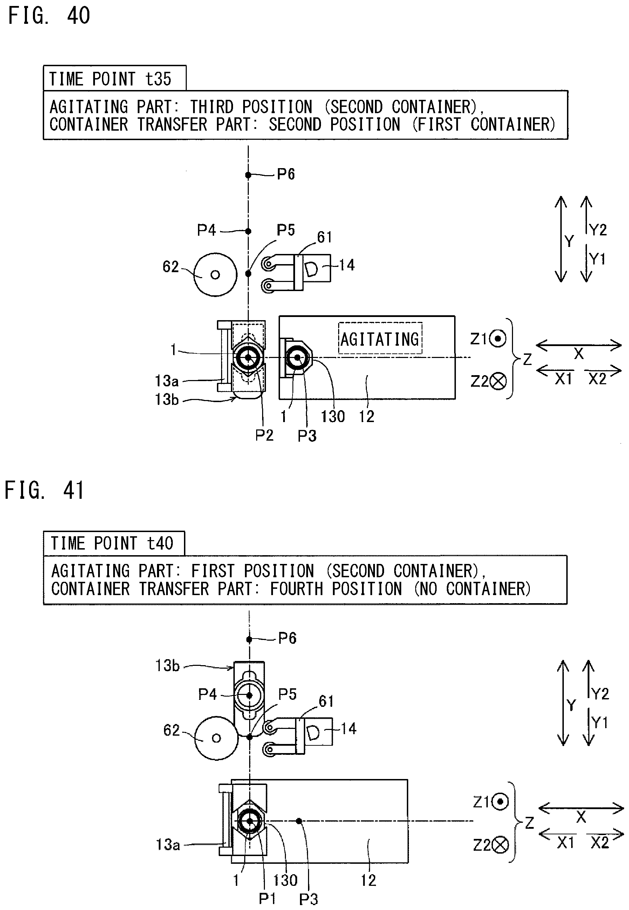

In step S10, the container transfer part 13b moves the specimen container 1 to the second position P2 (see FIG. 40). In step S11, with the hand part 13a, the container transfer part 13b, and the rack transport part 20, the specimen container 1 having been subjected to suctioning of the specimen and held by the container holding part 51 of the container transfer part 13b is taken out, and the specimen container 1 having been taken out is returned to the rack 2. Specifically, the hand part 13a takes out the specimen container 1 from the container holding part 51 at the second position P2. Thereafter, the container transfer part 13b causes the container holding part 51 to retract from the second position P2 to the fourth position P4, and the rack transport part 20 transports the rack 2 such that the original holding position in the rack 2 is present at the take-out position P0. The hand part 13a returns the gripped specimen container 1 to the rack 2.

In step S12, with the suction part 11 and the sample preparation part 15, a measurement sample is prepared. Specifically, the suction part 11 ejects, to one of the reaction parts 81a and 81b, the specimen suctioned (in step S9) from the specimen container 1. In an ordinary measurement order, the suction part 11 ejects the specimen to each of the reaction parts 81c and 81d. The sample preparation part 15 supplies reagents to the respective reaction parts each containing the specimen and mixes the specimen and the reagents with each other in the reaction parts, to prepare measurement samples.

In step S13, the measurement part 16 measures each of the measurement samples. Specifically, the measurement part 16 supplies the measurement sample prepared in the reaction part 81a or 81b to the first detector 16a and performs measurement through flow cytometry. In addition, the measurement part 16 supplies the measurement sample prepared in the reaction part 81c and the measurement sample prepared in the reaction part 81d to the corresponding second detector 16b and third detector 16c, respectively, and executes detection according to the sheath flow DC detection method and detection according to the SLS-hemoglobin method.

In step S14, the controller 91 outputs, to the analysis part 30, measurement data based on electric signals obtained from the respective detectors through the measurement in step S13. The analysis part 30 having received the measurement data performs analysis processing on the measurement data. As a result of the analysis processing, an analysis result regarding each of measurement items specified in the measurement order is generated.

### (Control to Be Executed by Controller)

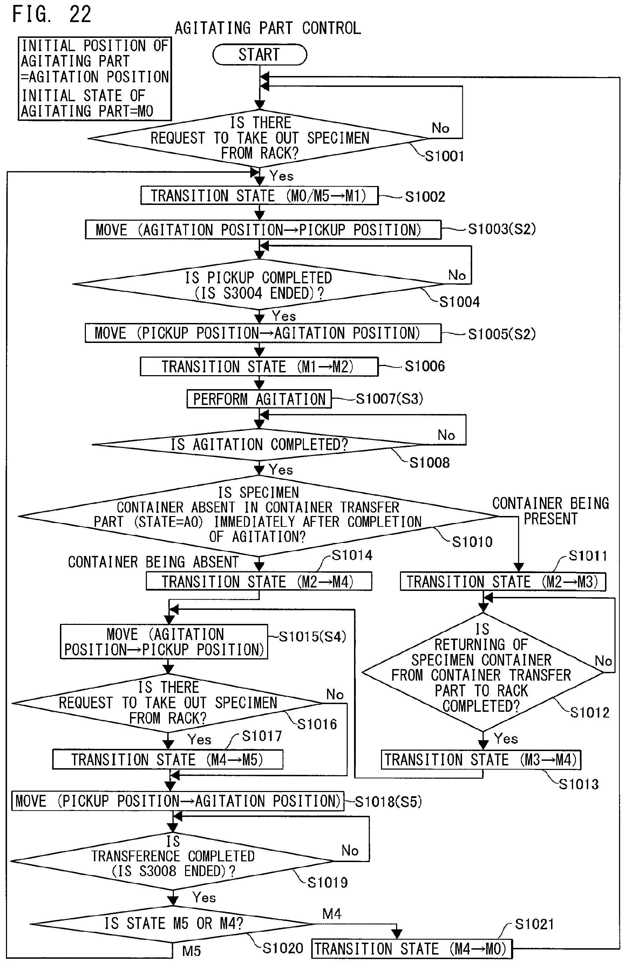

As described above, the present embodiment is such that, in the case of consecutively measuring specimens in a plurality of specimen containers, the specimen analyzer 100 executes, while the agitation operation in step S3 is being executed on one of the specimen containers 1, transferring to the suction position P6 (S8), suctioning of a specimen (S9), and returning to the rack 2 (S11) on another one of the specimen containers 1. Hereinafter, control to be executed by the controller 91 in order to realize this feature will be described with reference to FIG. 22 to FIG. 29.

The controller 91 concurrently executes a series of control operations for the suction part 11 (suction part control), a series of control operations for the agitating part 12 (agitating part control), a series of control operations for the hand part 13a (hand part control), a series of control operations for the container transfer part 13b (container transfer part control), a series of control operations for the reading part 14 (reading part control), a series of control operations for the sample preparation part 15 (sample preparation part control), a series of control operations for the measurement part 16 (measurement part control), and a series of control operations for the rack transport part 20 (rack transport part control).

The suction part control is control in which the suction part 11 suctions a specimen from each specimen container 1 placed at the suction position P6 and ejects the suctioned specimen to relevant ones of the reaction parts 81a to 81e. The agitating part control is control in which the agitating part 12 receives each specimen container 1 at the first position P1 (pickup position), agitates the received specimen container 1, and transfers the agitated specimen container 1 to the first position P1 again. The container transfer part control is control in which the container transfer part 13b receives each specimen container 1 at the pickup position P2, transfers the received specimen container 1 to the reading position P5 and the suction position P6, and transfers the specimen container 1 having been subjected to suctioning of a specimen to the pickup position P2 again. The hand part control is control in which: each specimen container 1 placed at the take-out position P0 is transferred (picked up) from the rack 2 to the agitating part 12; the specimen container 1 placed at the first position P1 is transferred from the agitating part 12 to the container transfer part 13b; and the specimen container 1 placed at the second position P2 is transferred (returned) from the container transfer part 13b to the rack 2.

The reading part control is control in which identification information is read from the barcode on each specimen container 1 placed at the reading position P5. The sample preparation part control is control in which specimens and reagents contained in the reaction parts 81a to 81e are mixed with each other so that measurement samples are prepared. The measurement part control is control in which the measurement samples are supplied to the first detector 16a, the second detector 16b, and the third detector 16c, and measurement data is obtained. The rack transport part control is control in which: the rack 2 placed on the pre-analysis rack holding part 21 is transported such that a specimen container 1 to be subjected to suctioning is placed at the take-out position P0; after the specimen container 1 is taken into the measurement unit 10, the rack 2 is, in order to receive the specimen container 1 for which suctioning has been completed, transported such that the holding hole 2a having held this specimen container 1 is placed at the take-out position P0 again; and, after this specimen container 1 is returned to the rack 2, the rack 2 is transported to the post-analysis rack holding part 22.

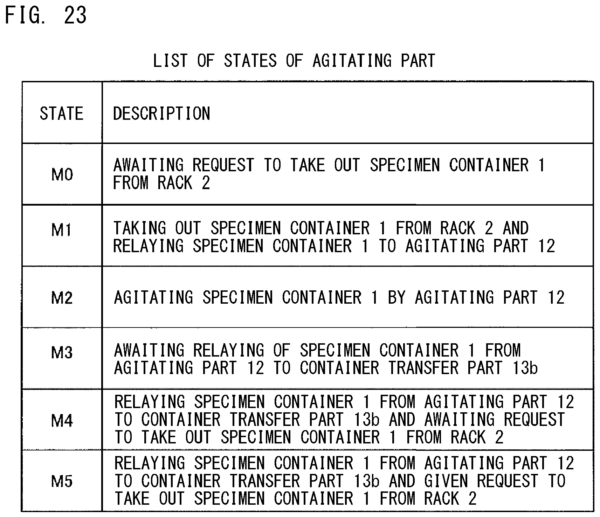

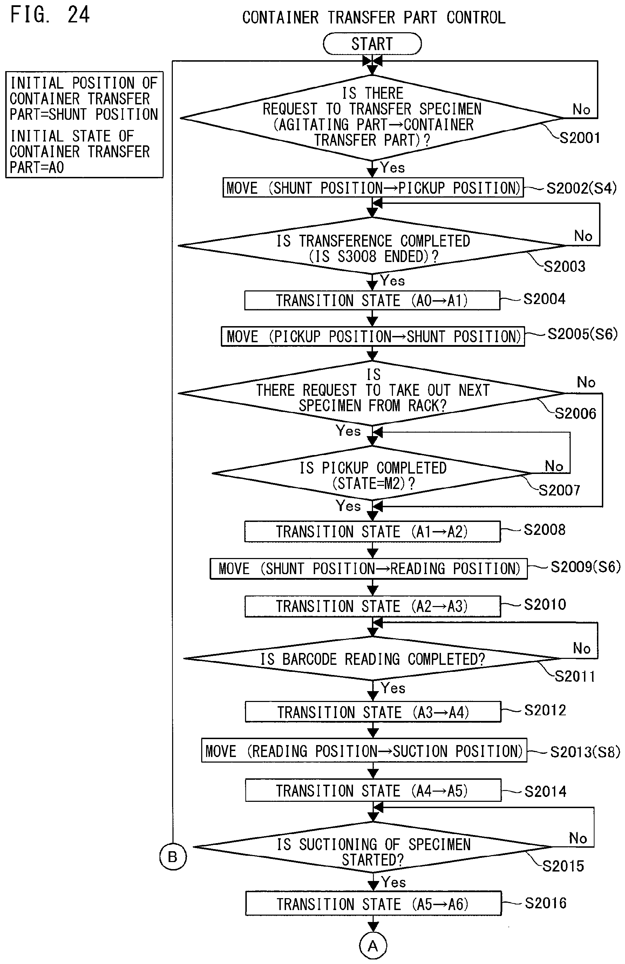

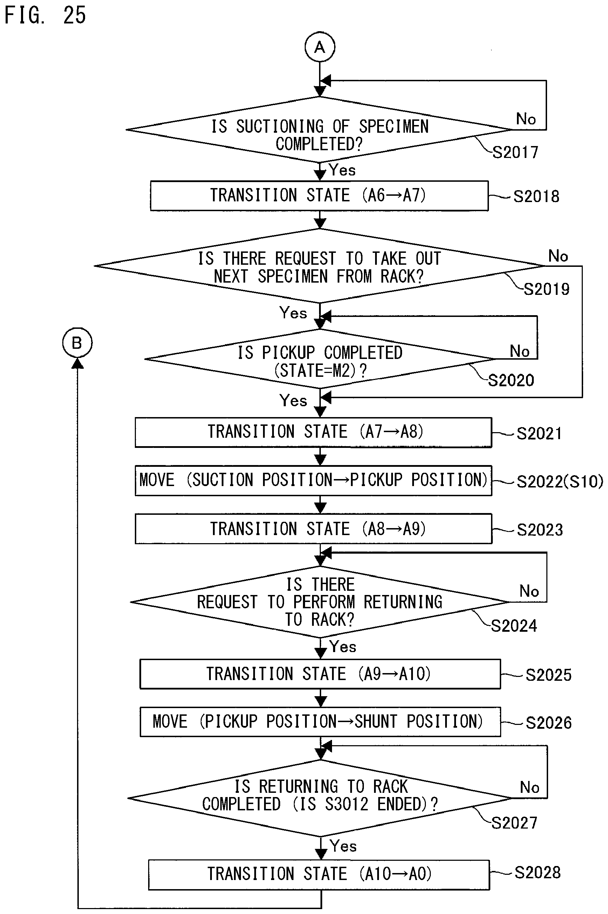

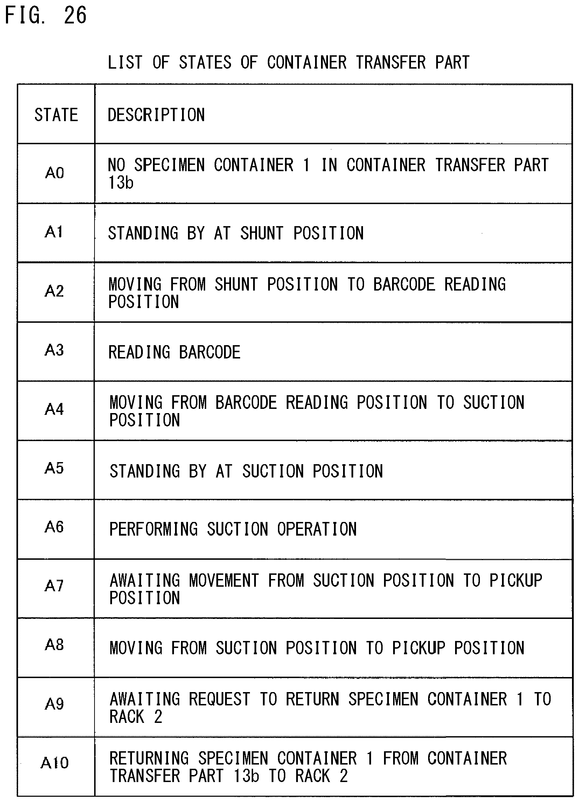

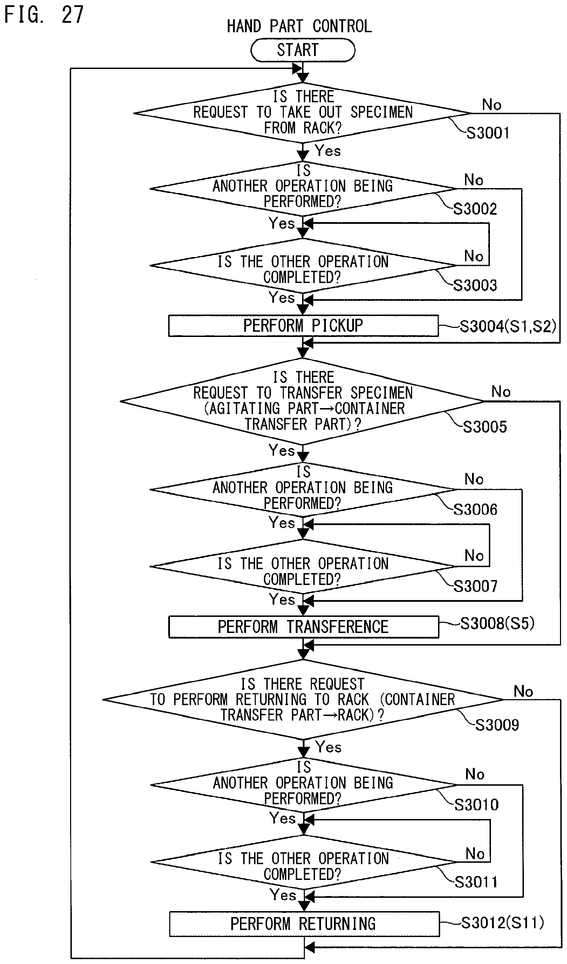

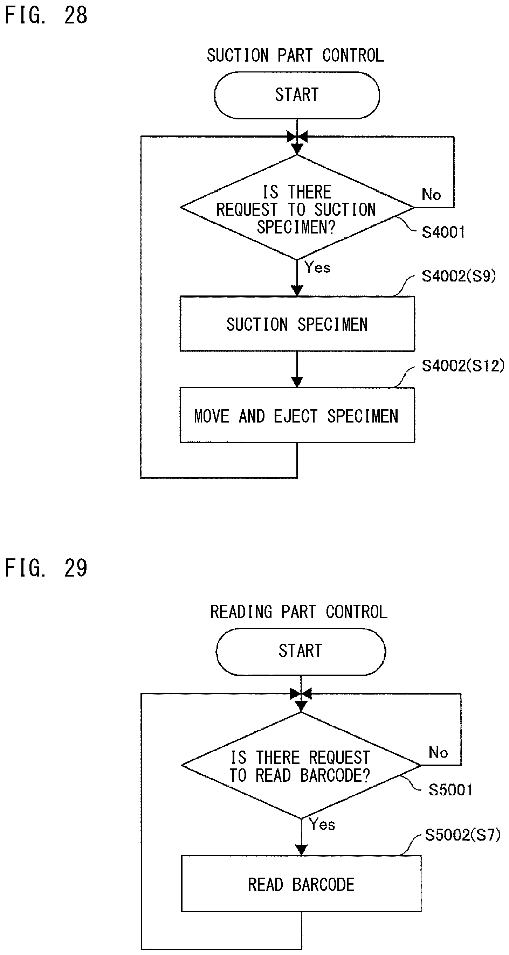

Hereinafter, the agitating part control, the container transfer part control, the hand part control, the suction part control, and the reading part control among the above control operations to be concurrently executed by the controller 91 will be further described. FIG. 22 is a flowchart showing the flow of a process to be performed through the agitating part control. FIG. 23 is a diagram for explaining each of states of the agitating part 12. FIG. 24 and FIG. 25 are each a flowchart showing the flow of a process to be performed through the container transfer part control. FIG. 26 is a diagram for explaining each of states of the container transfer part 13b. FIG. 27 is a flowchart showing the flow of a process to be performed through the hand part control. FIG. 28 is a flowchart showing the flow of a process to be performed through the suction part control. FIG. 29 is a flowchart showing the flow of a process to be performed through the reading part control.

The state of the agitating part 12 is transitioned according to the process shown in FIG. 22. The state of the agitating part 12 is stored in the storage 92, and, when the state of the agitating part 12 is transitioned, the controller 91 updates the state stored in the storage 92 to the post-transition state. As shown in FIG. 23, a state M0 is a state where a request to take out a specimen container 1 from the rack 2 placed on the rack transport part 20 is awaited. A state M1 is a state where a specimen container 1 is being taken out from the rack 2 and relayed to the agitating part 12. A state M2 is a state where the specimen container 1 is being agitated by the agitating part 12. A state M3 is a state where relaying of the specimen container 1 from the agitating part 12 to the container transfer part 13b is awaited. A state M4 is a state where: the specimen container 1 is being relayed from the agitating part 12 to the container transfer part 13b; and a request to take out a specimen container 1 from the rack 2 is awaited. A state M5 is a state where: the specimen container 1 is being relayed from the agitating part 12 to the container transfer part 13b; and there is a request to take out a specimen container 1 from the rack 2.

As shown in FIG. 22, the initial state of the agitating part 12 is M0. In the initial state, the agitating part 12 is located at the third position P3 (agitation position). In step S1001, the controller 91 determines whether or not there is a request to take out a specimen container 1 from the rack 2. A request to take out a specimen container 1 from the rack 2 is reported when a specimen container 1 containing a specimen that needs to be measured is placed at the take-out position P0. The controller 91 stands by (step S1001: No) until a request to take out a specimen container 1 from the rack 2 is given. Meanwhile, when there is a request to take out a specimen container 1 from the rack 2 (step S1001: Yes), the controller 91 advances the process to step S1002. In step S1002, the controller 91 transitions the state of the agitating part 12 from M0 to M1. In step S1003, the controller 91 causes the agitating part 12 to move from the third position P3 (agitation position) to the first position P1 (pickup position) at a timing at which the hand part 13a has moved to the position above the first position P1 (pickup position) through processing in step S3004 of the hand part control described later. In step S1004, the controller 91 determines whether or not completion of relaying of the specimen container 1 from the rack 2 to the agitating part 12 has been reported (completion of pickup of the specimen container 1 has been reported). Completion of pickup of the specimen container 1 is reported when the hand part 13a has relayed the specimen container 1 having been taken out from the rack 2 to the agitating part 12 through the processing in step S3004 of the hand part control described later. The controller 91 stands by (step S1004: No) until completion of pickup of the specimen container 1 has been reported. Meanwhile, when completion of pickup of the specimen container 1 is reported (step S1004: Yes), the controller 91 advances the process to step S1005.

In step S1005, the controller 91 causes the agitating part 12 to move from the first position P1 (pickup position) to the third position P3 (agitation position). In step S1006, the controller 91 transitions the state of the agitating part 12 from M1 to M2. The processing in step S1003 and the processing in step S1005 correspond to the operation in step S2 in FIG. 21. In step S1007, the controller 91 controls the agitating part 12 to agitate the specimen in the specimen container 1. About 12 seconds are required from start to end of the processing in step S1007. The processing in step S 1007 corresponds to the operation in step S3 in FIG. 21.

In step S1008, the controller 91 determines whether or not completion of agitation of the specimen container 1 by the agitating part 12 has been reported. Completion of agitation of the specimen container 1 by the agitating part 12 is reported when the last time of reciprocating rotation by the agitating part 12 is completed. The controller 91 stands by (step S1008: No) until completion of agitation of the specimen container 1 by the agitating part 12 is reported. Meanwhile, when completion of agitation of the specimen container 1 by the agitating part 12 is reported (step S1008: Yes), the controller 91 advances the process to step S1010. In step S1010, the controller 91 determines whether or not a specimen container 1 is held by the container transfer part 13b at a timing immediately after agitation of the specimen container 1 is completed, i.e., a timing at which completion of agitation of the specimen container 1 by the agitating part 12 is reported. Specifically, in step S1010, the controller 91 determines whether or not the state of the container transfer part 13b is A0 described later at the timing at which completion of agitation of the specimen container 1 by the agitating part 12 is reported. The controller 91 controls operation of the agitating part 12 according to whether or not the state of the container transfer part 13b is A0.

On one hand, in a case where, at the timing at which completion of agitation of the specimen container 1 by the agitating part 12 is reported, a specimen container 1 is held by the container transfer part 13b, i.e., the state of the container transfer part 13b is not A0 (step S1010: container being present), the controller 91 transitions the state of the agitating part 12 from M2 to M3 in step S1011. The controller 91 stands by (step S1012: No) until the specimen container 1 in the container transfer part 13b is returned to the rack in step S1012. Meanwhile, when the specimen container 1 in the container transfer part 13b is returned to the rack 2, i.e., the state of the container transfer part 13b becomes A0 (step S1012: Yes), the controller 91 advances the process to step S1013. In step S1013, the controller 91 transitions the state of the agitating part 12 from M3 to M4 and advances the process to step S 1015.

On the other hand, in a case where, at the timing at which completion of agitation of the specimen container 1 by the agitating part 12 is reported, no specimen container 1 is held by the container transfer part 13b, i.e., the state of the container transfer part 13b is A0 (step S1010: container being absent), the controller 91 transitions the state of the agitating part 12 from M2 to M4 in step S1014 and advances the process to step S1015 without standing by. In step S1015, the controller 91 causes the agitating part 12 to move from the third position P3 (agitation position) to the first position P1 (pickup position). The processing in step S1015 corresponds to the operation in step S4 in FIG. 21.

In step S1016, the controller 91 determines whether or not there is a request to take out a specimen container 1 from the rack 2. A request to take out a specimen container 1 from the rack 2 in the present step is reported when a specimen container 1 containing a specimen that needs to be measured next to the specimen container 1 having been taken into the measurement unit 10 is placed at the take-out position P0. When there is a request to take out a specimen container 1 from the rack 2 (step S1016: Yes), the controller 91 transitions the state of the agitating part 12 from M4 to M5 in S 1017. Meanwhile, when there is no request to take out a specimen container 1 from the rack 2 (step S1016: No), the controller 91 skips step S1017 and advances the process to step S1018.

In step S1018, the controller 91 causes the agitating part 12 to move from the first position P1 (pickup position) to the third position P3 (agitation position) after the hand part 13a takes out the specimen container 1 from the agitating part 12 through processing in step S3008 of the hand part control described later. The processing in step S1018 corresponds to the operation in step S5 in FIG. 21. In step S1019, the controller 91 determines whether or not completion of relaying of the specimen container 1 from the agitating part 12 to the container transfer part 13b has been reported. Completion of relaying of the specimen container 1 from the agitating part 12 to the container transfer part 13b is reported when the hand part 13a relays the specimen container 1 having been taken out from the agitating part 12 to the container transfer part 13b through the processing in step S3008 of the hand part control described later. The controller 91 stands by (step S1019: No) until completion of relaying of the specimen container 1 from the agitating part 12 to the container transfer part 13b is reported (completion of transference of the specimen container 1 is reported). Meanwhile, when completion of transference of the specimen container 1 is reported (step S1019: Yes), the controller 91 advances the process to step S1020.

In step S1020, the controller 91 determines whether the state of the agitating part 12 is M5 or M4. When the state of the agitating part 12 is M5 (step S1020: M5), the controller 91 returns the process to step S 1002, transitions the state of the agitating part 12 from M5 to M1 in step S1002, and executes processing for the specimen container 1 containing the specimen that needs to be measured next. Meanwhile, when the state of the agitating part 12 is M4 (step S1020: M4), the controller 91 transitions the state of the agitating part 12 from M4 to M0 in step S1021, returns the process to step S1001, and awaits a request to take out a next specimen container 1 from the rack 2.

Next, the container transfer part control will be described. The state of the container transfer part 13b is transitioned according to the process shown in FIG. 24 and the FIG. 25. The state of the container transfer part 13b is stored in the storage 92, and, when the state of the container transfer part 13b is transitioned, the controller 91 updates the state stored in the storage 92 to the post-transition state. As shown in FIG. 26, the state A0 is a state where no specimen container 1 is placed in the container transfer part 13b. A state A1 is a state where the container transfer part 13b is standing by at the fourth position P4 (shunt position). A state A2 is a state where the container transfer part 13b is moving from the fourth position P4 (shunt position) to the fifth position P5 (reading position). A state A3 is a state where the reading part 14 is reading identification information from the barcode label 1b on a specimen container 1. A state A4 is a state where the container transfer part 13b is moving from the reading position P5 to the suction position P6. A state A5 is a state where the container transfer part 13b is standing by at the suction position P6. A state A6 is a state where the suction part 11 is suctioning a specimen from the specimen container 1. A state A7 is a state where the container transfer part 13b awaits start of movement from the suction position P6 to the second position P2 (pickup position). A state A8 is a state where the container transfer part 13b is moving from the suction position P6 to the second position P2 (pickup position). A state A9 is a state where a request to return the specimen container 1 to the rack 2 placed on the rack transport part 20 is awaited. A state A10 is a state where the hand part 13a is returning the specimen container 1 from the container transfer part 13b to the rack 2.

As shown in FIG. 24, the initial state of the container transfer part 13b is A0. In the initial state, the container transfer part 13b is located at the fourth position P4 (shunt position). In step S2001, the controller 91 determines whether or not there is a request to transfer the specimen container 1 from the agitating part 12 to the container transfer part 13b. A request to transfer the specimen container 1 from the agitating part 12 to the container transfer part 13b is reported when agitation of the specimen in the specimen container 1 by the agitating part 12 is completed. The controller 91 stands by (step S2001: No) until a request to transfer the specimen container 1 from the agitating part 12 to the container transfer part 13b is given. Meanwhile, when there is a request to transfer the specimen container 1 from the agitating part 12 to the container transfer part 13b (step S2001: Yes), the controller 91 advances the process to step S2002. In step S2002, the controller 91 causes the container transfer part 13b to move from the fourth position P4 (shunt position) to the second position P2 (pickup position). The processing in step S2002 corresponds to the operation in step S4 in FIG. 21. The processing in step S2002 is executed after the processing in step S1018 in FIG. 22.

In step S2003, the controller 91 determines whether or not completion of relaying of the specimen container 1 from the agitating part 12 to the container transfer part 13b has been reported (completion of transference of the specimen container 1 has been reported). Completion of relaying of the specimen container 1 from the agitating part 12 to the container transfer part 13b is reported when the hand part 13a takes out the specimen container 1 from the agitating part 12 and relays the specimen container 1 to the container transfer part 13b through the processing in step S3008 of the hand part control described later. This reporting is the same as the reporting in step S1019 shown in FIG. 22.

The controller 91 stands by (step S2003: No) until completion of relaying of the specimen container 1 from the agitating part 12 to the container transfer part 13b has been reported (completion of transference of the specimen container 1 has been reported). Meanwhile, when completion of transference of the specimen container 1 is reported (step S2003: Yes), the controller 91 transitions the state of the container transfer part 13b from A0 to A1 in step S2004. In step S2005, the controller 91 causes the container transfer part 13b to move from the second position P2 (pickup position) to the fourth position P4 (shunt position). The processing in step S2005 corresponds to the operation in step S6 in FIG. 21.

In step S2006, the controller 91 determines whether or not there is a request to take out a next specimen container 1 from the rack 2. A request to take out a next specimen container 1 from the rack 2 is reported when, at the present timing, a specimen container 1 containing a specimen that needs to be measured next to the specimen in the specimen container 1 placed in the container transfer part 13b is placed at the take-out position P0 and the state of the agitating part 12 is M0. When there is no request to take out a next specimen container 1 from the rack 2 (step S2006: No), the controller 91 skips the processing in step S2007 and advances the process to step S2008. Meanwhile, when there is a request to take out a next specimen container 1 from the rack 2 (step S2006: Yes), the controller 91 determines in step S2007 whether or not completion of relaying of the next specimen container 1 from the rack 2 to the agitating part 12 has been reported (completion of pickup of the next specimen container 1 has been reported), i.e., whether or not the state of the agitating part 12 is M2. Completion of pickup of the specimen container 1 is reported when, through the processing in step S3004 of the hand part control described later, the hand part 13a relays the next specimen container 1 having been taken out from the rack 2 to the agitating part 12, and the agitating part 12 moves to the third position P3 (agitation position). The controller 91 stands by (step S2007: No) until completion of pickup of the next specimen container 1 is reported, i.e., until the state of the agitating part 12 becomes M2. Meanwhile, when completion of pickup of the next specimen container 1 is reported, i.e., the state of the agitating part 12 becomes M2 (step S2007: Yes), the controller 91 advances the process to step S2008. In this manner, the controller 91 controls operation of the container transfer part 13b according to whether or not the state of the agitating part 12 is M2. In the processing in steps S2006 to S2007, the container transfer part 13b stands by at the fourth position P4 (shunt position) until relaying of the next specimen container 1 from the rack 2 to the agitating part 12 is completed. Consequently, it is possible to avoid collision between the container transfer part 13b and the agitating part 12 which has moved to the first position P1 (pickup position) in order to receive the next specimen container 1.

In step S2008, the controller 91 transitions the state of the container transfer part 13b from A1 to A2. In step S2009, the controller 91 causes the container transfer part 13b to move from the fourth position P4 (shunt position) to the reading position P5. The processing in step S2009 corresponds to the operation in step S6 in FIG. 21. In step S2010, the controller 91 transitions the state of the container transfer part 13b from A2 to A3. In step S2011, the controller 91 determines whether or not completion of reading of identification information on the specimen container 1 by the reading part 14 has been reported. Completion of reading of identification information on the specimen container 1 by the reading part 14 is reported when the reading part 14 completes reading of the identification information from the barcode label 1b on the specimen container 1 through processing in step S5002 of the reading part control described later. The controller 91 stands by (step S2011: No) until completion of reading of identification information on the specimen container 1 by the reading part 14 has been reported. Meanwhile, when completion of reading of identification information on the specimen container 1 by the reading part 14 is reported (step S2011: Yes), the controller 91 advances the process to step S2012.

In step S2012, the controller 91 transitions the state of the container transfer part 13b from A3 to A4. In step S2013, the controller 91 causes the container transfer part 13b to move from the reading position P5 to the suction position P6. The processing in step S2013 corresponds to the operation in step S8 in FIG. 21. In step S2014, the controller 91 transitions the state of the container transfer part 13b from A4 to A5.

In step S2015, the controller 91 determines whether or not start of suctioning of the specimen from the specimen container 1 by the suction part 11 has been reported. Start of suctioning of the specimen from the specimen container 1 by the suction part 11 is reported when the suction tube 71 of the suction part 11 starts to be lowered from above the specimen container 1 through processing in step S4002 of the suction part control described later. The controller 91 stands by (step S2015: No) until start of suctioning of the specimen from the specimen container 1 by the suction part 11 has been reported. Meanwhile, when start of suctioning of the specimen from the specimen container 1 by the suction part 11 has been reported (step S2015: Yes), the controller 91 transitions the state of the container transfer part 13b from A5 to A6 in step S2016.

In step S2017, the controller 91 determines whether or not completion of suctioning of the specimen from the specimen container 1 by the suction part 11 has been reported. Completion of suctioning of the specimen from the specimen container 1 by the suction part 11 is reported when the suction tube 71 of the suction part 11 is, after suctioning of the specimen, lifted so as to be positioned above the specimen container 1 through the processing in step S4002 of the suction part control described later. The controller 91 stands by (step S2017: No) until completion of suctioning of the specimen from the specimen container 1 by the suction part 11 is reported. Meanwhile, when completion of suctioning of the specimen from the specimen container 1 by the suction part 11 is reported (step S2017: Yes), the controller 91 advances the process to step S2018. In step S2018, the controller 91 transitions the state of the container transfer part 13b from A6 to A7.

In step S2019, the controller 91 determines whether or not there is a request to take out a next specimen container 1 from the rack 2. A request to take out a next specimen container 1 from the rack 2 is reported when, at the present timing, a specimen container 1 containing a specimen that needs to be measured next to the specimen in the specimen container 1 placed in the container transfer part 13b is placed at the take-out position P0 and the state of the agitating part 12 is M0. When there is no request to take out a next specimen container 1 from the rack 2 (step S2019: No), the controller 91 skips processing in step S2020 and advances the process to step S2021. Meanwhile, when there is a request to take out a next specimen container 1 from the rack 2 (step S2019: Yes), the controller 91 determines in step S2020 whether or not completion of relaying of the next specimen container 1 from the rack 2 to the agitating part 12 has been reported (completion of pickup of the next specimen container 1 has been reported), i.e., whether or not the state of the agitating part 12 is M2. Completion of pickup of the specimen container 1 is reported when, through the processing in step S3004 of the hand part control described later, the hand part 13a relays the next specimen container 1 having been taken out from the rack 2 to the agitating part 12, and the agitating part 12 moves to the third position P3 (agitation position). The controller 91 stands by (step S2020: No) until completion of pickup of the next specimen container 1 is reported, i.e., until the state of the agitating part 12 becomes M2. Meanwhile, when completion of pickup of the next specimen container 1 is reported, i.e., the state of the agitating part 12 becomes M2 (step S2020: Yes), the controller 91 advances the process to step S2021. In this manner, the controller 91 controls operation of the container transfer part 13b according to whether or not the state of the agitating part 12 is M2. In the processing in steps S2014 to S2020, the container transfer part 13b stands by at the suction position P6 until relaying of the next specimen container 1 from the rack 2 to the agitating part 12 is completed. Consequently, it is possible to avoid collision between the container transfer part 13b and the agitating part 12 which has moved to the first position P1 (pickup position) in order to receive the next specimen container 1.

In step S2021, the controller 91 transitions the state of the container transfer part 13b from A7 to A8. In step S2022, the controller 91 causes the container transfer part 13b to move from the suction position P6 to the second position P2 (pickup position). The processing in step S2022 corresponds to the operation in step S10 in FIG. 21. In step S2023, the controller 91 transitions the state of the container transfer part 13b from A8 to A9. In step S2024, the controller 91 determines whether or not there is a request to return the specimen container 1 present in the container transfer part 13b to the rack 2. A request to return the specimen container 1 present in the container transfer part 13b to the rack 2 is reported when the holding hole 2a, of the rack 2, having held this specimen container 1 is placed at the take-out position P0. The controller 91 stands by (step S2024: No) until a request to return the specimen container 1 present in the container transfer part 13b to the rack 2 is given. Meanwhile, when there is a request to return the specimen container 1 present in the container transfer part 13b to the rack 2 (step S2024: Yes), the controller 91 advances the process to step S2025. In step S2025, the controller 91 transitions the state of the container transfer part 13b from A9 to A10.

In step S2026, the controller 91 causes the container transfer part 13b to move from the second position P2 (pickup position) to the fourth position P4 (shunt position) after the hand part 13a takes out the specimen container 1 from the container transfer part 13b through processing in step S3012 of the suction part control described later. In step S2027, the controller 91 determines whether or not completion of returning of the specimen container 1 having been present in the container transfer part 13b to the rack 2 has been reported. Completion of returning of the specimen container 1 having been present in the container transfer part 13b to the rack 2 is reported when relaying of the specimen container 1 into the holding hole 2a of the rack 2 by the hand part 13a is completed through the processing in step S3012 of the suction part control described later. The controller 91 stands by (step S2027: No) until completion of returning of the specimen container 1 having been present in the container transfer part 13b to the rack 2 has been reported. Meanwhile, when completion of returning of the specimen container 1 having been present in the container transfer part 13b to the rack 2 is reported (step S2027: Yes), the controller 91 advances the process to step S2028. In step S2028, the controller 91 transitions the state of the container transfer part 13b from A10 to A0, returns the process to step S2001, and awaits a request to transfer a specimen container 1 from the agitating part 12 to the container transfer part 13b.

About 12 seconds are required from start of the processing in step S2008 to end of the processing in step S2027 with the result of the determination being Yes. In a case where a plurality of specimens are being consecutively measured, the result of the determination in step S2007 becomes Yes at a timing at which the state of the agitating part 12 is transitioned to M2 through the processing in step S1006. Therefore, the timing of start of the agitation processing in step S1007 and the timing of start of the processing in step S2008 coincide with each other. As described above, about 12 seconds are required from start to end of the agitation processing in step S1007. Thus, during the period from start to end of agitation of one specimen container 1, processing of transferring to the suction position P6 (step S2013), processing of suctioning of a specimen (steps S2015 to S2017/step S4002 described later), and processing of returning to the rack 2 (steps S2026 and S2027/S3012 described later) are executed on another specimen container 1 that precedes the one specimen container 1.

Next, the hand part control will be described. As shown in FIG. 27, the controller 91 determines in step S3001 whether or not there is a request to take out a specimen container 1 from the rack 2. A request to take out a specimen container 1 from the rack 2 is reported when: a specimen container 1 containing a specimen that needs to be measured is placed at the take-out position P0; and the state of the agitating part 12 is M0. On one hand, when there is a request to take out a specimen container 1 from the rack 2 (step S3001: Yes), the controller 91 determines in step S3002 whether or not the hand part 13a is executing another operation other than a pickup operation of transferring the specimen container 1 from the rack 2 to the agitating part 12. When the hand part 13a is executing another operation (step S3002: Yes), the controller 91 stands by (step S3003: No) until the other operation is completed, and, when the other operation is completed (step S3003: Yes), the controller 91 advances the process to step S3004. Meanwhile, when the controller 91 determines in step S3002 that the hand part 13a is not executing another operation (step S3002: No), the controller 91 skips step S3003 and advances the process to step S3004. On the other hand, when the controller 91 determines in step S3001 that there is no request to take out a specimen container 1 from the rack 2 (step S3001: No), the controller 91 skips steps S3002 and S3003 and advances the process to step S3004.

In step S3004, the controller 91 controls the hand part 13a to execute pickup processing of: taking out, from the rack 2, the specimen container 1 placed at the take-out position P0; lifting the movable part 41 to the position above the first position P1 (pickup position); lowering the movable part 41 after the agitating part 12 moves to the first position P1 (pickup position); and relaying the specimen container 1 to the agitating part 12. The processing in step S3004 corresponds to the operation in step S4 in FIG. 21.

In step S3005, the controller 91 determines whether or not there is a request to transfer the specimen container 1 from the agitating part 12 to the container transfer part 13b. A request to transfer the specimen container 1 from the agitating part 12 to the container transfer part 13b is reported when: agitation of a specimen in the specimen container 1 by the agitating part 12 is completed; and the state of the container transfer part 13b is A0. On one hand, when there is a request to transfer the specimen container 1 from the agitating part 12 to the container transfer part 13b (step S3005: Yes), the controller 91 determines in step S3006 whether or not the hand part 13a is executing another operation other than an operation of transferring the specimen container 1 from the agitating part 12 to the container transfer part 13b. When the hand part 13a is executing another operation (step S3006: Yes), the controller 91 stands by (step S3007: No) until the other operation is completed, and, when the other operation is completed (step S3007: Yes), the controller 91 advances the process to step S3008. Meanwhile, when the controller 91 determines in step S3006 that the hand part 13a is not executing another operation (step S3006: No), the controller 91 skips step S3007 and advances the process to step S3008. On the other hand, when the controller 91 determines in step S3005 that there is no request to transfer the specimen container 1 from the agitating part 12 to the container transfer part 13b (step S3005: No), the controller 91 skips steps S3006 and S3007 and advances the process to step S3008.

In step S3008, the controller 91 controls the hand part 13a to execute transfer processing of: taking out, from the agitating part 12 having moved to the first position P1 (pickup position), the specimen container 1 held by the agitating part 12; lifting the movable part 41 to the position above the first position P1 (pickup position); lowering the movable part 41 after the agitating part 12 moves to the third position (agitation position) and the container transfer part 13b moves to the second position P2 (pickup position); and relaying the specimen container 1 to the container transfer part 13b. The processing in step S3008 corresponds to the operation in step S5 in FIG. 21.

In step S3009, the controller 91 determines whether or not there is a request to return the specimen container 1 present in the container transfer part 13b to the rack 2. A request to return the specimen container 1 present in the container transfer part 13b to the rack 2 is reported when: the holding hole 2a, of the rack 2, having held this specimen container 1 is placed at the take-out position P0; and the state of the container transfer part 13b is A9. On one hand, when there is a request to return the specimen container 1 present in the container transfer part 13b to the rack 2 (step S3009: Yes), the controller 91 determines in step S3010 whether or not the hand part 13a is executing another operation other than a returning operation of returning the specimen container 1 present in the container transfer part 13b to the rack 2. When the hand part 13a is executing another operation (step S3010: Yes), the controller 91 stands by (step S3011: No) until the other operation is completed, and, when the other operation is completed (step S3011: Yes), the controller 91 advances the process to step S3012. Meanwhile, when the controller 91 determines in step S3010 that the hand part 13a is not executing another operation (step S3010: No), the controller 91 skips step S3011 and advances the process to step S3012. On the other hand, when the controller 91 determines in step S3009 that there is no request to return the specimen container 1 present in the container transfer part 13b to the rack 2 (step S3009: No), the controller 91 skips steps S3010 and S3011 and advances the process to step S3012.

In step S3012, the controller 91 controls the hand part 13a to execute returning processing of: taking out, from the container transfer part 13b having moved to the second position P2 (pickup position), the specimen container 1 held by the container transfer part 13b; lifting the movable part 41 to a position above the second position P2 (pickup position); lowering the movable part 41 after the container transfer part 13b moves to the fourth position P4 (shunt position); and relaying the specimen container 1 to the rack 2. The processing in step S3012 corresponds to the operation in step S11 in FIG. 21.

Next, the suction part control will be described. As shown in FIG. 28, the controller 91 determines in step S4001 whether or not there is a request to suction the specimen from the specimen container 1. A request to suction the specimen from the specimen container 1 is reported when the container transfer part 13b holding the specimen container 1 moves to the suction position P6. When there is a request to suction the specimen from the specimen container 1 (step S4001: Yes), the controller 91 causes, in step S4002: lowering of the suction tube 71 which is thus inserted into the specimen container 1; suctioning of the specimen into the suction tube 71; and lifting of the suction tube 71 to a position above the specimen container 1. The processing in step S4002 corresponds to the operation in step S9 in FIG. 21. Meanwhile, when there is no request to suction the specimen from the specimen container 1 (step S4001: No), the controller 91 stands by until such a request is given. In step S4003, the controller 91 controls, according to the measurement item included in the measurement order, the suction part 11 to: move the suction tube 71 from the position above the specimen container 1 to a position above any of the reaction parts 81a to 81e; lower the suction tube 71; and eject the specimen inside the suction tube 71. Then, the controller 91 returns the process to step S4001. The processing in step S4003 corresponds to the operation in step S12 in FIG. 21.

Next, the reading part control will be described. As shown in FIG. 29, the controller 91 determines in step S5001 whether or not there is a request to read the barcode from the specimen container 1. A request to read the barcode from the specimen container 1 is reported when the container transfer part 13b holding the specimen container 1 moves to the reading position P5. When there is a request to read the barcode from the specimen container 1 (step S5001: Yes), the controller 91 controls the reading part 14 to read the barcode from the specimen container 1 in step S5002 and returns the process to step S5001. The processing in step S5002 corresponds to the operation in step S7 in FIG. 21. Meanwhile, when there is no request to read the barcode from the specimen container 1 in step S5001 (step S5001: No), the controller 91 stands by until such a request is given.

### (Timing Chart)

Next, description will be given regarding the timing and the manner of operation of each of the hand part 13a, the agitating part 12a, the container transfer part 13b, and the suction part 11 performed through concurrent processing operations which are the suction part control, the agitating part control, the hand part control, the container transfer part control, the reading part control, the sample preparation part control, the measurement part control, and the rack transport part control described above, in the case of consecutively processing a plurality of specimen containers 1.

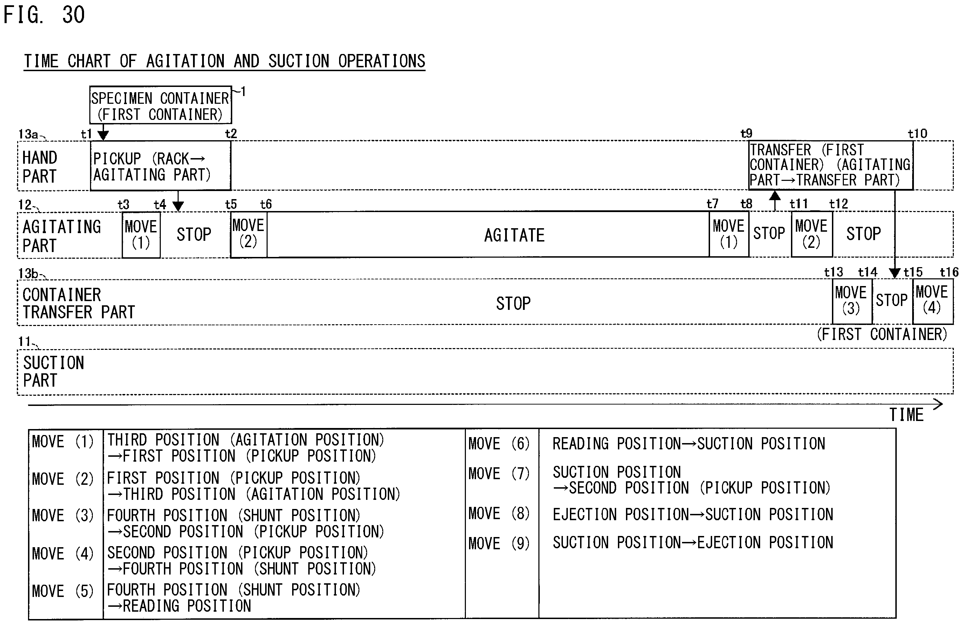

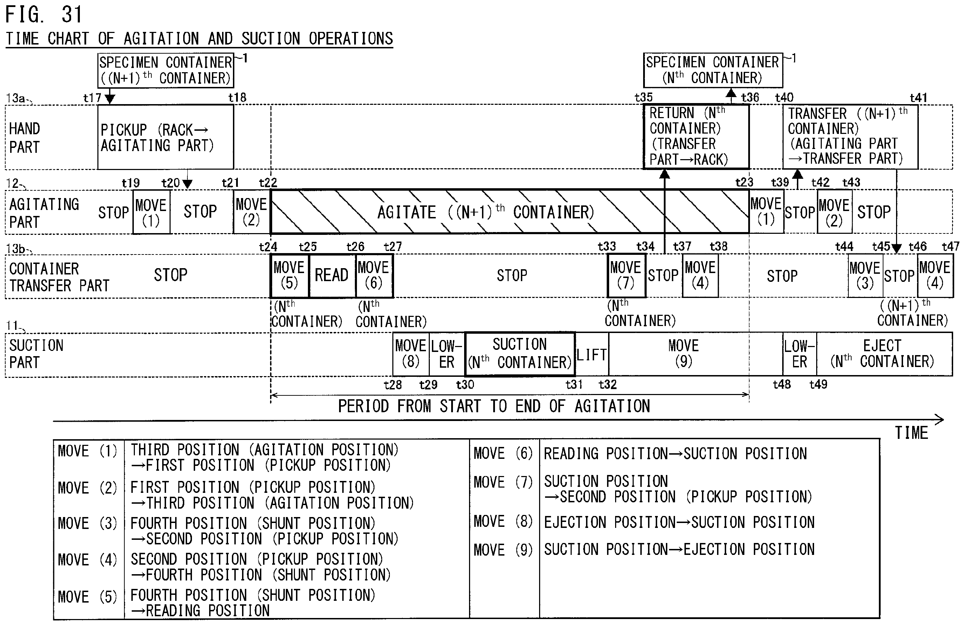

FIG. 30 shows the flow of a process in the case of taking in a first specimen container 1 in a state where no specimen container 1 is taken into the measurement unit 10. FIG. 31 shows the flow of a process in the case of taking in one specimen container 1 as a subsequent container in a state where another specimen container 1 is taken into the measurement unit 10. That is, FIG. 31 shows the flow of a process for the second or subsequent specimen container 1. In each of FIG. 30 and FIG. 31, the flow of time is shown from the left to right of the drawing, the hand part 13a, the agitating part 12, the container transfer part 13b, and the suction part 11 are arranged in the vertical direction in the drawing, and operations of these respective parts are separately indicated.

In FIG. 30, during a period from a time point t1 to a time point t2, the hand part 13a first takes out the first specimen container 1 placed at the take-out position P0 from the rack 2 and relays the first specimen container 1 to the agitating part 12. During this period, the agitating part 12 moves (move (1)) from the third position P3 to the first position P1 at a time point t3 after the hand part 13a takes out the specimen container 1 from the rack 2. During a period from a time point t4 (see FIG. 32) of completion of the movement to a time point t5, the specimen container 1 is inserted into the holding part 130 at the first position P1 from the hand part 13a. During a period from the time point t5 to a time point t6, the agitating part 12 moves (move (2)) from the first position P1 to the third position P3.

During a period from the time point t6 (see FIG. 33) to a time point t7, the agitating part 12 executes the agitation operation at the third position P3.

During a period from the time point t7 of end of the agitation to a time point t8 (see FIG. 34), the agitating part 12 moves (move (1)) from the third position P3 to the first position P1. During a period that is subsequent to the time point t8 of completion of the movement and that lasts from a time point t9 to a time point t10, the hand part 13a takes out, from the agitating part 12 placed at the first position P1, the specimen container 1 having been agitated and relays the specimen container 1 to the container transfer part 13b. Specifically, during a period from the time point t8 to a time point 111, the hand part 13a takes out the specimen container 1, and then, at the time point t11, the agitating part 12 moves (move (2)) from the first position P1 to the third position P3. After a time point t12 of completion of the movement of the agitating part 12, the container transfer part 13b moves (move (3)) from the fourth position P4 to the second position P2 at a time point t13. After a time point t14 (see FIG. 35) of completion of the movement, the hand part 13a inserts the specimen container 1 having been agitated into the container holding part 51 of the container transfer part 13b. During a period that is subsequent to the time point t10 of completion of the relaying and that lasts from a time point t15 to a time point t16, the container transfer part 13b moves from the second position P2 to the fourth position P4.

The above operations during a period from the time point t1 to the time point t16 are processing for the first specimen container 1, and there is no specimen container 1 to be subjected to agitation processing prior to the first specimen container 1. Thus, there is no operation that is executed concurrently with the agitation operation. After the time point t16, processing operations in FIG. 31 follow.

In FIG. 31, specimen containers 1 being processed are indicated as an "N^{th} container" and an "(N+1)^{th} container" in a generalized manner. However, description will be given here with N being 1, i.e., with the "N^{th} container" and the "(N+1)^{th} container" being respectively replaced with a first specimen container 1 and a second specimen container 1.

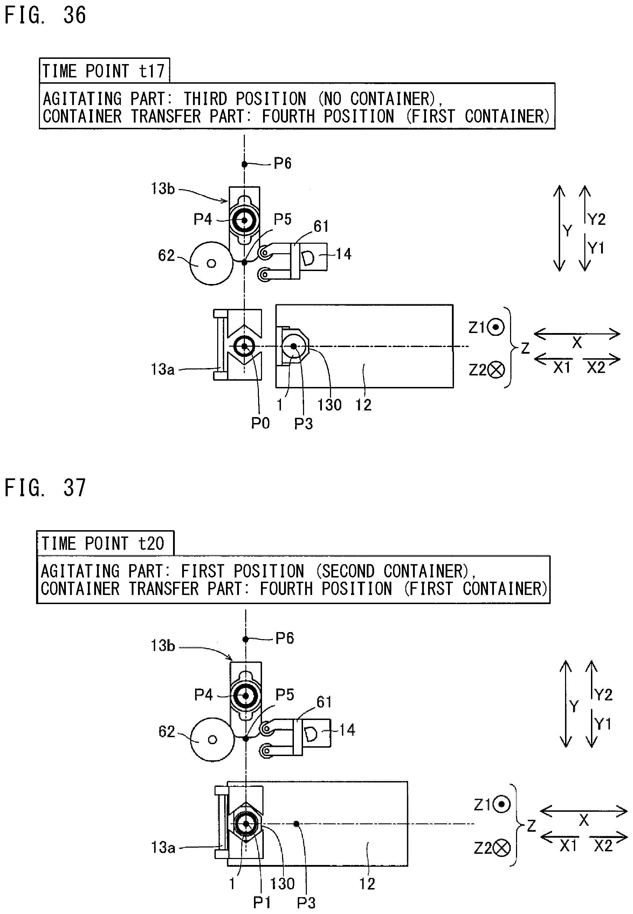

During a period that is subsequent to the movement of the container transfer part 13b to the fourth position P4 and that lasts from a time point t17 (see FIG. 36) to a time point t18, the hand part 13a takes out, from the rack 2, one subsequent specimen container 1 (second container) placed at the take-out position P0 and relays the one specimen container 1 to the agitating part 12. That is, step S1 is executed on the second specimen container 1.

During this period, the agitating part 12 moves (move (1)) from the third position P3 to the first position P1 at a time point t19 after the hand part 13a takes out the specimen container 1 from the rack 2. During a period from a time point t20 (see FIG. 37) of completion of the movement to a time point t21, the specimen container 1 is inserted into the holding part 130 of the agitating part 12 from the hand part 13a. During a period from the time point t21 to a time point t22, the agitating part 12 moves (move (2)) from the first position P1 to the third position P3. During a period from the time point t17 to the time point t22, the container transfer part 13b stands by at the fourth position P4 as shown in FIG. 37.

During a period from the time point t22 to a time point t23, the agitating part 12 executes the agitation operation. That is, the agitating part 12 performs the agitation operation on the one subsequent specimen container 1 (second container) in a state where the other specimen container 1 (first container) having been taken in first is held by the container transfer part 13b.

In FIG. 31, the following processing operations are concurrently executed on the other specimen container 1 (first container) during the period from the time point t22 to the time point t23 during which the agitation operation is being performed on the one specimen container 1 (second container).
- Transferring to the reading position P5 (from a time point t24 to a time point t25)
- Reading of information by the reading part 14 (from the time point t25 to a time point t26)
- Transferring to the suction position P6 (from the time point t26 to a time point t27)
- Suctioning of a specimen from the specimen container 1 placed at the suction position P6 (from a time point t30 to a time point t31)
- Transferring of the specimen container 1 having been subjected to suctioning of the specimen to the second position P2 (from a time point t33 to a time point t34)
- Returning of the specimen container 1 having been subjected to suctioning of the specimen to the rack 2 (from a time point t35 to a time point t36)

First, at the time point t24, the container transfer part 13b holding the other specimen container 1 (first container) moves (move (5)) the container holding part 51 from the fourth position P4 to the reading position P5. During a period from the time point t25 (see FIG. 38) of completion of the movement to the time point t26, the reading part 14 performs an operation of reading identification information on the other specimen container 1. At the time point t26 subsequent to end of the reading, the container transfer part 13b moves (move (6)) the container holding part 51 from the reading position P5 to the suction position P6.

During a period that is subsequent to completion of the movement and that lasts from a time point t28 (see FIG. 39) to a time point t29, the suction part 11 moves (move (8)) the suction tube 71 from an ejection position to the suction position P6. Then, during a period from the time point t29 to the time point t30, the suction part 11 moves the suction tube 71 in the downward direction from a position above the suction position P6 so as to insert the suction tube 71 into the specimen container 1. During a period from the time point t30 to the time point t31, the suction part 11 suctions, by the suction tube 71, the specimen contained in the other specimen container 1 (first container).

During a period that is subsequent to end of the suctioning and that lasts from the time point t31 to a time point t32, the suction part 11 moves the suction tube 71 (see FIG. 39) upward to the position above the specimen container 1. When the suction tube 71 is drawn out from the specimen container 1 after the suctioning of the specimen, the container transfer part 13b moves (move (7)) the container holding part 51 from the suction position P6 to the second position P2 during a period from the time point t33 to the time point t34.

During a period that is subsequent to completion of the movement and that lasts from the time point t35 (see FIG. 40) to the time point t36, the hand part 13a takes out, from the container holding part 51 of the container transfer part 13b placed at the second position P2, the other specimen container 1 (first container) having been subjected to suctioning of the specimen and returns the other specimen container 1 to the rack 2. That is, step S11 is executed. When the hand part 13a takes out the specimen container 1 from the container holding part 51 during a period from the time point t34 to the time point t35, the container transfer part 13b moves (move (4)) the container holding part 51 from the second position P2 to the fourth position P4 at a time point t37. After the time point t38 of completion of the movement of the container holding part 51, the hand part 13a inserts the specimen container 1 (first container) having been subjected to suctioning of the specimen to the holding position, of the rack 2, present at the take-out position P0.

The above operations are operations executed during the agitation operation for the one specimen container 1 (second container) by the agitating part 12.

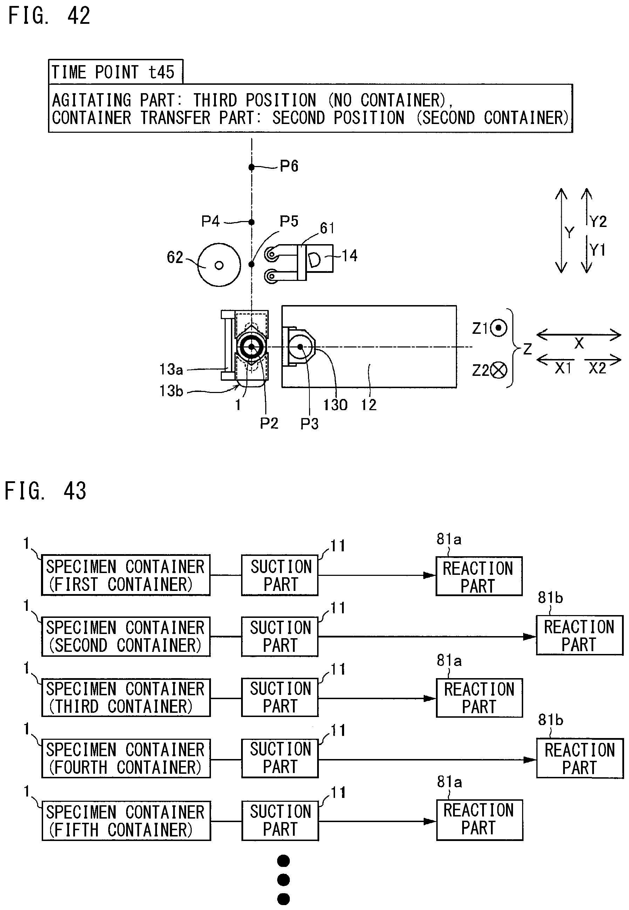

During a period that is subsequent to end of the agitation and that lasts from the time point t23 to a time point t39, the agitating part 12 moves (move (1)) from the third position P3 to the first position P1. Then, during a period from a time point t40 (see FIG. 41) to a time point t41, the hand part 13a relays the one specimen container 1 (second container) having been agitated, from the agitating part 12 to the container transfer part 13b. The operation during the period from the time point t40 to the time point t41 is the same as the operation during the period from the time point t9 to the time point t16 in FIG. 30. That is, during a period from the time point t39 to a time point t42, the hand part 13a takes out the specimen container 1, and, during a period from the time point t42 to a time point t43, the agitating part 12 moves (move (2)) from the first position P1 to the third position P3. During a period that is subsequent to completion of the movement of the agitating part 12 and that lasts from a time point t44 to a time point t45, the container transfer part 13b moves (move (3)) from the fourth position P4 to the second position P2, and, during a period from the time point t45 (see FIG. 42) to a time point t46, the hand part 13a inserts the specimen container 1 having been taken out into the container holding part 51 of the container transfer part 13b. During a period that is subsequent to the time point t41 of completion of the relaying and that lasts from the time point t46 to a time point t47, the container transfer part 13b moves from the second position P2 to the fourth position P4.

During a period from the time point t32 to a time point t48, the suction part 11 moves from the suction position P6 to the ejection position for any of the reaction parts (81a to 81e). Then, during a period from the time point t48 to a time point t49, the suction part 11 moves the suction tube 71 downward into the reaction part (81a to 81e). Then, from the time point t49, the suction part 11 performs an operation of ejecting the suctioned specimen (the specimen from the first specimen container 1).

Processing for third and subsequent specimen containers 1 is performed through repetition of the operations during the period from the time point t17 to the time point t47 shown in FIG. 31. Strictly speaking, during a period from the time point t17 to the time point t28, the suction part 11 performs an operation of completing an ejection operation and completing an operation of making ready for suctioning of a next specimen. This operation is not shown in this drawing.

Therefore, the specimen analyzer 100 is configured such that control by the controller 91 causes the following operations before agitation of the one specimen container 1 (third container). That is, during the period from the time point t40 to the time point t41, the hand part 13a transfers the other specimen container 1 (second container) having been agitated by the agitating part 12, from the agitating part 12 to the container transfer part 13b, and then, during a period from the time point t17 to the time point t18, the hand part 13a transfers the one specimen container 1 (third container) having yet to be agitated, from the rack 2 to the agitating part 12. Consequently, the hand part 13a can relay the other specimen container 1 having been agitated, from the agitating part 12 to the container transfer part 13b, and furthermore, the same hand part 13a can relay the one specimen container 1 having yet to be agitated, from the rack 2 to the agitating part 12. For example, an unnecessary operation does not need to be performed, and thus the processing efficiency can be improved as compared to a case where, after the other specimen container 1 is agitated by the agitating part 12, the specimen container 1 is returned to the rack 2 once, and then, relayed from the rack 2 to the container transfer part 13b.

When one specimen container 1 as an (N+1)^{th} container is taken in, the following operations are performed in the same manner as that for the third container. That is, during the agitation process (from the time point t22 to the time point t23) for the one specimen container 1 as the (N+1)^{th} container, another specimen container 1 as an N^{th} container is subjected to each of the operations of transferring to the reading position P5 (from the time point t24 to the time point t25), reading of information (from the time point t25 to the time point t26), transferring to the suction position P6 (from the time point t26 to the time point t27), suctioning of a specimen (from the time point t30 to the time point t31), transferring to the second position P2 (from the time point t33 to the time point t34), and returning to the rack 2 (from the time point t35 to the time point t36).

As described above, the specimen analyzer 100 in the present embodiment is configured such that control by the controller 91 causes, during the period from start (time point t22) to end (time point t23) of agitation of the one specimen container 1, the container transport part 13 to perform transferring of the other specimen container 1 to the suction position P6 (from the time point t26 to the time point t27). Thus, during the agitation of the one specimen container 1, the transferring of the other specimen container 1 to the suction position P6 can be concurrently executed, whereby the processing efficiency can be improved as compared to a case where the agitation and the transferring to the suction position P6 are separately executed.

In addition, the specimen analyzer 100 is configured such that control by the controller 91 causes, during the period from start (time point t22) to end (time point t23) of agitation of the one specimen container 1, completion of the transferring of the other specimen container 1 to the suction position P6 (from the time point t26 to the time point t27) and causes, during said period, the suction part 11 to start (time point t30) suctioning of a specimen from the other specimen container 1 transferred to the suction position P6. Thus, during the agitation of the one specimen container 1, the suctioning of the specimen from the other specimen container 1 can be concurrently executed, whereby the processing efficiency can be improved as compared to a case where the agitation and the suctioning of the specimen are separately executed.

In addition, the specimen analyzer 100 is configured such that control by the controller 91 causes, during the period from start (time point t22) to end (time point t23) of agitation of the one specimen container 1, completion of the transferring of the other specimen container 1 to the suction position P6 (from the time point t26 to the time point t27) and the suctioning of the specimen from the other specimen container 1 (from the time point t30 to the time point t31) and causes, during said period, the container transport part 13 to start (time point t35) returning of the other specimen container 1 having been subjected to the suctioning to the rack 2. Thus, during the agitation of the one specimen container 1, the returning of the other specimen container 1 to the rack 2 can be concurrently executed, whereby the processing efficiency can be improved as compared to a case where the agitation and the returning to the rack 2 are separately executed.

In addition, the specimen analyzer 100 is configured such that control by the controller 91 causes, during the period from start (time point t22) to end (time point t23) of agitation of the one specimen container 1, execution of: movement of the other specimen container 1 to the reading position P5 by the container transport part 13 (from the time point t24 to the time point t25); and reading of the identification information on the other specimen container 1 by the reading part 14 (from the time point t25 to the time point t26). Thus, during the agitation of the one specimen container 1, the reading of the identification information on the other specimen container 1 can be concurrently executed, whereby the processing efficiency can be improved as compared to a case where the agitation and the reading of the identification information are separately executed.

In addition, the specimen analyzer 100 is configured such that control by the controller 91 causes the hand part 13a to: return the other specimen container 1 (N^{th} container) having been subjected to suctioning by the suction part 11, from the container transfer part 13b to the rack 2 (from the time point t35 to the time point t36); and, after agitation of the one specimen container 1 ((N+1)^{th} container), transfer the one specimen container 1 having been agitated by the agitating part 12, from the agitating part 12 to the container transfer part 13b (from the time point t40 to the time point t41). Thus, the hand part 13a can perform an operation of: returning the other specimen container 1 (N^{th} container) having been subjected to suctioning, from the container transfer part 13b to the rack 2; and furthermore, relaying the one specimen container 1 ((N+1)^{th} container) having been agitated, from the agitating part 12 to the container transfer part 13b which has become vacant. Therefore, an unnecessary operation does not need to be performed, and thus the processing efficiency can be improved as compared to a case where, after the one specimen container 1 ((N+1)^{th} container) is agitated by the agitating part 12, the specimen container 1 is returned to the rack 2 once, and then, relayed from the rack 2 to the container transfer part 13b.

In addition, as shown in FIG. 42 and FIG. 40, the specimen analyzer 100 is configured such that control by the controller 91 causes the agitating part 12 to be placed at the third position P3 away from the second position P2 in the top view while the hand part 13a is, at the second position P2, performing insertion (from the time point t45 to the time point t46) and taking-out (from the time point t34 to the time point t37) of the specimen container 1 with respect to the container transfer part 13b. Furthermore, as shown in FIG. 37 and FIG. 41, the specimen analyzer 100 is configured such that control by the controller 91 causes the container transfer part 13b to be placed at the fourth position P4 away from the first position P1 in the top view while the hand part 13a is, at the first position P1, performing insertion (from the time point t20 to the time point t21) and taking-out (from the time point t39 to the time point t42) of the specimen container 1 with respect to the agitating part 12. Consequently, even in a case where insertion and taking-out of the specimen container 1 with respect to the agitating part 12 and insertion and taking-out of the specimen container 1 with respect to the container transfer part 13b are executed at positions (the first position P1 and the second position P2) coinciding with each other in the top view, it is possible to inhibit, while work is being performed on one of the container transfer part 13b and the agitating part 12, the other one of the container transfer part 13b and the agitating part 12 from hindering the work. When the container transfer part 13b (container holding part 51) is placed at a position that is more distant than the fourth position P4, the container transfer part 13b does not interfere with the agitating part 12, and thus, may be placed at the suction position P6 which is more distant than the fourth position P4 while the agitating part 12 is placed at the first position P1.

Next, an example of processing times in FIG. 31 will be described. In the present embodiment, 12 seconds are required for the controller 91 to execute agitation processing (from the time point t22 to the time point t23) for each specimen container 1. 12 seconds are required for the controller 91 to execute: movement to the reading position P5 and reading of information (from the time point t24 to the time point t26); movement to the suction position P6 (from the time point t26 to the time point t27); suctioning of a specimen (from the time point t30 to the time point t31); movement from the suction position P6 to the second position P2 (from the time point t33 to the time point t34); and returning of the specimen container 1 to the rack 2 (from the time point t35 to the time point t36). The controller 91 repetitively executes the operations during the period from the time point t17 to the time point t47 in a cycle of 18 seconds.

Therefore, in a case where the series of operations is repeated without parallel processing, the series of operations which are reading of information, suctioning of the specimen, and returning of the specimen container 1 to the rack 2 is further executed for 12 seconds after agitation processing is executed for 12 seconds. However, in the present embodiment, agitation processing for one specimen container 1, and each of reading of information about another specimen container 1, suctioning of a specimen from the other specimen container 1, and returning of the other specimen container 1 to the rack 2, are executed simultaneously and in a parallel manner, whereby the number of specimens to be processed per unit time can be increased.

Meanwhile, for a plurality of specimen containers 1, suctioning of a specimen is executed in a cycle of 18 seconds, and thus it is important to set also the cycle of sample preparation and measurement per specimen to fall within the cycle of 18 seconds.

To this end, the reaction part 81a and the reaction part 81b receive the same type of reagent in the present embodiment (see FIG. 19). That is, the reaction part 81a and the reaction part 81b are used for preparing measurement samples regarding the same measurement item. The controller 91 controls the suction part 11 to selectively eject, to one of the reaction part 81a and the reaction part 81b, a specimen suctioned from any of the specimen containers 1.

Consequently, a measurement sample is prepared in one of the reaction part 81a and the reaction part 81b, and, before an operation of making ready for reception of a next specimen is completed, a measurement sample can be prepared from the next specimen by using the other one of the reaction part 81a and the reaction part 81b. Therefore, in a case where agitation processing for one of the specimen containers 1 and processing for another one of the specimen containers 1 are executed as concurrent processing operations so that suctioning of a specimen in the one specimen container 1 and suctioning of a specimen in the other specimen container 1 are executed at a short time interval, it is also made possible for preparation of measurement samples to be executed in a parallel manner by the plurality of reaction parts 81, whereby the number of specimens to be processed per unit time can be effectively increased.

Specifically, as shown in FIG. 43, the controller 91 controls the suction part 11 to eject, to one of the reaction parts 81a and 81b (reaction part 81a), the specimen suctioned from the one specimen container 1 (for example, an odd-numbered container) and eject, to the other one of the reaction parts 81a and 81b (reaction part 81b), the specimen suctioned from the other specimen container 1 (for example, an even-numbered container) transferred to the suction position P6 next to the one specimen container 1. Consequently, in the case of consecutively measuring a plurality of specimens, the reaction part 81a and the reaction part 81b are used while switching therebetween is performed every time a specimen is suctioned from any of specimen containers 1, whereby the number of specimens to be processed per unit time can be effectively increased.

Thus, even in a case where the cycle of sample preparation per specimen cannot be set to fall within the cycle of 18 seconds with one reaction part, the cycle can be set to fall within the cycle of 18 seconds by, before completing an operation of making ready for the next sample preparation in the one reaction part 81a, preparing a sample from the next specimen by using the other reaction part 81b.

As shown in FIG. 19, the reaction part 81a and the reaction part 81b are connected to the same detector, i.e., the first detector 16a of the measurement part 16. Therefore, measurement of a measurement sample prepared from the specimen contained in the one specimen container 1 and measurement of a measurement sample prepared from the specimen contained in the other specimen container 1 are executed by the measurement part 16 (first detector 16a) which is common to the measurements. Consequently, even in a case where the reaction parts 81a and 81b for preparing measurement samples regarding the same measurement item are provided for two systems, there is no need to provide two sets of measurement parts 16 (first detectors 16a) for performing measurement, whereby the device configuration is not complicated.

### [Modifications]

The embodiment and examples disclosed herein are merely illustrative in all aspects and should not be considered as being restrictive. The scope of the present disclosure is defined not by the description of the above embodiment or examples but by the scope of the claims, and further includes meaning equivalent to the scope of the claims and all modifications within said scope.

For example, in the above embodiment, an example has been described in which, during agitation processing for the one specimen container 1 as the (N+1)^{th} container (from the time point t22 to the time point t23), each processing, i.e., each of transferring to the reading position P5 (from the time point t24 to the time point t25), reading of information (from the time point t25 to the time point t26), transferring to the suction position P6 (from the time point t26 to the time point t27), suctioning of a specimen (from the time point t30 to the time point t31), transferring to the second position P2 (from the time point t33 to the time point t34), and returning to the rack 2 (from the time point t35 to the time point t36), is performed on the other specimen container 1 as the N^{th} container. However, the present disclosure is not limited thereto. In the present disclosure, during the period from start to end of agitation of the one specimen container 1, only at least one of transferring to the suction position P6, suctioning of a specimen, and returning to the rack 2 has to be executed on the other specimen container 1. In this case as well, completion of the processing for the specimen containers 1 can be hastened by the period of said operation that can be executed on the other specimen container 1 during the agitation of the one specimen container 1. Consequently, the number of specimens to be processed per unit time by the specimen analyzer 100 in the case of analyzing a plurality of specimens can be increased.

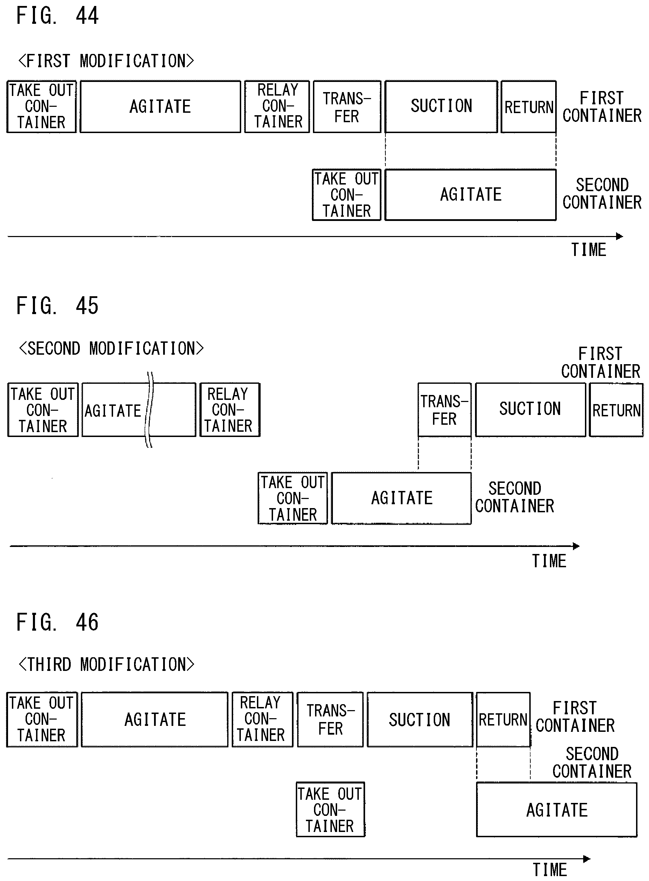

For example, a first modification shown in FIG. 44 indicates an example in which, during the period from start to end of agitation of the one specimen container 1 (second container), suctioning of a specimen and returning to the rack 2 are performed on the other specimen container 1 (first container). In the first modification, during the agitation, transferring to the suction position P6 is not performed on the other specimen container 1.

A second modification shown in FIG. 45 indicates an example in which, during the period from start to end of agitation of the one specimen container 1 (second container), transferring to the suction position P6 is performed on the other specimen container 1 (first container). In the second modification, during the agitation, neither suctioning of a specimen nor returning to the rack 2 is performed on the other specimen container 1.

A third modification shown in FIG. 46 indicates an example in which, during the period from start to end of agitation of the one specimen container 1 (second container), returning to the rack 2 is performed on the other specimen container 1 (first container). In the second modification, during the agitation, neither transferring to the suction position P6 nor suctioning of a specimen is performed on the other specimen container 1.

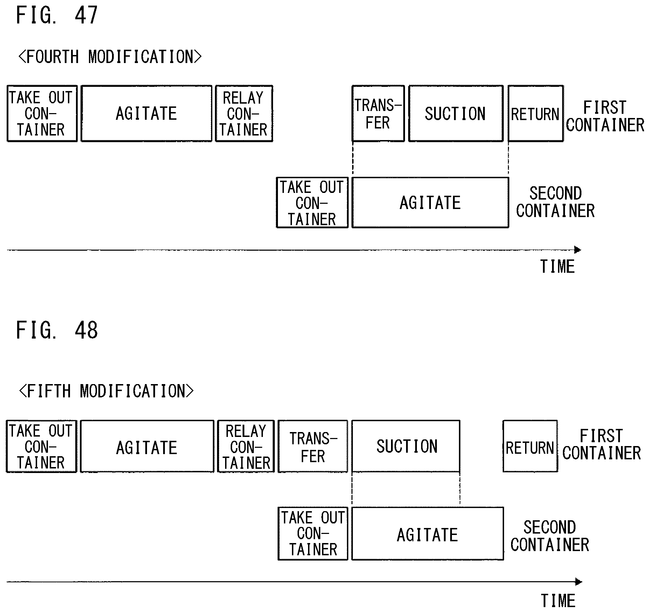

A fourth modification shown in FIG. 47 indicates an example in which, during the period from start to end of agitation of the one specimen container 1 (second container), transferring to the suction position P6 and suctioning of a specimen are performed on the other specimen container 1 (first container). In the fourth modification, during the agitation, returning to the rack 2 is not performed on the other specimen container 1.

A fifth modification shown in FIG. 48 is an example in which, during the period from start to end of agitation of the one specimen container 1 (second container), suctioning of a specimen is performed on the other specimen container 1 (first container). In the fifth modification, during the agitation, neither transferring to the suction position P6 nor returning to the rack 2 is performed on the other specimen container 1.

In addition, in the above embodiment, an example has been described in which the first position P1 at which each specimen container 1 is taken out from or put into the agitating part 12, the second position P2 at which the specimen container 1 is taken out from or put into the container transfer part 13b, and the take-out position P0 for taking out the specimen container 1 from the rack 2 are aligned in the up-down direction. However, the present disclosure is not limited thereto. The first position P1, the second position P2, and the take-out position P0 do not have to be aligned in the up-down direction. The first position P1, the second position P2, and the take-out position P0 may be present at positions different from one another in the top view. The first position P1 and the second position P2 may be the same position in the top view, and the take-out position P0 may be a position different from the first position P1 and the second position P2 in the top view. Alternatively, one of the first position P1 and the second position P2 may be the same position as the take-out position P0 in the top view, and the other one of the first position P1 and the second position P2 may be a position different from the take-out position P0 in the top view.

In addition, in the above embodiment, an example has been described in which the container transfer part 13b linearly moves in the Y direction, and the agitating part 12 linearly moves in the X direction. However, the present disclosure is not limited thereto. The container transfer part 13b may move in a direction other than the Y direction and may move in a curved manner. Likewise, the agitating part 12 may move in a direction other than the X direction and may move in a curved manner. The container transfer part 13b and the agitating part 12 may move in directions parallel to each other. In this case, the hand part 13a may be configured to be horizontally movable so as to extend on and between the container transfer part 13b and the agitating part 12. Alternatively, a mechanism for relaying the specimen container 1 between the container transfer part 13b and the agitating part 12 may be provided separately from the hand part 13a.

In addition, in the above embodiment, an example has been described in which the hand part 13a performs taking-out and insertion of the specimen container 1 with respect to each of the rack 2, the agitating part 12, and the container transfer part 13b, through upward/downward movement without moving in the horizontal direction. However, the present disclosure is not limited thereto. The hand part 13a may be configured to move not only upward/downward but also horizontally. For example, the agitating part 12 may be fixed at the third position P3, and the hand part 13a may move horizontally between the take-out position P0 and the third position P3 in the top view.

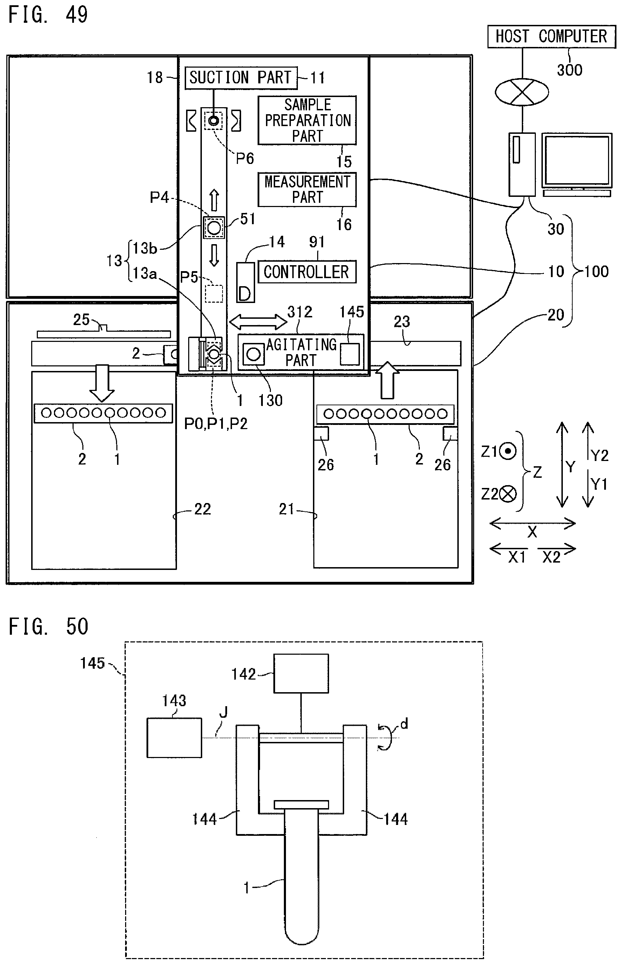

In addition, in the above embodiment, an example has been described in which the agitating part 12 includes the holding part 130 which holds the lower portion of the specimen container 1 and the drive part 121 which causes reciprocating movement of the holding part 130 in the predetermined direction. However, the present disclosure is not limited thereto. The agitating part 12 may be configured to perform agitation in a manner in which: the upper portion of the specimen container 1 is held such that the specimen container 1 is hung; and the specimen container 1 is rotated around a horizontal axis as a pendulum. FIG. 49 is a schematic diagram showing a specimen analyzer for performing agitation in a modification. As shown in FIG. 49, the specimen analyzer 100 for performing agitation in the modification includes an agitating part 312 instead of the agitating part 12. The agitating part 312 linearly moves the holding part 130 in the horizontal direction (X direction) in the same manner as the agitating part 12. The agitating part 312 includes a gripping/agitating part 145. FIG. 50 is a schematic diagram showing the gripping/agitating part. The gripping/agitating part 145 includes: a pair of hand parts 144 which grip the specimen container 1; a drive source 142 for performing an opening/closing operation of the pair of hand parts 144; and a drive source 143 which causes reciprocating rotation (see an arrow d) of the pair of hand parts 144 around a rotation axis J.

In addition, in the above embodiment, an example has been described in which the drive part 121 causes reciprocating rotation of the holding part 130 around the rotation shaft 125 extending in the horizontal direction. However, the present disclosure is not limited thereto. The drive part 121 may be configured to perform agitation by rotating the holding part 130 around a rotation shaft extending in a direction other than the horizontal direction, e.g., the vertical direction. Alternatively, the agitating part 12 does not have to rotate the holding part 130 around a specific rotation shaft. For example, the agitating part 12 may include a linear motion mechanism for causing reciprocating movement of the holding part 130 in a linear trajectory and may perform agitation by causing linear reciprocating movement of the holding part 130.

In addition, in the above embodiment, an example has been described in which the drive part 121 rotates the holding part 130 from the origin position Q0 to the predetermined angle Q1 larger than 90 degrees. However, the present disclosure is not limited thereto. In the present disclosure, the predetermined angle Q1 may be 90 degrees or smaller. The predetermined angle Q1 may be another angle other than 140 degrees. When the origin position Q0 is defined as the horizon (0 degrees), the upper limit of the predetermined angle Q1 is, for example, 270 degrees. When the predetermined angle Q1 is larger than 270 degrees, the upper end portion (cap 1a) of the specimen container 1 is oriented upward, whereby it becomes difficult to cause adhesion matter to fall off. In a case where the fall-off object reception part 115 is not provided, the predetermined angle Q1 may be any angle smaller than 360 degrees. The drive part 121 may cause, instead of reciprocating rotation, rotation by 360 degrees or larger in a direction around the rotation shaft.

In addition, in the above embodiment, an example has been described in which the fall-off object reception part 115 for receiving adhesion matter having fallen off from the specimen container 1 is provided at a position that is present below the upper end portion of the specimen container 1 in a state of having been rotated to the predetermined angle Q1. However, the present disclosure is not limited thereto. In the present disclosure, the fall-off object reception part 115 does not have to be provided.

In addition, in the above embodiment, an example has been described in which the agitating part 12 is configured to abruptly stop rotation of the holding part 130 when the holding part 130 reaches the predetermined angle Q1 from the origin position Q0. However, the present disclosure is not limited thereto. The agitating part 12 may slowly stop rotation of the holding part 130.

In addition, in the above embodiment, an example has been described in which: the arm 124 of the agitating part 12 is connected to the holding part 130 in a state of allowing relative movement in the rotation direction by the predetermined amount CL; and, when the arm 124 is stopped, the holding part 130 moves by the predetermined amount CL and collides with the arm 124 owing to inertia, to be abruptly stopped. However, the present disclosure is not limited thereto. In the present disclosure, the arm 124 and the holding part 130 may be connected so as not to move relative to each other. In the case of abruptly stopping the holding part 130, a stopper that is to collide with a rotating portion (the arm 124 or the holding part 130) may be separately provided to the agitating part 12. Alternatively, a braking device may be provided to the pulley 123b which is rotated together with the arm 124, and rotation of the pulley 123b may be forcedly stopped so as to abruptly stop the holding part 130. The holding part 130 may be abruptly stopped through only drive control of the rotation motor 122

In addition, in the above embodiment, an example has been described in which the holding part 130 is abruptly stopped at the time of end of agitation. However, the present disclosure is not limited thereto. In the present disclosure, the holding part 130 does not have to be abruptly stopped at the origin position Q0 at the time of end of agitation.

In addition, in the above embodiment, an example has been described in which: the plurality of reaction parts 81a and 81b which receive the same type of reagent are provided; and the specimen suctioned from the specimen container 1 is selectively ejected to one of the plurality of reaction parts 81a and 81b. However, the present disclosure is not limited thereto. In the present disclosure, the number of provided reaction parts which receive the same type of reagent may be only one or may be three or more.

In the above embodiment, an example has been described in which the slide drive part 113 which moves the holding part 130 of the agitating part 12 to the first position P1 and the third position P3 is an air cylinder. However, the present disclosure is not limited thereto. The slide drive part 113 may be an electric motor. In this case, drive force of the slide drive part 113 is converted into drive force in the front-rear direction by a belt-pulley mechanism, whereby the slide part 120 can be moved.

In the above embodiment, the measurement unit 10 includes the reading part 14, and the container transport part 13 positions the specimen container 1 at the reading position P5, whereby identification information on the specimen container 1 is read. However, reading of identification information in the measurement unit 10 may be omitted. That is, the measurement unit 10 does not have to include the reading part 14. In this case, the identification information read by the information reading part 24 may be used for identifying the specimen in the specimen container 1.

In the above embodiment, the controller 91 controls operation of each of the parts of the measurement unit 10 and the rack transport part 20. However, the controller 31 may control operations of some of the parts of the measurement unit 10 and the rack transport part 20. Alternatively, the measurement unit 10 does not have to include the controller 91, and the controller 31 may control the operations of the measurement unit 10 and the rack transport part 20. The analysis part 30 does not have to include the controller 31, and the controller 91 may perform analysis processing for measurement data.

In the above embodiment, an example has been described in which the specimen analyzer 100 is provided with one measurement unit 10. However, the present disclosure is not limited thereto. The specimen analyzer 100 may be provided with a plurality of measurement units 10. For example, with respect to the one rack transport part 20, two measurement units 10 may be arranged side-by-side.

In the above embodiment, an example has been described in which specimens contained in a plurality of specimen containers 1 (ordinary specimens) held by a rack 2 transported by the rack transport part 20 are measured. However, the present disclosure is not limited thereto. The specimen analyzer 100 may measure, in preference to the plurality of specimen containers 1 (ordinary specimens) held by the rack 2 transported by the rack transport part 20, a specimen contained in a specimen container 1 (interruption specimen) having been placed on the container holding part 51 by a user.

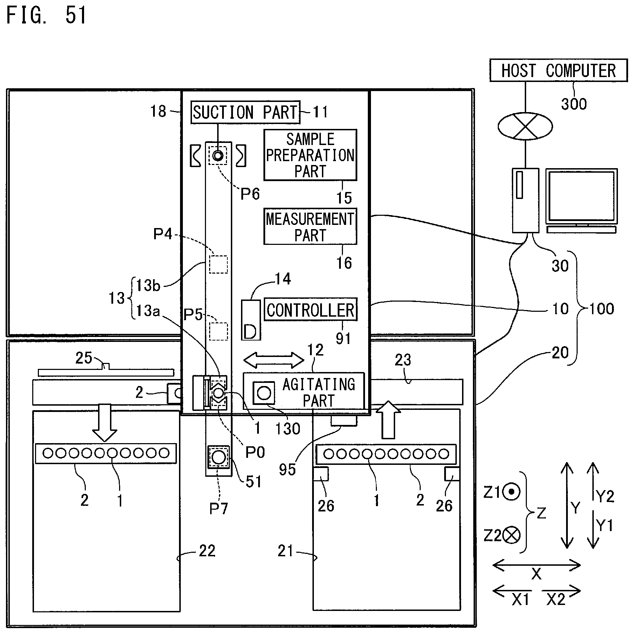

FIG. 51 is a schematic diagram showing a specimen analyzer in a sixth modification. In the specimen analyzer 100 in the present modification, the container transfer part 13b is configured such that the container holding part 51 is movable to a container placement position P7 on the front side (Y1 direction side) relative to the front surface portion on the Y1 side of the unit cover 18. Consequently, the user can place the specimen container 1 (interruption specimen) onto the container holding part 51 having moved to the container placement position P7. The specimen analyzer 100 in the present modification includes, on the front surface portion on the Y1 side of the unit cover 18, a switch 95 for performing switching from an ordinary specimen measurement mode to an interruption specimen measurement mode and starting measurement of the interruption specimen.

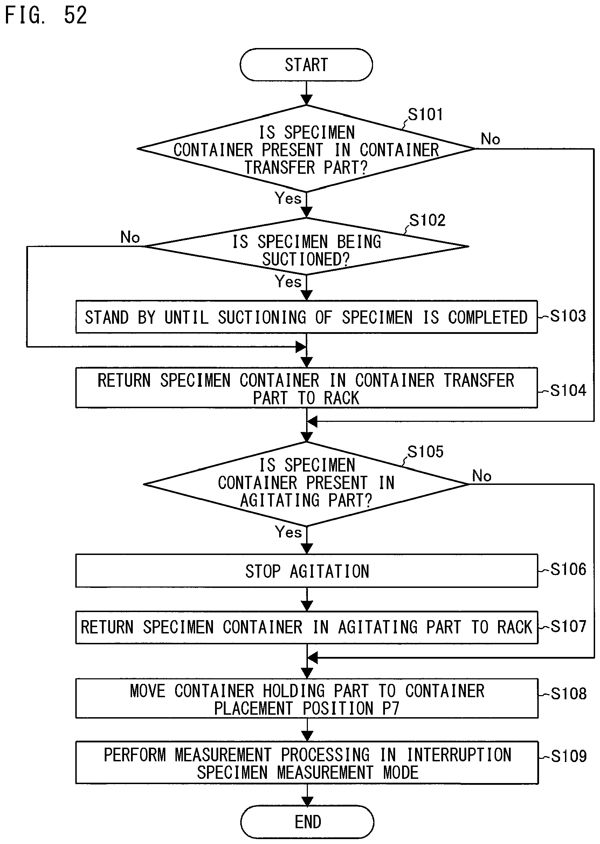

FIG. 52 is a flowchart showing a measurement process for the interruption specimen to be executed by the controller of the specimen analyzer in the sixth modification. The measurement process for the interruption specimen is started when the user presses the switch 95. When the measurement process for the interruption specimen is started, the controller 91 determines in step S101 whether or not a specimen container 1 is present in the container transfer part 13b. The determination as to whether or not a specimen container 1 is present in the container transfer part 13b may be performed on the basis of an output from a sensor for detecting a specimen container 1 held by the container holding part 51 or may be performed by means of software. In the case of performing the determination by means of software, the controller 91 may perform the determination according to, for example, whether or not any of the operations in steps S4 to S11 (FIG. 21) is being executed. In a case where any of the operations in steps S4 to S11 is being executed, the controller 91 determines that a specimen container 1 is present in the container transfer part 13b. Meanwhile, in a case where none of the operations in steps S4 to S11 is being executed, the controller 91 determines that no specimen container 1 is present in the container transfer part 13b.

In a case where the controller 91 determines in step S101 that a specimen container 1 is present in the container transfer part 13b (step S101: Yes), the controller 91 determines in step S102 whether or not a specimen is being suctioned. The determination as to whether or not a specimen is being suctioned may be performed according to, for example, whether or not the operation in step S9 (FIG. 21) is being executed. In a case where the operation in step S9 is being executed, the controller 91 determines that a specimen is being suctioned. Meanwhile, in a case where the operation in step S9 is not being executed, the controller 91 determines that no specimen is being suctioned. In a case where the controller 91 determines in step S102 that a specimen is being suctioned (step S102: Yes), the controller 91 stands by in step S103 until suctioning of the specimen is completed. Meanwhile, in a case where the controller 91 determines in step S102 that no specimen is being suctioned (step S102: No), or after step S103 is executed, the controller 91 causes the specimen container 1 present in the container transfer part 13b to be returned to the original holding hole 2a of the original rack 2 in step S104. Through this processing, the specimen container 1 held by the container holding part 51 is placed at the second position P2, and the hand part 13a returns this specimen container 1 to the rack 2 transported to the position at which this specimen container 1 has been taken out.

In a case where the controller 91 determines in step S101 that no specimen container is present in the container transfer part 13b (step S101: No), or after step S104 is executed, the controller 91 determines in step S105 whether or not a specimen container 1 is present in the agitating part 12. The determination as to whether or not a specimen container 1 is present in the agitating part 12 is performed on the basis of an output from a sensor (container detector 137) for detecting a specimen container 1 held by the holding part 130 or may be performed by means of software. In the case of performing the determination by means of software, the controller 91 may perform the determination according to, for example, whether or not any of the operations in steps S1 to S3 (FIG. 21) is being executed. In a case where any of the operations in steps S1 to S3 is being executed, the controller 91 determines that a specimen container 1 is present in the agitating part 12. Meanwhile, in a case where none of the operations in steps S1 to S3 is being executed, the controller 91 determines that no specimen container 1 is present in the agitating part 12.

In a case where the controller 91 determines in step S105 that a specimen container is present in the agitating part 12 (step S105: Yes), the controller 91 causes the agitating part 12 to stop agitating the specimen container 1 in step S106 even when the predetermined number of times of agitation has not yet been completed. Through this processing, the agitating part 12 is stopped in a state where the specimen container 1 held by the holding part 130 is placed at the third position P3. In step S107, the controller 91 causes the specimen container 1 in the agitating part 12 to be returned to the original holding hole 2a of the original rack 2. Through this processing, the specimen container 1 held by the holding part 130 is placed at the first position P1, and the hand part 13a returns this specimen container 1 to the rack 2 transported to the position at which this specimen container 1 has been taken out. Alternatively, the processing in step S107 may be executed after step S108 is executed.

In a case where the controller 91 determines in step S105 that no specimen container 1 is present in the agitating part 12 (step S105: No), or after step S107 is executed, the controller 91 controls the container transfer part 13b such that the container holding part 51 moves to the container placement position P7 in step S108. Next, processing in step S109 is started when the user places an agitated interruption specimen into the container holding part 51 placed at the container placement position P7 and presses the switch 95. In step S109, the controller 91 executes measurement processing in the interruption specimen measurement mode. Specifically, in step S109, the controller 91 causes the container transfer part 13b to move the interruption specimen placed in the container holding part 51 from the container placement position P7 to the reading position P5, and then, controls the suction part 11, the hand part 13a, the container transfer part 13b, the reading part 14, the sample preparation part 15, and the measurement part 16 to execute the operations in steps S7 to S14 (FIG. 21).

In this manner, the specimen analyzer 100 in the sixth modification can be operated in a first mode of measuring a plurality of ordinary specimens accommodated in the rack 2 on the rack transport part 20 and a second mode of measuring an interruption specimen in preference to the plurality of ordinary specimens. The controller 91 is configured to, when the second mode is set, perform control to return the ordinary specimens present in the agitating part 12 and the container transport part 13 to the rack 2. That is, in the case of measuring the interruption specimen, each specimen container 1 taken into the unit cover 18 is returned to the corresponding original holding hole 2a of the original rack 2. Consequently, the specimen analyzer in the sixth modification can swiftly perform measurement of the interruption specimen even during measurement of the plurality of ordinary specimens.

In the above embodiment, an example has been described in which the specimen analyzer 100 is a blood cell counter. However, the specimen analyzer 100 may be another type of analyzer such as an immunoassay device, a biochemical measurement device, a blood coagulation measurement device, or a urine analyzer. Therefore, the specimen in the present disclosure is not limited to a blood specimen and may be urine, tissue fluid, another body fluid (such as cerebrospinal fluid, ascitic fluid, pleural fluid, synovial fluid, or peritoneal dialysis effluent), or the like.

### [Remarks]

The present disclosure includes the following items 1-44.

Item 1: A specimen analyzer comprising:
a rack transport part configured to transport a rack accommodating a plurality of specimen containers;
an agitating part configured to agitate a specimen in each of the specimen containers;
a suction part configured to suction the specimen from the specimen container;
a container transport part configured to transport the specimen container between the rack on the rack transport part, the agitating part, and a suction position for specimen suctioning by the suction part;
a measurement part configured to measure a measurement sample prepared from the specimen suctioned by the suction part; and
a controller configured to control at least the agitating part, the suction part, and the container transport part, wherein
the specimen analyzer is configured such that control by the controller causes, during a period from start to end of agitation of one of the specimen containers, at least one of transferring to the suction position, suctioning of a specimen, and returning to the rack to be executed on another one of the specimen containers.

Item 2: The specimen analyzer of item 1, wherein
the container transport part includes
a hand part capable of being placed above the rack transport part and configured to grip each of the specimen containers and move in at least an up-down direction, and
a container transfer part configured to receive, from the hand part, the specimen container having been agitated by the agitating part and transfer the specimen container to the suction position, and
the agitating part receives, from the hand part, the specimen container having been taken out from the rack and agitates the specimen in the specimen container.

Item 3: The specimen analyzer of item 2, wherein
the specimen analyzer is configured such that control by the controller causes, before agitation of the one specimen container, the hand part to: transfer the other specimen container having been agitated by the agitating part, from the agitating part to the container transfer part; and transfer the one specimen container having yet to be agitated, from the rack to the agitating part.

Item 4: The specimen analyzer of item 2, wherein
the specimen analyzer is configured such that control by the controller causes the hand part to: return the other specimen container having been subjected to suctioning by the suction part, from the container transfer part to the rack; and, after agitation of the one specimen container, transfer the one specimen container having been agitated by the agitating part, from the agitating part to the container transfer part.

Item 5: The specimen analyzer of any one of items 2 to 4, wherein
the agitating part is configured to move to a first position,
the container transfer part is configured to move to a second position that coincides with the first position in a top view, and
the hand part is configured to execute, at the first position, insertion and taking-out of the specimen container with respect to the agitating part and execute, at the second position, insertion and taking-out of the specimen container with respect to the container transfer part.

Item 6: The specimen analyzer of item 5, wherein
the specimen analyzer is configured such that
control by the controller causes the agitating part to be placed at a third position away from the second position in the top view while the hand part is, at the second position, performing insertion and taking-out of the specimen container with respect to the container transfer part, and
control by the controller causes the container transfer part to be placed at a fourth position away from the first position in the top view while the hand part is, at the first position, performing insertion and taking-out of the specimen container with respect to the agitating part.

Item 7: The specimen analyzer of item 6, wherein
the first position, the second position, and a take-out position for taking out the specimen container from the rack are aligned in the up-down direction, and
the hand part is configured to perform taking-out and insertion of the specimen container with respect to the rack, insertion and taking-out of the specimen container with respect to the agitating part placed at the first position, and insertion and taking-out of the specimen container with respect to the container transfer part placed at the second position, through upward/downward movement without moving in a horizontal direction.

Item 8: The specimen analyzer of item 7, wherein
the agitating part is configured to linearly move to the first position and the third position along a first direction in a horizontal plane,
the container transfer part is configured to linearly move to the second position and the fourth position along a second direction intersecting with the first direction in the top view, and
the first position and the second position are each a position at which a movement path of the container transfer part and a movement path of the agitating part intersect with each other in the top view.

Item 9: The specimen analyzer of any one of items 1 to 4, wherein
the specimen analyzer is configured such that control by the controller causes, during the period from start to end of agitation of the one specimen container, the container transport part to perform transferring of the other specimen container to the suction position.

Item 10: The specimen analyzer of item 9, wherein
the specimen analyzer is configured such that control by the controller causes, during the period from start to end of agitation of the one specimen container, completion of the transferring of the other specimen container to the suction position and causes, during said period, the suction part to start suctioning of the specimen from the other specimen container transferred to the suction position.

Item 11: The specimen analyzer of item 10, wherein
the specimen analyzer is configured such that control by the controller causes, during the period from start to end of agitation of the one specimen container, completion of the transferring of the other specimen container to the suction position and the suctioning of the specimen from the other specimen container and causes, during said period, the container transport part to start returning of the other specimen container having been subjected to the suctioning to the rack.

Item 12: The specimen analyzer of item 11, further comprising
a reading part configured to read identification information provided on each of the specimen containers, wherein
the container transport part transports the specimen container to a reading position for reading by the reading part, and
the specimen analyzer is configured such that control by the controller causes, during the period from start to end of agitation of the one specimen container, execution of: movement of the other specimen container to the reading position by the container transport part; and reading of the identification information on the other specimen container by the reading part.

Item 13: The specimen analyzer of item 2, further comprising
a storage configured to store therein a state of the agitating part and a state of the container transfer part, wherein
the controller
   updates the state of the agitating part stored in the storage, according to transition of the state of the agitating part,
   updates the state of the container transfer part stored in the storage, according to transition of the state of the container transfer part,
   controls operation of the agitating part according to the state of the container transfer part stored in the storage, and
   controls operation of the container transfer part according to the state of the agitating part stored in the storage.

Item 14: The specimen analyzer of any one of items 1 to 4, wherein
the agitating part includes
a holding part configured to hold a lower portion of each of the specimen containers and
a drive part configured to cause reciprocating movement of the holding part in a predetermined direction.

Item 15: The specimen analyzer of item 14, wherein
the drive part is configured to cause reciprocating rotation of the holding part around a rotation shaft,
the holding part is configured to hold the specimen container oriented such that a longitudinal direction of the specimen container extends along a tangent line direction to a rotation trajectory around the rotation shaft, and
the agitating part further includes a fixation part configured to fix the specimen container placed in the holding part and release the fixed specimen container.

Item 16: The specimen analyzer of item 15, wherein
the drive part is configured to cause reciprocating rotation of the holding part around the rotation shaft extending in a horizontal direction, and
the holding part is configured to hold the lower portion of the specimen container oriented so as to extend along an up-down direction at an origin position for the reciprocating rotation.

Item 17: The specimen analyzer of item 16, wherein
the controller controls the drive part to rotate the holding part from the origin position to a predetermined angle larger than 90 degrees, and
the agitating part further includes a fall-off object reception part disposed at a position below an upper end portion of the specimen container in a state of having been rotated to the predetermined angle, the fall-off object reception part being configured to receive adhesion matter having fallen off from the specimen container.

Item 18: The specimen analyzer of item 17, wherein
the agitating part is configured to abruptly stop rotation of the holding part when the holding part reaches the predetermined angle from the origin position.

Item 19: The specimen analyzer of item 18, wherein
the drive part is connected to the holding part via an arm rotatable around the rotation shaft,
the arm is connected to the holding part in a state of allowing relative movement in a rotation direction by a predetermined amount, and,
when the arm rotated to the predetermined angle is stopped, the holding part moves by the predetermined amount and collides with the arm owing to inertia, to be abruptly stopped.

Item 20: The specimen analyzer of item 19, wherein
the controller is configured to perform control to abruptly stop rotation of the holding part when the holding part reaches the origin position from the predetermined angle at a time of end of agitation.

Item 21: The specimen analyzer of any one of items 1 to 4, further comprising
a plurality of reaction parts each configured to receive the specimen suctioned by the suction part and a reagent and react the specimen and the reagent with each other, to prepare the measurement sample, wherein
the plurality of reaction parts receive a same type of reagent, and
the controller controls the suction part to selectively eject, to one of the plurality of reaction parts, the specimen suctioned from each of the specimen containers.

Item 22: The specimen analyzer of item 21, wherein
the controller controls the suction part to
eject, to one of the plurality of reaction parts, the specimen suctioned from the one specimen container and
eject, to another one of the plurality of reaction parts, the specimen suctioned from the other specimen container transferred to the suction position next to the one specimen container.

Item 23: The specimen analyzer of item 1, wherein
the specimen analyzer is configured such that measurement of a measurement sample prepared from the specimen contained in the one specimen container and measurement of a measurement sample prepared from the specimen contained in the other specimen container are executed by the measurement part which is common to the measurements.

Item 24: The specimen analyzer of item 1, wherein
the specimen analyzer can be operated in
   a first mode of measuring a plurality of ordinary specimens accommodated in the rack on the rack transport part and
   a second mode of measuring an interruption specimen in preference to the plurality of ordinary specimens, and
the specimen analyzer is configured such that control by the controller causes, when the second mode is set, the ordinary specimens present in the agitating part and the container transport part to be returned to the rack.

Item 25: A specimen analysis method to be performed by a specimen analyzer configured to analyze a specimen contained in a specimen container, the specimen analysis method comprising:
taking out, from a rack accommodating a plurality of the specimen containers, any of the specimen containers;
agitating a specimen in the specimen container having been taken out;
transferring, to a suction position, the specimen container having been agitated;
suctioning the specimen from the specimen container transferred to the suction position;
returning, to the rack, the specimen container having been subjected to the suctioning of the specimen; and
measuring a measurement sample prepared from the suctioned specimen, wherein,
during a period from start to end of the agitating of one of the specimen containers, at least one of the transferring to the suction position, the suctioning of a specimen, and the returning to the rack is executed on another one of the specimen containers.

Item 26: The specimen analysis method of item 25, wherein
the taking-out, the agitating, the transferring, the suctioning, and the returning are executed on each of the plurality of specimen containers, and,
before the agitating is executed on the one specimen container, the transferring is executed on the other specimen container.

Item 27: The specimen analysis method of item 25 or 26, further comprising:
performing, in a state where a container transfer part configured to transfer each of the specimen containers between a second position and the suction position is placed at the second position, insertion and taking-out of the specimen container with respect to the container transfer part; and
performing, in a state where an agitating part configured to agitate the specimen container is placed at a first position that coincides with the second position in a top view, insertion and taking-out of the specimen container with respect to the agitating part.

Item 28: The specimen analysis method of item 27, wherein
the performing of insertion and taking-out of the specimen container with respect to the container transfer part comprises performing insertion and taking-out of the specimen container with respect to the container transfer part in a state where the container transfer part is moved to the second position and the agitating part is moved to a third position away from the second position, and
the performing of insertion and taking-out of the specimen container with respect to the agitating part comprises performing insertion and taking-out of the specimen container with respect to the agitating part in a state where the agitating part is moved to the first position and the container transfer part is moved to a fourth position away from the first position.

Item 29: The specimen analysis method of item 28, wherein
the first position, the second position, and a take-out position for taking out the specimen container from the rack are aligned in an up-down direction, and
the taking-out of the specimen container from the rack and insertion of the specimen container into the rack, the insertion and taking-out of the specimen container with respect to the agitating part placed at the first position, and the insertion and taking-out of the specimen container with respect to the container transfer part placed at the second position are performed through only upward movement.

Item 30: The specimen analysis method of item 29, further comprising:
linearly moving the agitating part along a first direction in a horizontal plane, to place the agitating part at each of the first position and the third position; and
linearly moving the container transfer part along a second direction intersecting with the first direction in the top view, to place the container transfer part at each of the second position and the fourth position, wherein
the first position and the second position are each a position at which a movement path of the container transfer part and a movement path of the agitating part intersect with each other in the top view.

Item 31: The specimen analysis method of item 25 or 26, wherein
the taking-out, the agitating, the transferring to the suction position, the suctioning, and the returning are executed on each of the plurality of specimen containers, and,
during the period from start to end of the agitating of the one specimen container, the transferring to the suction position is executed on the other specimen container.

Item 32: The specimen analysis method of item 31, wherein,
during the period from start to end of the agitating of the one specimen container, the transferring to the suction position is completed with respect to the other specimen container, and the suctioning is started with respect to the other specimen container transferred to the suction position.

Item 33: The specimen analysis method of item 32, wherein,
during the period from start to end of the agitating of the one specimen container, the transferring to the suction position and the suctioning are completed with respect to the other specimen container, and the returning is started with respect to the other specimen container having been subjected to the suctioning of the specimen.

Item 34: The specimen analysis method of item 33, further comprising:
transferring each of the specimen containers to a reading position at which identification information provided on the specimen container is read; and
reading the identification information, wherein,
during the period from start to end of the agitating of the one specimen container, the transferring to the reading position and the reading are further executed on the other specimen container.

Item 35: The specimen analysis method of item 27, further comprising:
storing a state of the agitating part and a state of the container transfer part;
updating the state of the agitating part according to transition of the state of the agitating part;
updating the state of the container transfer part according to transition of the state of the container transfer part;
controlling operation of the agitating part according to the state of the container transfer part; and
controlling operation of the container transfer part according to the state of the agitating part.

Item 36: The specimen analysis method of item 25 or 26, wherein
the agitating comprises causing, by a drive part, reciprocating movement of a holding part in a predetermined direction in a state where a lower portion of each of the specimen containers is held by the holding part.

Item 37: The specimen analysis method of item 36, wherein
the agitating further comprises
   holding, by the holding part, the specimen container oriented such that a longitudinal direction of the specimen container extends along a tangent line direction to a rotation trajectory around a rotation shaft separated from the holding part, and
   fixing, by a fixation part, the specimen container placed in the holding part to the holding part, and
the causing comprises causing, by the drive part, reciprocating rotation of the holding part around the rotation shaft.

Item 38: The specimen analysis method of item 37, wherein
the causing, by the drive part, of reciprocating rotation comprises causing, by the drive part, reciprocating rotation of the holding part around the rotation shaft extending in a horizontal direction, and
the holding comprises holding, by the holding part, the lower portion of the specimen container oriented so as to extend along an up-down direction at an origin position for the reciprocating rotation.

Item 39: The specimen analysis method of item 38, wherein
the causing, by the drive part, of reciprocating rotation of the holding part around the rotation shaft extending in the horizontal direction comprises rotating, by the drive part, the holding part from the origin position to a predetermined angle larger than 90 degrees, and
the agitating further comprises receiving, by a fall-off object reception part disposed at a position below an upper end portion of the specimen container in a state of having been rotated to the predetermined angle, adhesion matter having fallen off from the specimen container.

Item 40: The specimen analysis method of item 39, wherein
the agitating further comprises abruptly stopping rotation of the holding part when the holding part reaches the predetermined angle from the origin position.

Item 41: The specimen analysis method of item 40, wherein
the drive part is connected to the holding part via an arm rotatable around the rotation shaft,
the arm is connected to the holding part in a state of allowing relative movement in a rotation direction by a predetermined amount, and
the abrupt stopping comprises stopping the arm rotated to the predetermined angle so that the holding part moves by the predetermined amount and collides with the arm owing to inertia, to be abruptly stopped.

Item 42: The specimen analysis method of item 41, wherein
the agitating further comprises abruptly stopping rotation of the holding part when the holding part reaches the origin position from the predetermined angle at a time of end of the agitating.

Item 43: The specimen analysis method of item 25 or 26, further comprising
preparing the measurement sample by selectively ejecting, to one of a plurality of reaction parts, the specimen suctioned from each of the specimen containers and reacting the specimen and a reagent with each other in the reaction part to which the specimen has been ejected, wherein
the plurality of reaction parts receive a same type of reagent.

Item 44: The specimen analysis method of item 43, wherein
the preparing of the measurement sample from the specimen suctioned from the one specimen container comprises ejecting, to one of the plurality of reaction parts, the specimen suctioned from the one specimen container, and
the preparing of the measurement sample from the specimen suctioned from the other specimen container transferred to the suction position next to the one specimen container comprises ejecting, to another one of the plurality of reaction parts, the specimen suctioned from the other specimen container.

## Claims

1. A specimen analyzer comprising:
a rack transport part configured to transport a rack accommodating a plurality of specimen containers;
an agitating part configured to agitate a specimen in each of the specimen containers;
a suction part configured to suction the specimen from the specimen container;
a container transport part configured to transport the specimen container between the rack on the rack transport part, the agitating part, and a suction position for specimen suctioning by the suction part;
a measurement part configured to measure a measurement sample prepared from the specimen suctioned by the suction part; and
a controller configured to control at least the agitating part, the suction part, and the container transport part, wherein
the specimen analyzer is configured such that control by the controller causes, during a period from start to end of agitation of one of the specimen containers, at least one of transferring to the suction position, suctioning of a specimen, and returning to the rack to be executed on another one of the specimen containers.

2. The specimen analyzer of claim 1, wherein
the container transport part includes
a hand part capable of being placed above the rack transport part and configured to grip each of the specimen containers and move in at least an up-down direction, and
a container transfer part configured to receive, from the hand part, the specimen container having been agitated by the agitating part and transfer the specimen container to the suction position, and
the agitating part receives, from the hand part, the specimen container having been taken out from the rack and agitates the specimen in the specimen container.

3. The specimen analyzer of claim 2, wherein
the specimen analyzer is configured such that control by the controller causes, before agitation of the one specimen container, the hand part to: transfer the other specimen container having been agitated by the agitating part, from the agitating part to the container transfer part; and transfer the one specimen container having yet to be agitated, from the rack to the agitating part.

4. The specimen analyzer of claim 2, wherein
the specimen analyzer is configured such that control by the controller causes the hand part to: return the other specimen container having been subjected to suctioning by the suction part, from the container transfer part to the rack; and, after agitation of the one specimen container, transfer the one specimen container having been agitated by the agitating part, from the agitating part to the container transfer part.

5. The specimen analyzer of any one of claims 2 to 4, wherein
the agitating part is configured to move to a first position,
the container transfer part is configured to move to a second position that coincides with the first position in a top view, and
the hand part is configured to execute, at the first position, insertion and taking-out of the specimen container with respect to the agitating part and execute, at the second position, insertion and taking-out of the specimen container with respect to the container transfer part.

6. The specimen analyzer of claim 5, wherein
the specimen analyzer is configured such that
control by the controller causes the agitating part to be placed at a third position away from the second position in the top view while the hand part is, at the second position, performing insertion and taking-out of the specimen container with respect to the container transfer part, and
control by the controller causes the container transfer part to be placed at a fourth position away from the first position in the top view while the hand part is, at the first position, performing insertion and taking-out of the specimen container with respect to the agitating part.

7. The specimen analyzer of claim 6, wherein
the first position, the second position, and a take-out position for taking out the specimen container from the rack are aligned in the up-down direction, and
the hand part is configured to perform taking-out and insertion of the specimen container with respect to the rack, insertion and taking-out of the specimen container with respect to the agitating part placed at the first position, and insertion and taking-out of the specimen container with respect to the container transfer part placed at the second position, through upward/downward movement without moving in a horizontal direction.

8. The specimen analyzer of claim 7, wherein
the agitating part is configured to linearly move to the first position and the third position along a first direction in a horizontal plane,
the container transfer part is configured to linearly move to the second position and the fourth position along a second direction intersecting with the first direction in the top view, and
the first position and the second position are each a position at which a movement path of the container transfer part and a movement path of the agitating part intersect with each other in the top view.

9. The specimen analyzer of any one of claims 1 to 4, wherein
the specimen analyzer is configured such that control by the controller causes, during the period from start to end of agitation of the one specimen container, the container transport part to perform transferring of the other specimen container to the suction position.

10. The specimen analyzer of claim 9, wherein
the specimen analyzer is configured such that control by the controller causes, during the period from start to end of agitation of the one specimen container, completion of the transferring of the other specimen container to the suction position and causes, during said period, the suction part to start suctioning of the specimen from the other specimen container transferred to the suction position.

11. The specimen analyzer of claim 10, wherein
the specimen analyzer is configured such that control by the controller causes, during the period from start to end of agitation of the one specimen container, completion of the transferring of the other specimen container to the suction position and the suctioning of the specimen from the other specimen container and causes, during said period, the container transport part to start returning of the other specimen container having been subjected to the suctioning to the rack.

12. The specimen analyzer of claim 11, further comprising
a reading part configured to read identification information provided on each of the specimen containers, wherein
the container transport part transports the specimen container to a reading position for reading by the reading part, and
the specimen analyzer is configured such that control by the controller causes, during the period from start to end of agitation of the one specimen container, execution of: movement of the other specimen container to the reading position by the container transport part; and reading of the identification information on the other specimen container by the reading part.

13. The specimen analyzer of claim 2, further comprising
a storage configured to store therein a state of the agitating part and a state of the container transfer part, wherein
the controller
updates the state of the agitating part stored in the storage, according to transition of the state of the agitating part,
updates the state of the container transfer part stored in the storage, according to transition of the state of the container transfer part,
controls operation of the agitating part according to the state of the container transfer part stored in the storage, and
controls operation of the container transfer part according to the state of the agitating part stored in the storage.

14. The specimen analyzer of any one of claims 1 to 4, wherein
the agitating part includes
a holding part configured to hold a lower portion of each of the specimen containers and
a drive part configured to cause reciprocating movement of the holding part in a predetermined direction.

15. The specimen analyzer of claim 14, wherein
the drive part is configured to cause reciprocating rotation of the holding part around a rotation shaft,
the holding part is configured to hold the specimen container oriented such that a longitudinal direction of the specimen container extends along a tangent line direction to a rotation trajectory around the rotation shaft, and
the agitating part further includes a fixation part configured to fix the specimen container placed in the holding part and release the fixed specimen container.

16. The specimen analyzer of claim 15, wherein
the drive part is configured to cause reciprocating rotation of the holding part around the rotation shaft extending in a horizontal direction, and
the holding part is configured to hold the lower portion of the specimen container oriented so as to extend along an up-down direction at an origin position for the reciprocating rotation.

17. The specimen analyzer of claim 16, wherein
the controller controls the drive part to rotate the holding part from the origin position to a predetermined angle larger than 90 degrees, and
the agitating part further includes a fall-off object reception part disposed at a position below an upper end portion of the specimen container in a state of having been rotated to the predetermined angle, the fall-off object reception part being configured to receive adhesion matter having fallen off from the specimen container.

18. The specimen analyzer of claim 17, wherein
the agitating part is configured to abruptly stop rotation of the holding part when the holding part reaches the predetermined angle from the origin position.

19. The specimen analyzer of claim 18, wherein
the drive part is connected to the holding part via an arm rotatable around the rotation shaft,
the arm is connected to the holding part in a state of allowing relative movement in a rotation direction by a predetermined amount, and,
when the arm rotated to the predetermined angle is stopped, the holding part moves by the predetermined amount and collides with the arm owing to inertia, to be abruptly stopped.

20. The specimen analyzer of claim 19, wherein
the controller is configured to perform control to abruptly stop rotation of the holding part when the holding part reaches the origin position from the predetermined angle at a time of end of agitation.

21. The specimen analyzer of any one of claims 1 to 4, further comprising
a plurality of reaction parts each configured to receive the specimen suctioned by the suction part and a reagent and react the specimen and the reagent with each other, to prepare the measurement sample, wherein
the plurality of reaction parts receive a same type of reagent, and
the controller controls the suction part to selectively eject, to one of the plurality of reaction parts, the specimen suctioned from each of the specimen containers.

22. The specimen analyzer of claim 21, wherein
the controller controls the suction part to
eject, to one of the plurality of reaction parts, the specimen suctioned from the one specimen container and
eject, to another one of the plurality of reaction parts, the specimen suctioned from the other specimen container transferred to the suction position next to the one specimen container.

23. The specimen analyzer of claim 1, wherein
the specimen analyzer is configured such that measurement of a measurement sample prepared from the specimen contained in the one specimen container and measurement of a measurement sample prepared from the specimen contained in the other specimen container are executed by the measurement part which is common to the measurements.

24. The specimen analyzer of claim 1, wherein
the specimen analyzer can be operated in
a first mode of measuring a plurality of ordinary specimens accommodated in the rack on the rack transport part and
a second mode of measuring an interruption specimen in preference to the plurality of ordinary specimens, and
the specimen analyzer is configured such that control by the controller causes, when the second mode is set, the ordinary specimens present in the agitating part and the container transport part to be returned to the rack.

25. A specimen analysis method to be performed by a specimen analyzer configured to analyze a specimen contained in a specimen container, the specimen analysis method comprising:
taking out, from a rack accommodating a plurality of the specimen containers, any of the specimen containers;
agitating a specimen in the specimen container having been taken out;
transferring, to a suction position, the specimen container having been agitated;
suctioning the specimen from the specimen container transferred to the suction position;
returning, to the rack, the specimen container having been subjected to the suctioning of the specimen; and
measuring a measurement sample prepared from the suctioned specimen, wherein,
during a period from start to end of the agitating of one of the specimen containers, at least one of the transferring to the suction position, the suctioning of a specimen, and the returning to the rack is executed on another one of the specimen containers.
